(19) Europäisches Patentamt
European Patent Office
Office européen des brevets

(11) Publication number: 0 357 460 B1

# (12) EUROPEAN PATENT SPECIFICATION

(45) Date of publication of patent specification: 18.01.95

(51) Int. Cl.[6]: C07D 493/22, A61K 31/365, A01N 43/90, //(C07D493/22, 313:00,311:00,311:00,307:00)

(21) Application number: 89308900.3

(22) Date of filing: 01.09.89

The file contains technical information submitted after the application was filed and not included in this specification

## (54) 13-Substituted milbemycin derivatives, their preparation and use.

(30) Priority: 02.09.88 JP 220235/88

(43) Date of publication of application: 07.03.90 Bulletin 90/10

(45) Publication of the grant of the patent: 18.01.95 Bulletin 95/03

(84) Designated Contracting States: AT BE CH DE ES FR GB GR IT LI LU NL SE

(56) References cited:
EP-A- 0 184 173
EP-A- 0 246 739

(73) Proprietor: Sankyo Company Limited
5-1 Nihonbashi Honcho 3-chome
Chuo-ku
Tokyo (JP)

(72) Inventor: Morisawa, Yasuhiro c/o Sankyo Company Limited
No. 2-58, 1-chome Hiromachi
Shinagawa-ku
Tokyo 140 (JP)
Inventor: Saito, Akio c/o Sankyo Company Limited
No. 2-58, 1-chome Hiromachi
Shinagawa-ku
Tokyo 140 (JP)
Inventor: Toyama, Toshimitsu c/o Sankyo Company Limited
No. 2-58, 1-chome Hiromachi
Shinagawa-ku
Tokyo 140 (JP)
Inventor: Kaneko, Susumu c/o Sankyo Company Limited
No. 2-58, 1-chome Hiromachi
Shinagawa-ku
Tokyo 140 (JP)

(74) Representative: Gibson, Christian John Robert et al
MARKS & CLERK,
57/60 Lincoln's Inn Fields
London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates to a series of new macrolide compounds which are chemically related to certain known classes of macrolides including those known as the milbemycins and the avermectins. These compounds have valuable acaricidal, insecticidal and anthelmintic activities. The invention also provides methods of preparing these compounds and compositions and methods for using them.

There are several classes of known compounds with a structure based on a 16-membered macrolide ring, which compounds are obtained by fermentation of various microorganisms or are obtained semi-synthetically by chemical derivatization of such natural fermentation products, and which exhibit acaricidal, insecticidal, anthelmintic and antiparasitic activities. The milbemycins and avermectins are examples of two such classes of known compounds, but various others also exist and are identified in the art by different names or code numbers. The names for these various macrolide compounds have generally been taken from the names or code numbers of the microorganisms which produce the naturally occurring members of each class, and these names have then been extended to cover the chemical derivatives of the same class, with the result that there has been no standardized systematic nomenclature for such compounds generally.

In order to avoid confusion, a standardized system of nomenclature will be used herein, which follows the normal rules for naming derivatives of organic compounds as recommended by the International Union of Pure and Applied Chemistry, Organic Chemistry Division, Commission on Nomenclature of Organic Chemistry, and which is based primarily on the hypothetical parent compound hereby defined as "milbemycin" and represented by the formula (II):

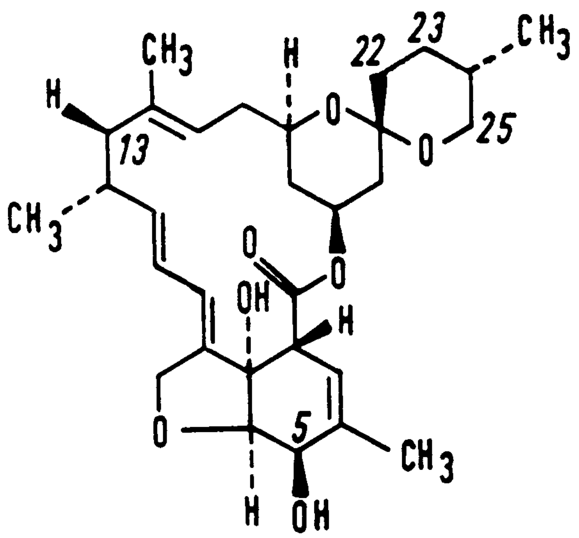

(II)

For the avoidance of doubt, formula (II) also shows the numbering of positions of the macrolide ring system applied to those positions most relevant to the compounds of the present invention and of the prior art.

The naturally produced milbemycins are a series of macrolide compounds known to have anthelmintic, acaricidal and insecticidal activities. Milbemycin D was disclosed in U.S. Patent No. 4,346,171, where it was referred to as "Compound B-41D", and milbemycins $A_3$ and $A_4$ were disclosed in U.S. Patent No. 3,950,360. These compounds may be represented by the above formula (II) in which there is a hydrogen atom at position 13 and position 25 is substituted with a methyl group, an ethyl group or an isopropyl group, these compounds being designated as milbemycin $A_3$, milbemycin $A_4$ and milbemycin D, respectively. The milbemycin analogue having a hydrogen atom at position 13 and substituted at position 25 with a sec-butyl group was disclosed in U.S. Patent No. 4,173,571, where it was known as "13-deoxy-22,23-dihydroavermectin $B_{1a}$ aglycone". Certain of the compounds of the present invention are named as derivatives of this and related compounds, the numbering system being as shown above on formula (II).

Subsequently, various derivatives of the original milbemycins and avermectins have been prepared and their activities investigated. For example, 5-esterified milbemycins have been disclosed in U.S. Patents No. 4,201,861, No. 4,206,205, No. 4,173,571, No. 4,171,314, No. 4,203,976, No. 4,289,760, No. 4,457,920, No. 4,579,864 and No. 4,547,491, in European Patent Publications No. 8184, No. 102,721, No. 115,930, No. 180,539 and No. 184,989 and in Japanese Patent Applications Kokai (i.e. as laid open to public inspection) No. 57-120589 and 59-16894.

13-Hydroxy-5-ketomilbemycin derivatives have been disclosed in U.S. Patent No. 4,423,209. Milbemycin 5-oxime derivatives were disclosed in U.S. Patent No. 4,547,520 and in European Patent Publication No. 203 832.

Milbemycins having an ether linkage at the 13 position are of particular relevance to the present invention and the lower alkyl, phenyl and benzyl ethers are described in general terms in U.S. Patent 4 696 945, but only the methyl and ethyl ethers are specifically described in the Examples of that U.S. Patent.

Like the milbemycins, the avermectins are based upon the same 16-membered ring macrolide compound. The avermectins are disclosed, for example in J. Antimicrob. Agents Chemother., 15(3), 361 - 367 (1979). These compounds may be represented by the above formula (II) but with a carbon-carbon double bond at positions 22 and 23, and having position 13 substituted with a 4'-(α-L-oleandrosyl)-α-L-oleandrosyloxy group. Position 25 may be substituted with an isopropyl group or a sec-butyl group, these compounds being designated as avermectin $B_{1b}$ and avermectin $B_{1a}$, respectively. 22,23-Dihydroavermectins $B_{1a}$ and $B_{1b}$ may be obtained by reduction of the double bond between the 22 and 23 positions and are disclosed in U.S. Patent No. 4,199,569. The aglycone derivatives of the avermectins, which are milbemycin analogues, have sometimes been referred to in the literature as C-076 compounds, and various derivatives of these are known. For example, U.S. Patent No. 4,201,861 discloses such derivatives substituted with a lower alkanoyl group at position 13.

Published European Patent Application No. 170006 discloses a family of bioactive compounds produced by fermentation, identified collectively by the code number LL-F28249. Some of these have a 16-membered macrolide structure corresponding to the above formula (II), substituted with a hydroxy group at position 23 and with a 1-methyl-1-propenyl, 1-methyl-1-butenyl or 1,3-dimethyl-1-butenyl group at position 25. In these compounds, the hydroxy group at position 5 may also be replaced by a methoxy group.

Published British Patent Application No. 2,176,182 discloses another group of macrolide antibiotics corresponding to the above formula (II) with a hydroxy or substituted hydroxy group at position 5, a hydroxy, substituted hydroxy or keto group at position 23, and an α-branched alkenyl group at position 25.

The various classes of milbemycin-related macrolide compounds described above are all disclosed as having one or more types of activity as antibiotic, anthelmintic, ectoparasiticidal, acaricidal or other pesticidal agents. However, there is still a continuing need to provide such agents with improved activity against one or more classes of pests.

It has now been discovered that the activity of such milbemycin-related derivatives can be improved by appropriately selecting the combination of substituents on the macrolide ring system, especially the substituents at position 13. In particular, it has now been found that the activity of the compounds can be improved upon by appropriate selection of certain highly specific ether groups at the 13 position, as specified below.

The compounds of the present invention are those compounds having the formula (I):

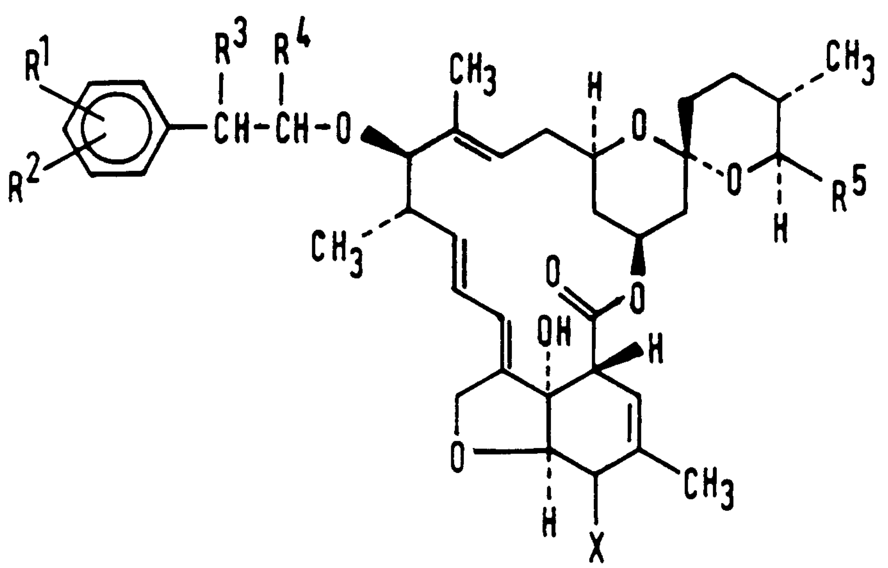

(I)

in which:

$R^1$ and $R^2$ are the same or different and each represents: a hydrogen atom; a halogen atom; a cyano group; a nitro group; a $C_1$ - $C_4$ alkyl group; a substituted $C_1$ - $C_4$ alkyl group having at least one of substituents (a),

defined below; a $C_1$ - $C_4$ alkoxy group; a $C_2$ - $C_6$ alkoxyalkoxy group; a group of formula $-(CH_2)_nNHR^9$,

in which: n represents 0 or the integer 1 or 2, and $R^9$ represents a hydrogen atom or a $C_1$ - $C_4$ alkyl group;

a group of formula $-(CH_2)_nNR^9C(=O)R^6$,

in which:

n and $R^9$ are as defined above, and

$R^6$ represents: a hydrogen atom; a $C_1$ - $C_4$ alkyl group; a substituted $C_1$ - $C_4$ alkyl group having at least one of substituents (b), defined below; a $C_2$ - $C_8$ aliphatic hydrocarbon group having one or two ethylenically unsaturated carbon-carbon double bonds, said group being unsubstituted or having at least one of substituents (b), defined below; a $C_2$ - $C_8$ alkynyl group; a substituted $C_2$ - $C_8$ alkynyl group having at least one of substituents (b), defined below a $C_3$ - $C_8$ cycloalkyl group; a substituted $C_3$ - $C_8$ cycloalkyl group having at least one of substituents (c), defined below; a carbocyclic aryl group having from 6 to 14 ring carbon atoms and being unsubstituted or having at least one of substituents (c), defined below; or a heterocyclic group having from 3 to 6 ring atoms of which at least one is a nitrogen and/or oxygen and/or sulphur hetero-atom, said heterocyclic group being monocyclic or being fused to one or two benzene rings and being unsubstituted or having at least one of substituents (c), defined below;

a group of formula $-(CH_2)_nNR^9COCOR^6$

in which n, $R^6$ and $R^9$ are as defined above;

a group of formula $-(CH_2)_nR^9COCOOR^7$

in which n and $R^9$ are as defined above and $R^7$ represents a $C_1$ - $C_4$ alkyl group, a $C_3$-$C_8$ cycloalkyl group or an aralkyl group as defined below;

a group of formula $-(CH_2)_nNR^9CHR^6NHCOR^6$

in which n, $R^6$ and $R^9$ are as defined above;

a group of formula $-(CH_2)_nNR^9CHR^6NHCONHR^6$

in which n, $R^6$ and $R^9$ are as defined above;

a group of formula $-(CH_2)_nNR^9CHR^6NHCOOR^7$

in which n, $R^6$, $R^7$ and $R^9$ are as defined above;

a group of formula $-(CH_2)_nNR^9C(=Y)YR^6$

in which n, $R^6$ and $R^9$ are as defined above and the two symbols Y are the same or different and each represents an oxygen or sulphur atom;

a group of formula $-(CH_2)_nNR^9C(=Y)NR^{6'}R^{6'}$

in which n, Y and $R^9$ are as defined above, and the two symbols $R^{6'}$ are the same or different and each represents one of the groups or atoms defined above for $R^6$, or the two, together with the nitrogen atom to which they are attached, form a heterocyclic group having from 3 to 7 ring atoms of which one is said nitrogen atoms and 0 or 1 is an additional nitrogen and/or oxygen and/or sulphur hetero-atom;

a group of formula $-(CH_2)_nNR^9C(=Y)NR^{6''}NR^{6''}R^{6''}$

in which n, Y and $R^9$ are as defined above, and each of the symbols $R^{6''}$ is the same or different and each represents one of the groups or atoms defined above for $R^6$, or any two of the symbols $R^{6''}$, together with the nitrogen atom to which each is attached, forms a heterocyclic group having from 3 to 7 ring atoms of which one or two is said nitrogen atom or atoms and 0 or 1 is an additional nitrogen and/or oxygen and/or sulphur hetero-atom;

a group of formula $-(CH_2)_nNR^9C(=Y)NR^6NHZ$

in which n, Y, $R^6$ and $R^9$ are as defined above and Z represents

a group of formula $-COOR^7$, in which $R^7$ is as defined above,

a group of formula $-COR^6$, in which $R^6$ is as defined above, or

a group of formula $-SO_2R^6$, in which $R^6$ is as defined above;

a group of formula $-(CH_2)_nNR^9C(=NR^{10})NHR^{10}$

in which n and $R^9$ are as defined above and the two symbols $R^{10}$ are the same or different and each represents one of the groups or atoms defined above for $R^6$, or a cyano group, a nitro group, a group of formula $-COOR^7$, in which $R^7$ is as defined above, or a group of formula $-COR^6$, in which $R^6$ is as defined above;

a group of formula $-(CH_2)_nNR^9C(=NR^{10})R^6$

in which n, $R^6$, $R^9$ and $R^{10}$ are as defined above;

a group of formula $-(CH_2)_nNR^9SO_mR^6$

in which n, $R^6$ and $R^9$ are as defined above and m is 1 or 2;

a group of formula $-CONHR^6$

in which $R^6$ is as defined above; and

a group of formula $-COOR^7$

in which $R^7$ is as defined above;

$R^3$ and $R^4$ are the same or different and each represents a hydrogen atom, a $C_1$ - $C_4$ alkyl group or a $C_1$ - $C_4$ alkoxy group;

$R^5$ represents a methyl group, an ethyl group, an isopropyl group or a sec-butyl group; and

X represents a hydroxy group, a $C_1$ - $C_5$ alkanoyloxy group, a substituted $C_1$ - $C_5$ alkanoyloxy group having at least one of substituents (d), defined below, or a hydroxyimino group;

said aralkyl groups have from 1 to 4 carbon atoms in the alkyl part and from 6 to 10 ring atoms in the aryl part, which is a carbocyclic aryl group which is unsubstituted or has at least one of substituents (c), defined below;

substituents (a):

halogen atoms, $C_1$ - $C_4$ alkoxy groups, $C_1$ - $C_4$ alkylthio groups and $C_1$ - $C_5$ alkanoyloxy groups;

substituents (b):

$C_3$ - $C_8$ cycloalkyl groups; $C_1$ - $C_4$ alkoxy groups; $C_1$ - $C_4$ alkylthio groups; $C_2$ - $C_5$ cyanoalkylthio groups; $C_2$ - $C_5$ alkoxycarbonyl groups; halogen atoms; cyano groups; nitro groups; amino groups; carbocyclic aryl groups having from 6 to 10 carbon atoms and being unsubstituted or having at least one of substituents (c), defined below; aromatic heterocyclic groups having from 5 to 8 ring atoms of which from 1 to 4 are nitrogen and/or oxygen and/or sulphur hetero-atoms, said heterocyclic group being monocyclic or being fused either to a benzene ring or to a heterocyclic group which has 5 or 6 ring atoms of which from 1 to 3 are nitrogen hetero-atoms and being unsubstituted or having at least one of substituents (c), defined below; and aryloxy and arylthio groups in which the aryl part has from 6 to 10 carbon atoms and is unsubstituted or has at least one of substituents (c), defined below;

substituents (c):

$C_1$ - $C_4$ alkyl groups, $C_1$ - $C_4$ alkoxy groups, $C_1$ - $C_4$ alkylthio groups, $C_1$ - $C_5$ alkanoyloxy groups, $C_2$ - $C_5$ alkoxycarbonyl groups, halogen atoms, cyano groups, nitro groups, amino groups, mono- and di- alkylamino groups in which the or each alkyl part is $C_1$ - $C_4$, carbamoyl groups, mono- and di-alkylcarbamoyl groups in which the or each alkyl part is $C_1$ - $C_4$, and $C_1$ - $C_5$ alkanoylamino groups;

substituents (d):

halogen atoms, $C_1$ - $C_4$ alkoxy groups, $C_2$ - $C_5$ alkoxycarbonyl groups and carboxy groups;
and salts thereof.

The invention still further provides an anthelmintic, acaricidal and insecticidal composition comprising an anthelmintic, acaricidal and insecticidal compound in admixture with a pharmaceutically, agriculturally, veterinarily or horticulturally acceptable carrier or diluent, wherein said compound is at least one compound of formula (I) or a salt thereof.

The invention still further provides the use for the manufacture of a medicament for the treatment of infections by helminths, acarids or insects of at least one compound of formula (I) or a salt thereof.

The invention still further provides a method of protecting animals or plants from damage by parasites selected from acarids, helminths and insects, which comprises applying an active compound to said animals, to said plants or to seeds of said plants or to a locus including said animals, plants or seeds, wherein the active compound is at least one compound of formula (I) or a salt thereof.

In the compounds of the present invention, where $R^1$ or $R^2$ or substituent (a), (b), (c) or (d) represents a halogen atom, this may be a fluorine, chlorine, bromine or iodine atom and is preferably a chlorine or fluorine atom.

Where $R^1$, $R^2$, $R^3$, $R^4$, $R^6$, $R^{6'}$, $R^7$, $R^9$ or $R^{10}$ or substituent (c) represents an alkyl group, this has from 1 to 4 carbon atoms and may be a straight or branched chain group. Examples of such groups include the methyl, ethyl, propyl, isopropyl, butyl, sec-butyl and t-butyl groups, of which the methyl, ethyl, propyl, isopropyl, butyl and sec-butyl groups are preferred and the methyl and ethyl groups are most preferred.

Where $R^1$, $R^2$, $R^6$, $R^{6'}$ or $R^{10}$ represents a substituted alkyl group, the alkyl part may be any of the alkyl groups exemplified above and: in the case of $R^1$ or $R^2$, the substituent is selected from substituents (a); and, in the case of $R^6$, $R^{6'}$ or $R^{10}$, the substituent is selected from substituents (b); the substituents being defined above and exemplified elsewhere herein.

Where $R^1$, $R^2$, $R^3$ or $R^4$ or substituent (a), (b), (c) or (d) represents an alkoxy group, this has from 1 to 4 carbon atoms and may be a straight or branched chain group. Examples of such groups include the methoxy, ethoxy, propoxy, isopropoxy, butoxy, isobutoxy, sec-butoxy and t-butoxy groups, especially the methoxy, ethoxy, propoxy, isopropoxy and butoxy groups.

Where $R^1$ or $R^2$ represents a $C_2$ - $C_6$ alkoxyalkoxy group, each of the alkoxy parts may have from 1 to 5, preferably from 1 to 4, carbon atoms, provided that the total number of carbon atoms in the two alkoxy groups does not exceed 6, and preferred examples of such alkoxy groups are as given above. Examples of the alkoxyalkoxy groups include the methoxymethoxy, ethoxymethoxy, propoxymethoxy, butoxymethoxy, 1- and 2-methoxyethoxy, 1- and 2- ethoxyethoxy, 1- and 2- butoxyethoxy and 1-, 2- and 3- methoxypropoxy groups, of which the methoxymethoxy, ethoxymethoxy, propoxymethoxy, butoxymethoxy, methoxyethoxy, ethoxyethoxy and butoxyethoxy groups are preferred.

Where $R^6$ represents a $C_2$ - $C_8$ alkenyl or alkynyl group, it may be, for example, a vinyl, 1-propenyl, allyl, 1-butenyl, 2-butenyl, 3-butenyl, butadienyl, 1-pentenyl, 2-pentenyl, 3-pentenyl, 4-pentenyl, 1,3-dimethylbutenyl, 1-hexenyl, 2-hexenyl, 3-hexenyl, 4-hexenyl, 5-hexenyl, 1,3-, 1,4-, 1,5-, 2,4-, 2,5- and 3,5-hexadienyl, 1-, 2-, 3-, 4-, 5-, and 6-heptenyl, 1-, 2-, 3-, 4-, 5-, 6- and 7- octenyl, ethynyl, 1-propynyl, 1-, 2- and 3- butynyl, 1-, 2-, 3-and 4- pentynyl, 1-, 2-, 3-, 4- and 5- hexynyl, 1-, 2-, 3-, 4-, 5- and 6- heptynyl, 1-, 2-, 3-, 4-, 5-, 6- and 7- octynyl and propargyl groups, of which the 1-propenyl, allyl, 1-butenyl, 2-butenyl, 3-butenyl, 1,3-dimethylbutenyl, hexadienyl and propargyl groups are preferred. Such groups may be unsubstituted or they may be substituted by at least one of substituents (b), defined above and exemplified generally herein. However, they are preferably unsubstituted.

Where $R^6$, $R^7$ or substituent (b) represents a cycloalkyl group, this may contain from 3 to 8 ring atoms, and examples are the cyclopropyl, cyclobutyl, cyclopentyl, cyclohexyl cycloheptyl and cyclooctyl groups, of which the cyclopentyl and cyclohexyl groups are more preferred. Such groups may be unsubstituted or they may be substituted by at least one of substituents (c), defined above and exemplified generally herein. However, they are preferably unsubstituted.

Where $R^6$ represents a heterocyclic group, this may be a saturated or unsaturated group containing from 3 to 6 ring atoms, of which at least one, and preferably from 1 to 3, is a nitrogen and/or oxygen and/or sulphur hetero-atom. More preferably the group has from 0 to 3 such nitrogen atoms, 0, 1 or 2 such oxygen atoms and 0, 1 or 2 such sulphur atoms, provided that the total number of hetero-atoms is not less than 1 and does not exceed 3. Where the group is unsaturated, it may be non-aromatic or aromatic in character. The group may be monocyclic or it may be fused to one or two benzene rings to produce a bicyclic or tricyclic group, in which the heterocyclic part may be aromatic or non-aromatic in character. Examples of such groups include the oxiranyl, oxetanyl, aziridinyl, azetidinyl, thiranyl, thietanyl, furyl, thienyl, pyrrolyl, pyridyl, thiazolyl, isothiazolyl, oxazolyl, isoxazolyl, imidazolyl, pyrazolyl, pyranyl, pyrazinyl, pyridazinyl, pyrimidinyl, benzofuranyl, isobenzofuranyl, benzothienyl, isobenzothienyl, indolyl, quinolyl, isoquinolyl, quinazolinyl, quinoxalinyl, naphthyridynyl, xanthenyl, tetrahydrofuranyl, tetrahydrothienyl, pyrrolidinyl, thiazolidinyl, imidazolidinyl, imidazolinyl, oxazolinyl, oxazolidinyl, pyrazolidinyl, piperazyl, tetrahydropyrimidinyl, dihydropyridazinyl, morpholinyl, thiomorpholinyl, indolinyl, tetrahydroquinolyl, pyrrolidonyl, piperidonyl, pyridonyl, thianthrenyl, chromenyl, phenoxathiinyl, 2<u>H</u>-pyrrolyl, isoindolyl, 3<u>H</u>-indolyl, indazolyl, phthalazinyl, quinoxalinyl, quinazolinyl, cinnolinyl, carbazolyl, phenanthridinyl, acridinyl, perimidinyl, phenazinylphenothiazinyl, furazanyl, phenoxazinyl, isochromanyl, chromanyl, pyrazolinyl, indolinyl and isoindolinyl groups. Such groups may be unsubstituted or they may be substituted by at least one of substituents (c), defined above and exemplified elsewhere herein.

Where $R^1$ or $R^2$ represents a group of formula $-(CH_2)_nNR^9C(=Y)NR^{6'}R^{6'}$, the two groups represented by $R^{6'}$ may be the same or different and may be selected from those groups and atoms represented by $R^6$ and defined and exemplified above. Alternatively, the two groups $R^{6'}$, together with the nitrogen atom to which they are attached, may form a nitrogen-containing heterocyclic group, which may optionally have an additional nitrogen and/or oxygen and/or sulphur hetero-atom; such a group may contain from 3 to 7 atoms in total (i.e. including the afore-mentioned nitrogen atom) and may be saturated or unsaturated. If it is unsaturated the unsaturation may be aromatic or non-aromatic in character, provided that the group has a nitrogen atom which can provide the nitrogen atom of the group $-NR^{6'}R^{6'}$ (i.e. that nitrogen atom cannot participate in the aromaticity). Examples of such groups include the aziridinyl, azetidinyl, pyrrolyl, imidazolyl, pyrazolyl, pyrrolidinyl, thiazolidinyl, imidazolidinyl, imidazolinyl, oxazolinyl, oxazolidinyl, pyrazolidinyl, piperazyl, tetrahydropyrimidinyl, dihydropyridazinyl, pyrrolidonyl, piperidonyl, pyridonyl, pyrazolinyl, azepinyl, perhydroazepinyl, oxazepinyl and thiazepinyl groups. Such groups may be unsubstituted or they may be substituted by at least one of substituents (c), defined above and exemplified elsewhere herein.

Where $R^1$ or $R^2$ represents a group of formula $-(CH_2)_nNR^9C(=Y)NR^{6''}NR^{6''}R^{6''}$, the group $-NR^{6''}R^{6''}$ may be a group of formula $-NR^6R^6$, in which each $R^6$ is as defined above, or it may be a group of formula $-NR^{6'}R^{6'}$, which forms a heterocyclic group as exemplified in the preceding paragraph. Alternatively, two of the symbols $R^{6''}$ attached to different nitrogen atoms may form a heterocyclic ring containing at least two nitrogen atoms and optionally another nitrogen and/or oxygen and/or sulphur hetero-atom. Examples of such groups include the divalent groups derived by removal of a hydrogen atom from each of the two adjacent nitrogen atoms of the ring systems; diaziridine, diazete, diazetidine, pyrazolidine, pyrazoline, 1,2-dihydropyridazine, 1,2,3,4-tetrahydropyridazine, 1,2,5,6-tetrahydropyridazine, perhydropyridazine, 1,2-dihydro-1,2-diazepine and perhydro-1,2-diazepine.

Where X or substituent (a) or (c) represents an alkanoyloxy group, it contains from 1 to 5 carbon atoms and may be a straight or branched chain group. Examples of such groups include the formyloxy, acetoxy, propionyloxy, butyryloxy, isobutyryloxy, valeryloxy, isovaleryloxy and pivaloyloxy groups. Such groups may be unsubstituted, or they may be substituted by at least one of substituents (d), defined above and exemplified elsewhere herein.

Where substituent (a), (b) or (c) is an alkylthio group, this contains from 1 to 4 carbon atoms and may be a straight or branched chain group. Examples of such groups include the methylthio, ethylthio, propylthio, isopropylthio, butylthio, isobutylthio, sec-butylthio and t-butylthio groups.

Where substituent (b), (c) or (d) is an alkoxycarbonyl group, this has a total of from 2 to 5 carbon atoms, i.e., the alkoxy part has from 1 to 4 carbon atoms, and this alkoxy part may be any of those alkoxy groups exemplified above. Examples of such alkoxycarbonyl groups include the methoxycarbonyl, ethoxycarbonyl, propoxycarbonyl, butoxycarbonyl, sec-butoxycarbonyl and t-butoxycarbonyl groups.

Where substituent (b) is a cyanoalkylthio group, this may be a straight or branched chain group having from 2 to 5 carbon atoms in total, i.e. the alkyl part has from 1 to 4 carbon atoms, and may be any of those alkyl groups exemplified above. Examples of such cyanoalkylthio groups include the cyanomethylthio, 1-cyanoethylthio, 2-cyanoethylthio, 1-cyanopropylthio, 2-cyanopropylthio, 3-cyanopropylthio, 1-cyanobutylthio, 2-cyanobutylthio, 3-cyanobutylthio, 4-cyanobutylthio, 3-cyano-2-methylpropylthio, 2-cyano-2-methylpropylthio and 2-cyano-1-methylethylthio groups.

Where substituent (b) is an aryl group, this has from 6 to 14 ring carbon atoms and is a carbocyclic group. Examples of such groups include the phenyl, naphthyl (1- or 2-) or anthryl groups, of which the phenyl and naphthyl groups are preferred and the phenyl group is most preferred.

Where substituent (b) is an aromatic heterocyclic group, this has from 5 to 8 ring atoms of which from 1 to 4 are nitrogen and/or oxygen and/or sulphur hetero-atoms and which has at least two conjugated double bonds to give an aromatic character to the ring. More preferably the group has from 0 to 4 such nitrogen atoms, 0, 1 or 2 such oxygen atoms and 0, 1 or 2 such sulphur atoms, provided that the total number of hetero-atoms is not less than 1 and does not exceed 4. The group may be monocyclic or it may be fused to a benzene ring to form a bicyclic ring system. Such groups may be substituted or unsubstituted and, if substituted, may be substituted by at least one of substituents (c), defined above and exemplified elsewhere herein. Examples of such aromatic heterocyclic groups include the pyridyl, thienyl, furyl, pyrrolyl, imidazolyl, triazolyl, tetrazolyl, thiazolyl, oxazolyl, indolyl, benzofuryl, isobenzofuryl, chromenyl, 2H-pyrrolyl, pyrazolyl, isothiazolyl, isoxazolyl, pyrazinyl, pyrimidinyl, pyridazinyl, isoindolyl, 3H-indolyl, 1H-indazolyl, isoquinolyl, quinolyl, phthalazinyl, quinoxalinyl, quinazolinyl and cinnolinyl groups.

Where substitutent (b) is an aryloxy or arylthio group, the aryl part has from 6 to 10 carbon atoms and is a carbocyclic aryl group. Examples include the phenoxy, phenylthio, 1-naphthyloxy, 2-naphthyloxy, 1-naphthylthio and 2-naphthylthio groups, of which the phenoxy and phenylthio groups are preferred. Such groups may be substituted or unsubstituted and, if substituted, the substituent is selected from substituents (c), defined above and exemplified elsewhere herein.

Where substituent (c) is a mono- or di- alkylamino group, the or each alkyl group may have from 1 to 4 carbon atoms and may be a straight or branched chain group. Examples of alkyl groups are given above. Examples of such mono- and di- alkylamino groups include the methylamino, ethylamino, propylamino, isopropylamino, butylamino, dimethylamino, diethylamino, N-methyl-N-ethylamino, N-methyl-N-propylamino and N-ethyl-N-butylamino groups.

Where substituent (c) is a mono- or di- alkylcarbamoyl group, the or each alkyl group may have from 1 to 4 carbon atoms and may be a straight or branched chain group. Examples of alkyl groups are given above. Examples of such mono- and di- alkylcarbamoyl groups include the methylcarbamoyl, ethylcarbamoyl, propylcarbamoyl, isopropylcarbamoyl, butylcarbamoyl, dimethylcarbamoyl, diethylcarbamoyl, N-methyl-N-ethylcarbamoyl, N-methyl-N-propylcarbamoyl and N-ethyl-N-butylcarbamoyl groups.

Where substituent (c) is a $C_1$ - $C_5$ alkanoylamino group, the alkanoyl part may be a straight or branched chain group and examples include the formylamino, acetylamino, propionylamino, butyrylamino,

isobutyrylamino, valerylamino, isovalerylamino and pivaloylamino groups.

Where $R^7$ represents an aralkyl group, the alkyl part has from 1 to 4 carbon atoms and may be any of the alkyl groups exemplified above. The aryl part has from 6 to 10 carbon atoms in its ring and, again, may be any of the aryl groups exemplified above. Examples of such aralkyl groups include the benzyl, phenethyl, $\alpha$-methylbenzyl, 1-phenylpropyl, 2-phenylpropyl, 3-phenylpropyl and 4-phenylbutyl groups, of which the benzyl and phenethyl groups are preferred.

Where substituent (d) is a carboxy group, the compounds can form salts with various sorts of bases. Such salts include, for example: salts with an alkali metal, such as lithium, sodium or potassium; salts with an alkaline earth metal, such as calcium or barium; salts with another metal, such as magnesium or aluminium; and salts with an organic amine, such as triethylamine or triethanolamine.

In general, in the discussion above, where reference is made to a substituted group, there is no particular restriction on the number of substituents, except such as may be imposed by the number of substitutable positions, or possibly by steric constraints, each of which is well recognised by those skilled in the art. However, as a general rule, we normally find it convenient to have no more than 3 such substituents, and sometimes fewer, i.e. 1, 2, or 3. More preferably, the number of the substituents is 1, 2 or 3 where the substituent is a halogen atom, and 1 in other cases.

Of the compounds of formula (I) of the present invention, representative preferred classes are as follows:

(1) those wherein:

$R^1$ represents a hydrogen atom; and

$R^2$ represents a hydrogen atom, a $C_1$ - $C_3$ alkyl group (such as a methyl, ethyl, propyl, or isopropyl group), a $C_1$ - $C_3$ alkoxy group (such as a methoxy, ethoxy, propoxy or isopropoxy group), a fluorine or chlorine atom, a nitro group or an amino group;

(2) those wherein:

$R^1$ represents a hydrogen atom; and

$R^2$ represents a group of formula $-(CH_2)_nNR^{9a}COR^{6a}$

[in which n is 0, $R^{9a}$ represents a hydrogen atom or a methyl group, and $R^{6a}$ represents: a $C_1$ - $C_4$ alkyl group (such as a methyl, ethyl, propyl, isopropyl or butyl group); a $C_3$ - $C_5$ cycloalkyl group (such as a cyclopropyl, cyclobutyl or cyclopentyl group); a $C_1$ - $C_3$ alkyl group substituted with a halogen, cyano, $C_1$ - $C_3$ alkoxy, $C_1$ - $C_3$ alkylthio, cyanomethylthio or phenoxy substituent (such as a fluoromethyl, bromoethyl, difluoromethyl, cyanomethyl, cyanopropyl, methoxymethyl, ethoxymethyl, methylthiomethyl, cyanomethylthiomethyl or phenoxymethyl group); an alkenyl group (such as a vinyl or allyl group); an unsubstituted phenyl group; a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group); a pyridyl group; a pyrimidyl group; a pyrazyl group; a furyl group; or a thienyl group],

(3) those wherein:

$R^1$ represents a hydrogen atom; and

$R^2$ represents a group of formula $-(CH_2)_nNR^{9a}COCOR^{6b}$

[in which n is 0, $R^{9a}$ represents a hydrogen atom or a methyl group, and $R^{6b}$ represents: a hydrogen atom; a $C_1$ - $C_4$ alkyl group (such as a methyl, ethyl, propyl, isopropyl or butyl group;) a $C_3$ - $C_5$ cycloalkyl group (such as a cyclopropyl, cyclobutyl or cyclopentyl group); an alkenyl group (such as a vinyl or allyl group;) an unsubstituted phenyl group; or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group)];

(4) those wherein:

$R^1$ represents a hydrogen atom; and

$R^2$ represents a group of formula $-(CH_2)_nNR^{9a}C(=Y)YR^{6c}$

[in which n is 0, $R^{9a}$ represents a hydrogen atom or a methyl group, both Y are oxygen atoms and $R^{6c}$ represents: a $C_1$ - $C_4$ alkyl group (such as a methyl, ethyl, propyl, isopropyl or butyl group); a $C_1$ - $C_4$ alkyl group substituted with a halogen or $C_1$ - $C_3$ alkoxy substituent (such as a fluoroethyl, trichloroethyl, methoxyethyl or ethoxyethyl group); an alkenyl group (such as a vinyl or allyl group); a benzyl group; a methoxybenzyl group; a nitrobenzyl group; a furfuryl group; a thenyl group; or a phenyl group],

(5) those wherein:

$R^1$ represents a hydrogen atom; and

$R^2$ represents a group of formula $-(CH_2)_nNR^{9a}C(=Y)NR^{6d}R^{6e}$

[in which n is 0, $R^{9a}$ represents a hydrogen atom or a methyl group, Y represents an oxygen atom or a sulphur atom, and $R^{6d}$ and $R^{6e}$ are the same or different and each represents: a $C_1$ - $C_4$ alkyl group

(such as a methyl, ethyl, propyl, isopropyl or butyl group); a $C_3$ - $C_6$ cycloalkyl group (such as a cyclopropyl, cyclobutyl, cyclopentyl or cyclohexyl group); an unsubstituted phenyl group; or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group); or $R^{6d}$ and $R^{6e}$, together with the nitrogen atom to which they are attached, represent a piperidino, piperazino, morpholino, pyrrolidino or aziridino group];

(6) those wherein:

$R^1$ represents a hydrogen atom; and

$R^2$ represents a group of formula $-(CH_2)_nNR^{9a}C(=Y)NR^{6f}NR^{6g}R^{6h}$

[in which n is 0, $R^{9a}$ represents a hydrogen atom or a methyl group, Y represents an oxygen atom or a sulphur atom, and $R^{6f}$, $R^{6g}$ and $R^{6h}$ are the same or different and each represents: a hydrogen atom; a $C_1$ - $C_4$ alkyl group (such as a methyl, ethyl, propyl, isopropyl or butyl group); a $C_3$ - $C_6$ cycloalkyl group (such as a cyclopropyl, cyclobutyl, cyclopentyl or cyclohexyl group); an unsubstituted phenyl group; or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group); or $R^{6g}$ and $R^{6h}$, together with the nitrogen atom to which they are attached, represent a piperidino, piperazino, morpholino, pyrrolidino or aziridino group; or $R^{6f}$ and $R^{6g}$, together with the nitrogen atoms to which they are attached, represent a pyrazolidinyl or tetrahydropyridazinyl group];

(7) those wherein:

$R^1$ represents a hydrogen atom; and

$R^2$ represents a group of formula $-(CH_2)_nNR^{9a}C(=Y)NR^{6j}NHZ$

{in which: n is 0; $R^{9a}$ represents a hydrogen atom or a methyl group; Y represents an oxygen atom or a sulphur atom; $R^{6j}$ represents a hydrogen atom, a $C_1$ - $C_4$ alkyl group (such as a methyl, ethyl, propyl, isopropyl or butyl group) or a $C_3$ - $C_6$ cycloalkyl group (such as a cyclopropyl, cyclobutyl, cyclopentyl or cyclohexyl group); and Z represents a group of formula $-COOR^{7a}$

[wherein $R^{7a}$ represents a $C_1$ - $C_4$ alkyl group (such as a methyl, ethyl, propyl, isopropyl or butyl group), a $C_3$ - $C_6$ cycloalkyl group (such as a cyclopropyl, cyclobutyl, cyclopentyl or cyclohexyl group) or a benzyl group],

a group of formula $-COR^{6k}$

[wherein $R^{6k}$ represents: a $C_1$ - $C_4$ alkyl group (such as a methyl, ethyl, propyl, isopropyl or butyl group); a $C_3$ - $C_6$ cycloalkyl group (such as a cyclopropyl, cyclobutyl, cyclopentyl or cyclohexyl group); an unsubstituted phenyl group; or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group]; or

a group of formula $-SO_2R^{6m}$

[wherein $R^{6m}$ represents: a $C_1$ - $C_4$ alkyl group (such as a methyl, ethyl, propyl, isopropyl or butyl group); an unsubstituted phenyl group; or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group]};

(8) those wherein:

$R^1$ represents a hydrogen atom; and

$R^2$ represents a group of formula $-(CH_2)_nNR^{9a}C(=NR^{10a})NHR^{10b}$

{in which n is 0, $R^{9a}$ represents a hydrogen atom or a methyl group, and $R^{10a}$ and $R^{10b}$ are the same or different and each represents: a hydrogen atom; a $C_1$ - $C_4$ alkyl group (such as a methyl, ethyl, propyl, isopropyl or butyl group); an unsubstituted phenyl group; a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group); a group of formula $-COOR^{7b}$

[wherein $R^{7b}$ represents a $C_1$ - $C_4$ alkyl group (such as a methyl, ethyl, propyl, isopropyl or butyl group), a $C_3$ - $C_6$ cycloalkyl group (such as a cyclopropyl, cyclobutyl, cyclopentyl or cyclohexyl group) or a benzyl group]; or

a group of formula $-COR^{6n}$

[wherein $R^{6n}$ represents a $C_1$ - $C_4$ alkyl group (such as a methyl, ethyl, propyl, isopropyl or butyl group), a $C_3$ - $C_6$ cycloalkyl group (such as a cyclopropyl, cyclobutyl, cyclopentyl or cyclohexyl group), an unsubstituted phenyl group or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group)]};

(9) those wherein:

$R^1$ represents a hydrogen atom; and

$R^2$ represents a group of formula $-(CH_2)_nNR^{9a}C(=NR^{10c})R^{6p}$

{in which n is 0; $R^{9a}$ represents a hydrogen atom or a methyl group; $R^{10c}$ represents: a hydrogen atom; a $C_1$ - $C_4$ alkyl group (such as a methyl, ethyl, propyl, isopropyl or butyl group); an unsubstituted phenyl group; a phenyl group substituted with a $C_1$ - $C_3$ alkyl, $C_1$ - $C_3$ alkoxy, halogen or nitro

substituent (such as a tolyl, methoxyphenyl, fluorophenyl or nitrophenyl group); a group of formula $-COOR^{7c}$

[wherein $R^{7c}$ represents a $C_1$ - $C_4$ alkyl group (such as a methyl, ethyl, propyl, isopropyl or butyl group), a $C_3$ - $C_6$ cycloalkyl group (such as a cyclopropyl, cyclobutyl, cyclopentyl or cyclohexyl group) or a benzyl group];

or a group of formula $-COR^{6q}$

[wherein $R^{6q}$ represents: a $C_1$ - $C_4$ alkyl group (such as a methyl, ethyl, propyl, isopropyl or butyl group); a $C_3$ - $C_6$ cycloalkyl group (such as a cyclopropyl, cyclobutyl, cyclopentyl or cyclohexyl group); an unsubstituted phenyl group; or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group)] and

$R^{6p}$ represents a $C_1$ - $C_4$ alkyl group (such as a methyl, ethyl, propyl, isopropyl or butyl group), a $C_3$ - $C_6$ cycloalkyl group (such as a cyclopropyl, cyclobutyl, cyclopentyl or cyclohexyl group), an unsubstituted phenyl group or a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group)]};

(10) those wherein:

$R^1$ represents a hydrogen atom; and

$R^2$ represents a group of formula $-(CH_2)_nNR^{9a}SO_mR^{6r}$

[in which <u>n</u> is 0, $R^{9a}$ represents a hydrogen atom or a methyl group, <u>m</u> is 1 or 2, and $R^{6r}$ represents a $C_1$ - $C_4$ alkyl group (such as a methyl, ethyl, propyl, isopropyl or butyl group), a $C_2$ - $C_4$ cyanoalkyl group (such as a cyanomethyl or cyanobutyl group), an unsubstituted phenyl group, a phenyl group substituted with a $C_1$ - $C_3$ alkyl, $C_1$ - $C_3$ alkoxy, halogen or nitro substituent (such as a tolyl, methoxyphenyl, fluorophenyl or nitrophenyl group)];

(11) those wherein:

$R^1$ represents a hydrogen atom, and

$R^2$ represents a group of formula $-CONHR^{6s}$

[in which $R^{6s}$ represents a $C_1$ - $C_4$ alkyl group (such as a methyl, ethyl, propyl, isopropyl or butyl group), a $C_3$ - $C_6$ cycloalkyl group (such as a cyclopropyl, cyclobutyl, cyclopentyl or cyclohexyl group), an unsubstituted phenyl group, a phenyl group substituted with a $C_1$ - $C_3$ alkyl, $C_1$ - $C_3$ alkoxy, halogen or nitro substituent (such as tolyl, methoxyphenyl, fluorophenyl or nitrophenyl group)];

(12) those wherein $R^2$ as defined in (2) to (11) above is attached at the <u>p</u>-position of the benzene ring,

(13) those wherein:

$R^1$ represents a hydrogen atom, and

$R^2$ represents a group of formula $-COOR^{7d}$

(in which $R^{7d}$ represents a methyl, ethyl or benzyl group);

(14) those wherein:

$R^1$ represents a methoxy group, and

$R^2$ represents a $C_1$ - $C_3$ alkoxy group (such as a methoxy, ethoxy or propoxy group) or a $C_2$ - $C_4$ alkoxyalkoxy group (such as a methoxymethoxy, ethoxymethoxy or propoxymethoxy group);

(15) those wherein $R^5$ represents an ethyl group;

(16) those wherein $R^5$ represents a mixture of an ethyl group and methyl group;

(17) those wherein $R^3$ and $R^4$ are hydrogen atoms; and

(18) those wherein X represents a hydroxy group.

Many of the compounds of the present invention will contain one or more basic nitrogen atoms and will, therefore, be capable of forming acid addition salts. There is no particular restriction on the nature of the acid employed to form the salt, provided only that, where the compound is intended to be used in human or animal therapy, the salt should be pharmaceutically acceptable, which, as is well known in the art, means that it should not have increased (or unacceptably increased) toxicity or reduced (or unacceptably reduced) activity compared with the parent compound. Where the compound is to be used for other purposes, e.g. as an intermediate in the preparation of other compounds or as an insecticidal, acaricidal or anthelmintic agent for application to non-living or non-animal matter, even this restriction does not apply. Examples of suitable acids include: inorganic acids, such as hydrochloric acid, hydrobromic acid, hydroiodic acid, phosphoric acid, sulphuric acid or nitric acid; organic sulphonic acids, such as methanesulphonic acid, ethanesulphonic acid, benzenesulphonic acid or <u>p</u>-toluenesulphonic acid; and organic carboxylic acids, such as oxalic acid, tartaric acid, citric acid, maleic acid, malonic acid, succinic acid, acetic acid, benzoic acid, mandelic acid, ascorbic acid, lactic acid, gluconic acid and malic acid.

Representative examples of the compounds of the present invention are as follows:

13-Phenethyloxymilbemycin $A_4$,

13-Phenethyloxymilbemycin $A_3$,

13-Phenethyloxymilbemycin D,
13-Deoxy-13-phenethyloxy-22,23-dihydroavermectin $B_{1a}$-aglycone,
13-(2-Phenylpropoxy)milbemycin $A_4$,
13-(1-Phenyl-1-methylethoxy)milbemycin $A_4$,
13-(2-Methoxy-2-phenylethoxy)milbemycin $A_4$,
13-[2-(4-Methylphenyl)ethoxy]milbemycin $A_4$,
13-[2-(4-Chlorophenyl)ethoxy]milbemycin $A_4$,
13-[2-(4-Fluorophenyl)ethoxy]milbemycin $A_4$,
13-[2-(4-Methoxyphenyl)ethoxy]milbemycin $A_4$,
13-[2-(4-Cyanophenyl)ethoxy]milbemycin $A_4$,
13-[2-(4-Carbamoylphenyl)ethoxy]milbemycin $A_4$,
13-[2-(4-Methoxycarbonylphenyl)ethoxy]milbemycin $A_4$,
13-[2-(2,5-Dimethylphenyl)ethoxy]milbemycin $A_4$,
13-[2-(2,6-Difluorophenyl)ethoxy]milbemycin $A_4$,
13-[2-(3,4-Dichlorophenyl)ethoxy]milbemycin $A_4$,
13-[2-(2,5-Dimethoxyphenyl)ethoxy]milbemycin $A_4$,
13-[2-(3,4-Dimethoxyphenyl)ethoxy]milbemycin $A_4$,
13-[2-(3,4-Dimethoxyphenyl)propoxy]milbemycin $A_4$,
13-[2-(3,4-Dimethoxyphenyl)ethoxy]milbemycin D,
13-Deoxy-13-[2-(3,4-dimethoxyphenyl)ethoxy]-22,23-dihydroavermectin $B_{1a}$-aglycone,
13-[2-(4-Ethoxy-3-methoxyphenyl)ethoxy]milbemycin $A_4$,
13-[2-(4-Methoxy-3-nitrophenyl)ethoxy]milbemycin $A_4$,
13-[2-(3-Methoxy-4-nitrophenyl)ethoxy]milbemycin $A_4$,
13-(2-(4-Nitrophenyl)ethoxy]milbemycin $A_4$
13-[2-(4-Aminophenyl)ethoxy]milbemycin $A_4$,
13-[2-(4-Amino-3-methoxyphenyl)ethoxy]milbemycin $A_4$,
13-[2-(3-Amino-4-methoxyphenyl)ethoxy]milbemycin $A_4$,
13-[2-(4-Formylaminophenyl)ethoxy]milbemycin $A_4$,
13-[2-(4-Acetamidophenyl)ethoxy]milbemycin $A_4$,
13-[2-(4-Acetamidophenyl)ethoxy]milbemycin $A_4$ 5-oxime,
13-[2-(4-Acetamidophenyl)ethoxy]milbemycin D,
13-(2-(4-Chloroacetamidophenyl)ethoxy]milbemycin $A_4$
13-[2-(4-Phenylacetamidophenyl)ethoxy]milbemycin $A_4$,
13-[2-(4-Phenoxyacetamidophenyl)ethoxy]milbemycin $A_4$,
13-[2-(4-Propionamidophenyl)ethoxy]milbemycin $A_4$,
13-[2-(4-Butyramidophenyl)ethoxy]milbemycin $A_4$,
13-[2-(4-Acryloylaminophenyl)ethoxy]milbemycin $A_4$,
13-[2-(4-Cyanoacetamidophenyl)ethoxy]milbemycin $A_4$,
13-[2-(4-Trifluoroacetamidophenyl)ethoxy]milbemycin $A_4$,
13-[2-(4-Cyclohexanecarbonylaminophenyl)ethoxy]milbemycin $A_4$,
13-[2-(4-Benzamidophenyl)ethoxy]milbemycin $A_4$,
13-[2-(4-Benzamidophenyl)ethoxy]milbemycin D,
13-Deoxy-13-[2-(4-benzamidophenyl)ethoxy]-22,23dihydroavermectin $B_{1a}$-aglycone,
13-[2-(4-Benzamido-3-methoxyphenyl)ethoxy]milbemycin $A_4$,
13-{2-[4-(p-Toluoylamino)phenyl]ethoxy milbemycin $A_4$,
13-{2-[4-(p-Anisoylamino)phenyl]ethoxy milbemycin $A_4$,
13-{2-[4-(4-Fluorobenzamido)phenyl]ethoxy milbemycin $A_4$,
13-{2-[4-(4-Chlorobenzamido)phenyl]ethoxy milbemycin $A_4$,
13-{2-[4-(4-Aminobenzamido)phenyl]ethoxy milbemycin $A_4$,
13-{2-[4-(4-Acetamidobenzamido)phenyl]ethoxy milbemycin $A_4$,
13-{2-[4-(4-Cyanobenzamido)phenyl]ethoxy milbemycin $A_4$,
13-{2-[4-(2-Methoxycarbonylbenzamido)phenyl]ethoxy - milbemycin $A_4$,
13-{2-[4-(2-Furoylamino)phenyl]ethoxy milbemycin $A_4$,
13-{2-[4-(2-Thenoylamino)phenyl]ethoxy milbemycin $A_4$,
13-{2-[4-(2-Pyridylcarbonylamino)phenyl]ethoxy milbemycin $A_4$,
13-{2-[4-(Nicotinoylamino)phenyl]ethoxy milbemycin $A_4$,
13-[2-(4-Piperidinocarbonylaminophenyl)ethoxy]milbemycin $A_4$,
13-[2-(4-Acetamidomethylphenyl)ethoxy]milbemycin $A_4$,

13-[2-(4-Phenylacetamidomethylphenyl)ethoxy]milbemycin $A_4$,
13-[2-(4-Benzamidomethylphenyl)ethoxy]milbemycin $A_4$,
13-[2-(4-Methoxycarbonylaminophenyl)ethoxy]milbemycin $A_4$,
13-[2-(4-Methoxycarbonylaminophenyl)ethoxy]milbemycin $A_4$ 5-oxime,
13-[2-(4-Ethoxycarbonylaminophenyl)ethoxy]milbemycin $A_4$,
13-[2-(4-Ethoxycarbonylaminophenyl)ethoxy]milbemycin D,
13-Deoxy-13-[2-(4-ethoxycarbonylaminophenyl)ethoxy]-22,23-dihydroavermectin $B_{1a}$-aglycone,
13-[2-(4-Ethoxycarbonylamino-3-methoxyphenyl)ethoxy]milbemycin $A_4$,
13-[2-(3-Ethoxycarbonylaminophenyl)ethoxy]milbemycin $A_4$,
13-[2-(2-Ethoxycarbonylaminophenyl)ethoxy]milbemycin $A_4$,
13-[2-(4-Propoxycarbonylaminophenyl)ethoxy]milbemycin $A_4$,
13-[2-(4-Isopropoxycarbonylaminophenyl)ethoxy]milbemycin $A_4$,
13-[2-(4-Butoxycarbonylaminophenyl)ethoxy)milbemycin $A_4$,
13-[2-(4-Vinyloxycarbonylaminophenyl)ethoxy]milbemycin $A_4$,
13-[2-(4-Allyloxycarbonylaminophenyl)ethoxy]milbemycin $A_4$,
13-[2-(4-Cyclohexyloxycarbonylaminophenyl)ethoxy]milbemycin $A_4$,
13-[2-(4-Benzyloxycarbonylaminophenyl)ethoxy]milbemycin $A_4$,
13-{2-[4-(4-Nitrobenzyloxycarbonylamino)phenyl]ethoxy - milbemycin $A_4$,
13-{2-[4-(4-Methoxybenzyloxycarbonylamino)phenyl]ethoxy - milbemycin $A_4$,
13-[2-(4-Methoxycarbonylaminomethylphenyl)ethoxy]milbemycin $A_4$,
13-[2-(4-Ethoxycarbonylaminomethylphenyl)ethoxy]milbemycin $A_4$,
13-[2-(4-Ethylthiocarbonylaminophenyl)ethoxy]milbemycin $A_4$
13-[2-(4-Ethylthiothiocarbonylaminophenyl)ethoxy]milbemycin $A_4$
13-[2-(4-Methanesulphonylaminophenyl)ethoxy]milbemycin $A_4$,
13-[2-(4-Ethanesulphonylaminophenyl)ethoxy]milbemycin $A_4$,
13-[2-(4-Benzenesulphonylaminophenyl)ethoxy]milbemycin $A_4$,
13-{2-[4-(p-Tosylamino)phenyl]ethoxy milbemycin $A_4$,
13-{2-[4-(3-Methylureido)phenyl]ethoxy milbemycin $A_4$,
13-{2-[4-(3-Chloromethylureido)phenyl]ethoxy milbemycin $A_4$,
13-{2-[4-(3-Methylureido)phenyl]ethoxy milbemycin D,
13-Deoxy-13-{2-[4-(3-methylureido)phenyl]ethoxy - 22,23-dihydroavermectin $B_{1a}$-aglycone,
13-{2-[4-(3-Ethylureido)phenyl]ethoxy milbemycin $A_4$,
13-{2-[4-(3-Ethylureido)-3-methoxyphenyl]ethoxy - milbemycin $A_4$,
13-{2-[4-(3-Ethylureido)-3-fluorophenyl]ethoxy - milbemycin $A_4$,
13-{2-[4-(3-Propylureido)phenyl]ethoxy milbemycin $A_4$,
13-{2-[4-(3-Isopropylureido)phenyl]ethoxy milbemycin $A_4$,
13-{2-[4-(3-Butylureido)phenyl]ethoxy milbemycin $A_4$,
13-{2-[4-(3-Benzylureido)phenyl]ethoxy milbemycin $A_4$,
13-{2-[4-(3-Methoxycarbonylmethylureido)phenyl]ethoxy - milbemycin $A_4$,
13-{2-[4-(3-Cyclohexylureido)phenyl]ethoxy milbemycin $A_4$,
13-{2-[4-(3-Allylureido)phenyl]ethoxy milbemycin $A_4$,
13-{2-[4-(3-Phenylureido)phenyl]ethoxy milbemycin $A_4$,
13-{2-[4-(3-Phenylureido)-3-methoxyphenyl]ethoxy - milbemycin $A_4$,
13-{2-[4-(3-p-Fluorophenylureido)phenyl]ethoxy milbemycin $A_4$,
13-{2-[4-(3-o-Fluorophenylureido)phenyl]ethoxy milbemycin $A_4$,
13-{2-[4-(3-p-Nitrophenylureido)phenyl]ethoxy milbemycin $A_4$,
13-{2-[4-(3-p-Methoxyphenylureido)phenyl]ethoxy milbemycin $A_4$,
13-{2-[4-(3-p-Aminophenylureido)phenyl]ethoxy milbemycin $A_4$,
13-{2-[4-(3-p-Acetamidophenylureido)phenyl]ethoxy - milbemycin $A_4$,
13-{2-[4-(3-Methylureidomethyl)phenyl]ethoxy milbemycin $A_4$,
13-{2-[4-(3-Phenylureidomethyl)phenyl]ethoxy milbemycin $A_4$,
13-{2-[4-(3-Methylthioureido)phenyl]ethoxy milbemycin $A_4$,
13-{2-[4-(3-Ethylthioureido)phenyl]ethoxy milbemycin $A_4$,
13-{2-[4-(3-Phenylthioureido)phenyl]ethoxy milbemycin $A_4$,
13-[2-(4-Acetimidoylaminophenyl)ethoxy]milbemycin $A_4$,
13-{2-[4-(Propanesulphonylamino)phenyl]ethoxy milbemycin $A_4$,
13-{2-[4-(Isopropanesulphonylamino)phenyl]ethoxy milbemycin $A_4$,
13-{2-[4-(Isonicotinoylamino)phenyl]ethoxy milbemycin $A_4$,

13-{2-[4-(Methoxyacetamido)phenyl]ethoxy milbemycin $A_4$,
13-{2-[4-(Fluoroacetamido)phenyl]ethoxy milbemycin $A_4$,
13-{2-[4-(Difluoroacetamido)phenyl]ethoxy milbemycin $A_4$,
13-{2-[4-(2-Cyanopropionamido)phenyl]ethoxy milbemycin $A_4$,
13-{2-[4-(Methoxalylamino)phenyl]ethoxy milbemycin $A_4$,
13-{2-[4-(Pyruvoylamino)phenyl]ethoxy milbemycin $A_4$,
13-{2-[4-(Methoxycarbonylacetamido)phenyl]ethoxy - milbemycin $A_4$,
13-{2-[4-(Ethoxycarbonylacetamido)phenyl]ethoxy - milbemycin $A_4$,
13-{2-[4-(Cyclopropylcarbonylamino)phenyl]ethoxy milbemycin $A_4$,
13-{2-[4-(Cyclobutylcarbonylamino)phenyl]ethoxy milbemycin $A_4$,
13-{2-[4-(Cinnamoylamino)phenyl]ethoxy milbemycin $A_4$,
13-{2-[4-(Methacryloylamino)phenyl]ethoxy milbemycin $A_4$,
13-{2-[4-(Tetroloylamino)phenyl]ethoxy milbemycin $A_4$,
13-{2-[4-(Pyrazin-2-ylcarbonylamino)phenyl]ethoxy - milbemycin $A_4$,
13-{2-[4-(3,4-Dihydro-2H-pyran-2-ylcarbonylamino)phenyl]ethoxy milbemycin $A_4$,
13-{2-[4-(4-Methoxycarbonylbenzoylamino)phenyl]ethoxy - milbemycin $A_4$,
13-{2-[4-(1-t-Butoxycarbonylpiperidin-4-ylcarbonylamino)phenyl]ethoxy milbemycin $A_4$,
13-{2-[4-(Glycylamino)phenyl]ethoxy milbemycin $A_4$,
13-{2-[4-(N-Acetylglycylamino)phenyl]ethoxy milbemycin $A_4$,
13-{2-[4-(Ethoxycarbonylglycylamino)phenyl]ethoxy - milbemycin $A_4$,
13-{2-{4-[(3-Methylureido)acetamido]phenyl ethoxy - milbemycin $A_4$,
13-{2-{4-[(3-Phenylureido)acetamido]phenyl ethoxy - milbemycin $A_4$,
13-[2-(4-Ethoxycarbonylamino-3-methoxyphenyl)ethoxy]milbemycin $A_4$,
13-[2-(4-Butanesulphonylaminophenyl)ethoxy]milbemycin $A_4$,
13-[2-(4-Cyanomethanesulphonylaminophenyl)ethoxy]milbemycin $A_4$,
13-{2-[4-(4-Methoxybenzenesulphonylamino)phenyl]ethoxy - milbemycin $A_4$,
13-{2-[4-(1,2,4-triazolo[4.3-a]pyridin-3-on-2-ylcarbonylamino)phenyl]ethoxy milbemycin $A_4$,
13-{2-{4-[3-(2-Chloroethyl)ureido]phenyl ethoxy - milbemycin $A_4$,
13-{2-[p-3-(2-Hydroxyethyl)ureidophenyl]ethoxy milbemycin $A_4$,
13-[2-(4-Ureidophenyl)ethoxy]milbemycin $A_4$,
13-{2-[4-(3,3-Dimethylureido)phenyl]ethoxy milbemycin $A_4$,
13-{2-[p-3-(2-Mercaptoethyl)ureidophenyl]ethoxy milbemycin $A_4$,
13-{2-[4-(3-Cyclopropylureido)phenyl]ethoxy milbemycin $A_4$,
13-{2-[p-3-(2-Pyridyl)ureidophenyl]ethoxy milbemycin $A_4$,
13-{2-[p-3-(2-Thiazolinyl)ureidophenyl]ethoxy milbemycin $A_4$,
13-{2-[p-3-(2-Thiazolyl)ureidophenyl]ethoxy milbemycin $A_4$,
13-{2-[4-(3-Propionylureido)phenyl]ethoxy milbemycin $A_4$,
13-{2-[4-(3-Benzoylureido)phenyl]ethoxy milbemycin $A_4$,
13-{2-[4-(3-Methanesulphonylureido)phenyl]ethoxy milbemycin $A_4$,
13-[2-[4-Morpholinocarbonylaminophenyl)ethoxy]milbemycin $A_4$,
13-[2-[4-Carbazoylaminophenyl)ethoxy]milbemycin $A_4$,
13-{2-[4-(2-Methylcarbazoylamino)phenyl]ethoxy milbemycin $A_4$,
13-{2-[4-(3,3-Dimethylcarbazoylamino)phenyl]ethoxy - milbemycin $A_4$,
13-{2-[4-(3-Phenylcarbazoylamino)phenyl]ethoxy milbemycin $A_4$,
13-{2-[p-3-(2-Pyridyl)carbazoylaminophenyl]ethoxy - milbemycin $A_4$,
13-{2-[4-(3-Acetylcarbazoylamino)phenyl]ethoxy milbemycin $A_4$,
13-{2-[4-(3-Benzoylcarbazoylamino)phenyl]ethoxy milbemycin $A_4$,
13-{2-[4-(3-Morpholinoureido)phenyl]ethoxy milbemycin $A_4$,
13-{2-[p-3-(Hexahydro-1H-azepin-1-yl)ureidophenyl]ethoxy - milbemycin $A_4$,
13-[2-(4-Formimidoylaminophenyl)ethoxy]milbemycin $A_4$,
13-[2-(4-Benzimidoylaminophenyl)ethoxy]milbemycin $A_4$,
13-{2-[p-3-(Methoxycarbonyl)guanidinophenyl]ethoxy - milbemycin $A_4$,
13-{2-[p-2,3-Bis(methoxycarbonyl)guanidinophenyl]ethoxy - milbemycin $A_4$,
13-[2-(4-Benzenesulphinylaminophenyl)ethoxy]milbemycin $A_4$,
13-{2-[p-(N-Ethoxycarbonyl)methylaminophenyl]ethoxy - milbemycin $A_4$,
13-{2-[p-N-(4-Methylphenyl)carbamoylphenyl]ethoxy - milbemycin $A_4$,

Of these, the preferred compounds are as follows:

13-[2-(4-Acetamidophenyl)ethoxy]milbemycin $A_4$

13-[2-(4-Cyanoacetamidophenyl)ethoxy]milbemycin $A_4$
13-{2-[4-(2-Cyanopropionamido)phenyl]ethoxy}milbemycin $A_4$
13-[2-(4-Methoxyacetamidophenyl)ethoxy]milbemycin $A_4$
13-{2-[4-(Cyclopropylcarbonylamino)phenyl]ethoxy}milbemycin $A_4$
13-{2-[4-(Cyclobutylcarbonylamino)phenyl]ethoxy}milbemycin $A_4$
13-{2-[4-(4-Cyanobenzamido)phenyl]ethoxy}milbemycin $A_4$
13-[2-(4-Methoxycarbonylaminophenyl)ethoxy]milbemycin $A_4$
13-[2-(4-Vinyloxycarbonylaminophenyl)ethoxy]milbemycin $A_4$
13-{2-[4-(3-Methylureido)phenyl]ethoxy}milbemycin $A_4$
13-{2-[4-(3-Phenylureido)phenyl]ethoxy}milbemycin $A_4$
13-{2-[4-(3-Cyclohexylureido)phenyl]ethoxy}milbemycin $A_4$
13-[2-(4-Methanesulphonylaminophenyl)ethoxy]milbemycin $A_4$
13-[2-(4-Ethanesulphonylaminophenyl)ethoxy]milbemycin $A_4$
13-{2-[4-(3,3-Dimethylcarbazoylamino)phenyl]ethoxy}milbemycin $A_4$
13-{2-[4-(3-o-Fluorophenylureido)phenyl]ethoxy}milbemycin $A_4$
13-{2-[4-(3-p-Fluorophenylureido)phenyl]ethoxy}milbemycin $A_4$
13-{2-[4-(3-p-Methoxyphenylureido)phenyl]ethoxy}milbemycin $A_4$ Also preferred are salts, where available, of the above compounds.

The compounds of the present invention may be prepared by a variety of processes known in the art for the preparation of compounds of this type. In general terms a suitable preparative procedure comprises reacting a compound of formula (IV):

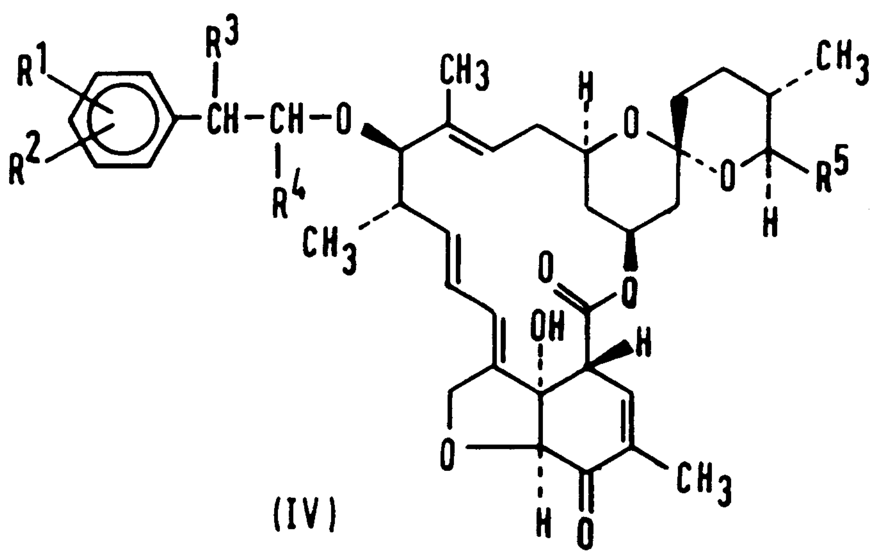

(IV)

(in which $R^1$, $R^2$, $R^3$, $R^4$ and $R^5$ are as defined above) with a reducing agent to prepare a compound of formula (I) in which X represents a hydroxy group and, if required, acylating said compound to prepare a compound of formula (I) in which X represents said alkanoyloxy group, or by reacting said compound of formula (IV) with hydroxylamine or a salt thereof to prepare a compound of formula (I) in which X represents a hydroxyimino group.

The compound of formula (IV), which is the starting material referred to above, may be prepared by reacting a compound of formula (III):

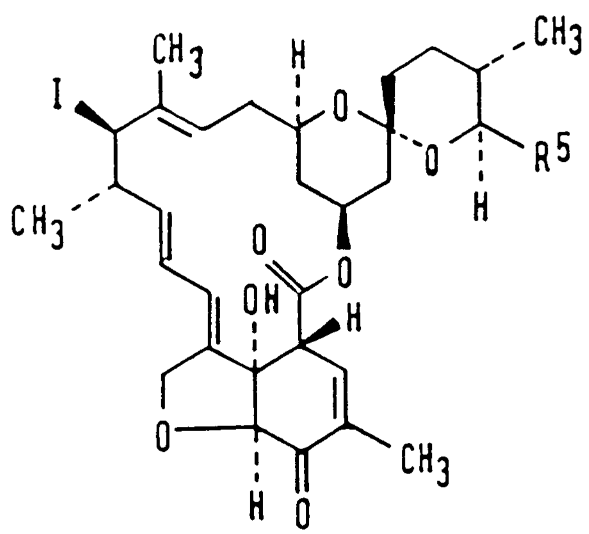

(III)

(in which $R^5$ is as defined above) with a compound of formula (III'):

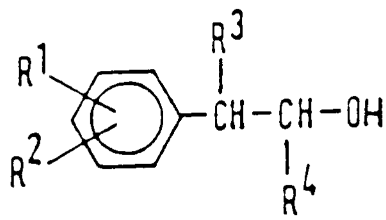

(III')

(in which $R^1$, $R^2$, $R^3$ and $R^4$ are as defined above).

In more detail, the compounds of formula (I) of the present invention can be prepared from a 13-iodomilbemycin of formula (III) as shown in the following Reaction Scheme A:

Reaction Scheme A:

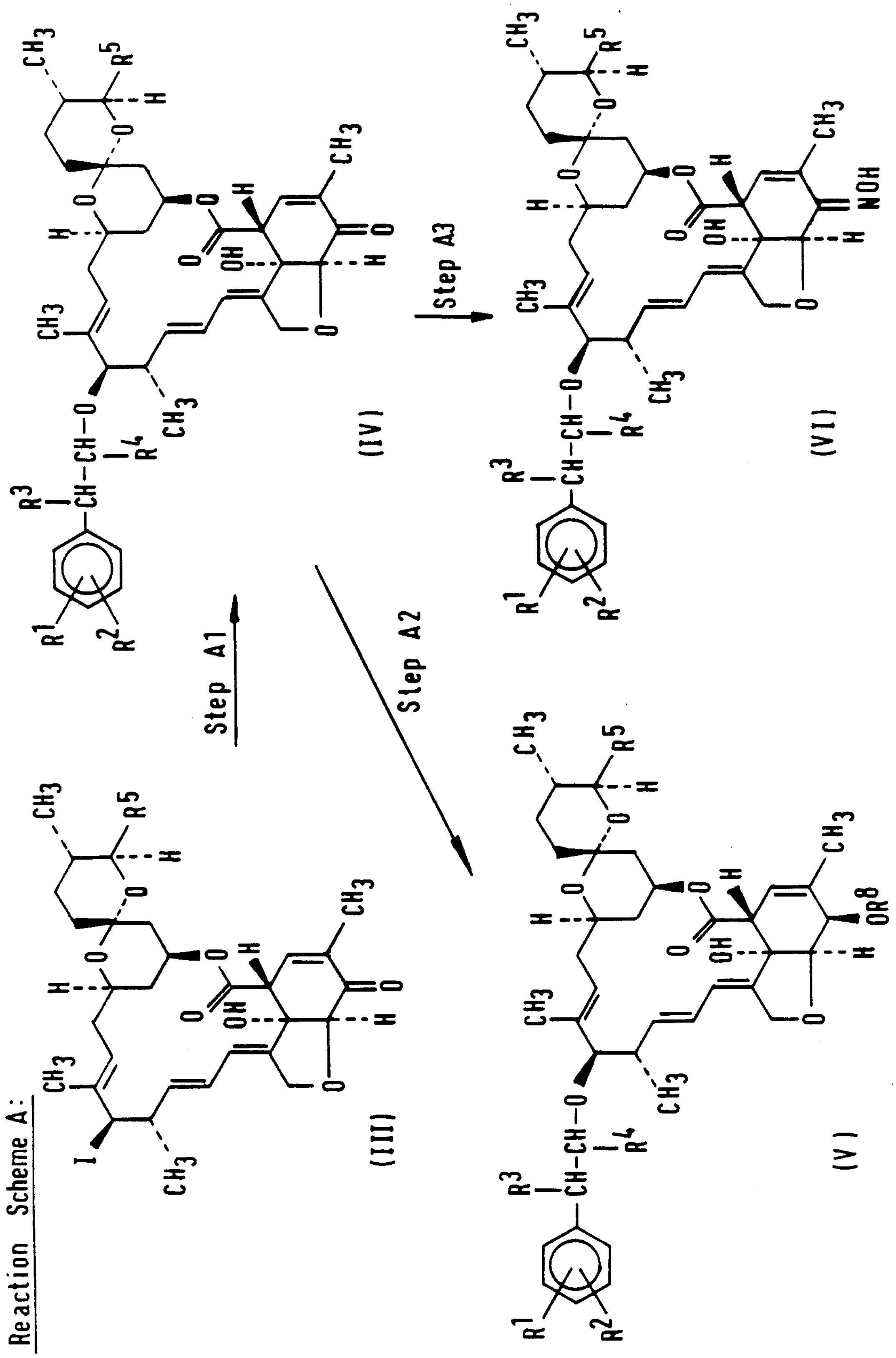


In the above formulae, $R^1$, $R^2$, $R^3$, $R^4$ and $R^5$ are as defined above and $R^8$ represents a hydrogen atom or a $C_1$ - $C_5$ alkanoyl group or substituted $C_1$ - $C_5$ alkanoyl group having at least one of substituents (d), defined above (i.e. the alkanoyl groups defined above for the alkanoyloxy groups of X).

In Step A1, a compound of formula (IV) is prepared by reacting a compound of formula (III) with a phenethyl alcohol of formula (III'):

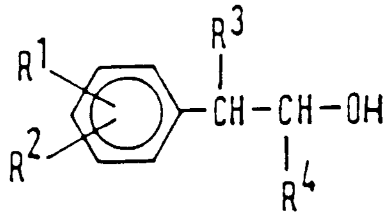

(III')

in the presence of a catalyst. Any catalyst capable of catalysing such etherification reactions, as are well known in the art, may equally be employed in this reaction, without any particular restriction. Examples of suitable catalysts include oxides and salts of mercury or silver, preferably a silver compound such as silver oxide, silver perchlorate or silver trifluoromethanesulphonate, or a mercury compound such as mercury oxide, mercury iodide, mercury bromide or mercury trifluoromethanesulphonate.

In certain cases, the reaction may be accelerated by addition of an acid-binding agent, There is no particular limitation on the nature of such an acid-binding agent, provided that it has no adverse effect on the reaction, but 2,6-lutidine and calcium carbonate are preferred examples.

There is no particular limitation on the nature of the solvent employed in the reaction, provided that it has no adverse effect on the reaction and that it is capable of solubilizing the starting compound, at least to some extent. Phenethyl alcohol itself can be employed as the solvent, but preferred solvents include: aromatic hydrocarbons, such as benzene, toluene or xylene; halogenated hydrocarbons, especially halogenated aliphatic hydrocarbons, such as methylene chloride, 1,2-dichloroethane or chloroform; esters, such as ethyl acetate or propyl acetate; ethers, such as diethyl ether, tetrahydrofuran, dioxane or dimethoxyethane; amides, such as dimethylformamide, dimethylacetamide or hexamethylphosphoric triamide; and sulphoxides, such as dimethyl sulphoxide.

The reaction can take place over a wide range of temperatures, and the precise reaction temperature is not critical to the invention. In general, we find it convenient to carry out the reaction at a temperature from -10 °C to 100 °C, preferably from 0 °C to 50 °C. The time required for the reaction may also vary widely, depending on many factors, notably the reaction temperature and the nature of the reagents and of the solvent. However, provided that the reaction is effected under the preferred conditions outlined above, a period of from 1 hour to 2 days will usually suffice.

After completion of the reaction, the reaction product may be recovered from the reaction mixture by conventional means. For example, the reaction mixture may be diluted with a water-immiscible organic solvent, after which the insoluble materials are removed by filtration, if necessary. The filtrate may then be washed successively with an aqueous solution of potassium iodide, an acid and water, and the solvent may be removed by distillation to afford the desired product. The product may, if required, be further purified by such conventional techniques as recrystallization or the various chromatography techniques, notably column chromatography.

In Step A2, a compound of formula (V) is prepared by reducing the carbonyl group at the 5-position of the compound of formula (IV) to a hydroxy group, which, if required, may then be subjected to acylation to give a compound of formula (V) in which $R^8$ represents an optionally substituted alkanoyl group. There is no particular limitation on the nature of the reducing agent to be used in this reduction, provided that it can reduce the carbonyl group and has no adverse effect on the other functional groups in the compound of formula (IV). Such reducing agents include, for example, hydride agents, such as sodium borohydride or diborane, preferably sodium borohydride.

There is equally no particular limitation on the nature of the solvent, provided that it has no adverse effect on the reaction, but a lower alcohol (such as methanol, ethanol or propanol) is preferably used when sodium borohydride is employed as the reducing agent.

The reaction can take place over a wide range of temperatures, and the precise reaction temperature is not critical to the invention. In general, we find it convenient to carry out the reaction at a temperature from 0 °C to 50 °C. The time required for the reaction may also vary widely, depending on many factors, notably the reaction temperature and the nature of the reagents. However, provided that the reaction is effected under the preferred conditions outlined above, a period of from 5 minutes to 2 hours will usually suffice.

After completion of the reaction, the reaction product can be recovered easily from the reaction mixture by conventional means. For example, the reaction mixture may be diluted with a water-immiscible organic solvent and washed with water, after which the solvent may be removed by distillation to afford the desired product. The product may, if required, be further purified by such conventional techniques as recrystallization or the various chromatography techniques, notably column chromatography.

The reduction product thus prepared may, if required, be acylated in an inert solvent using as the acylating agent an acid corresponding to the optionally substituted alkanoyl group which it is desired to introduce or using a reactive derivative of such an acid. The reaction can be carried out using conventional esterification techniques. Examples of suitable active derivatives of the acid include any of those commonly used for esterification such as acid halides (e.g. an acid chloride or acid bromide), acid anhydrides, mixed acid anhydrides, reactive esters (e.g. the N-hydroxybenztriazole ester) and reactive amides (e.g. the imidazolide).

Where the acid itself is employed, a dehydrating agent (such as dicyclohexylcarbodiimide, p-toluenesulphonic acid or sulphuric acid) is preferably also used. Where a reactive derivative of an acid is employed, an acid-binding agent is preferably also employed. There is no particular limitation on the nature of the acid-binding agent to be used, provided that it has the ability to bind to an acid and, hence, effectively eliminate it from the reaction system. Examples include: organic amines, such as triethylamine, N,N-diethylaniline, pyridine, 4-dimethylaminopyridine and 1,8-diazabicyclo[5.4.0]undecene-7.

There is no particular restriction on the nature of the solvent to be employed, provided that it has no adverse effect on the reaction or on the reagents involved. Examples of suitable solvents include: hydrocarbons, which may be aliphatic, aromatic or cycloaliphatic, such as hexane, benzene, toluene or xylene; halogenated hydrocarbons, especially halogenated aliphatic hydrocarbons, such as methylene chloride, 1,2-dichloroethane or chloroform; esters, such as ethyl acetate or propyl acetate; and ethers, such as diethyl ether, tetrahydrofuran, dioxane or dimethoxyethane.

After completion of the reaction, the reaction product can easily be recovered from the reaction mixture by conventional means. For example, the reaction mixture may be diluted with a water-immiscible organic solvent and washed successively with an acid, an alkali and water, after which the solvent may be removed by distillation to afford the desired product. The product may, if required, be further purified by such conventional techniques as recrystallization or the various chromatography techniques, notably column chromatography.

In Step A3 a compound of formula (VI) is prepared by oximation at the 5-position of the compound of formula (IV) with hydroxylamine or with a salt thereof (e.g. a salt with a mineral acid such as hydrochloric acid, nitric acid or sulphuric acid).

The reaction is usually carried out in an inert solvent, the nature of which is not critical, provided that it has no adverse effect on the reaction or on the reagents involved. Examples of suitable solvents include: alcohols, such as methanol or ethanol; ethers, such as tetrahydrofuran or dioxane; aliphatic acids, such as acetic acid; or a mixture of water with any one or more of these solvents.

The reaction can take place over a wide range of temperatures, and the precise reaction temperature is not critical to the invention. In general, we find it convenient to carry out the reaction at a temperature from 10°C to 80°C. The time required for the reaction may also vary widely, depending on many factors, notably the reaction temperature and the nature of the reagents. However, provided that the reaction is effected under the preferred conditions outlined above, a period of from 1 to 24 hours will usually suffice.

After completion of the reaction, the reaction product can easily be recovered from the reaction mixture by conventional means. For example, the reaction mixture may be diluted with a water-immiscible organic solvent and washed with water, after which the solvent may be removed by distillation to afford the desired product. The product may, if required, be further purified by such conventional techniques as recrystallization or the various chromatography techniques, notably column chromatography.

The compound of formula (V) wherein $R^1$ is a substituted amino group can be prepared as illustrated in the following Reaction Scheme B:

Reaction Scheme B:

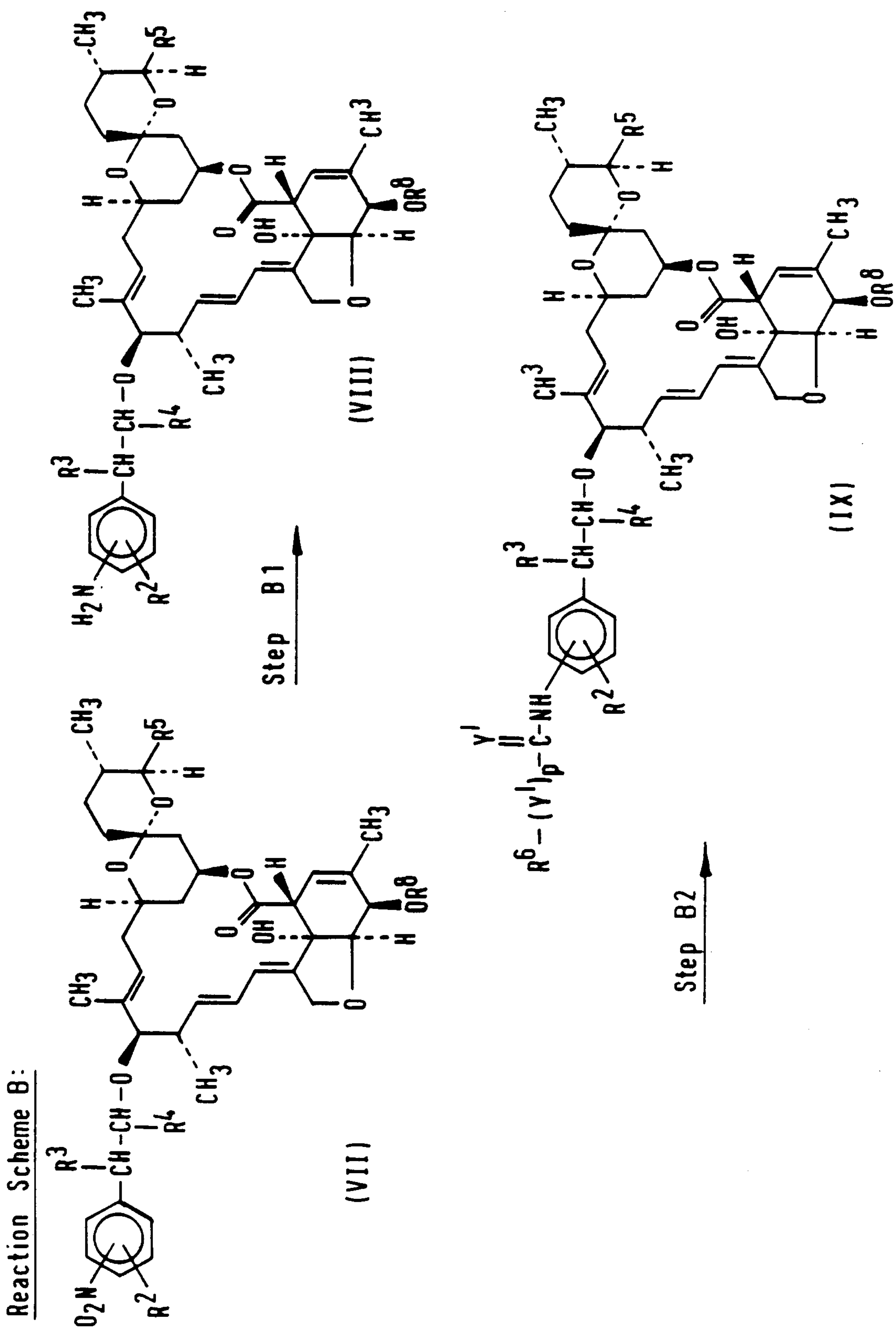


(VII) (VIII) (IX)

In the above formulae, $R^2$, $R^3$, $R^4$, $R^5$, $R^6$ and $R^8$ are as defined above, Y' represents an oxygen atom, a sulphur atom or an imino group, and p represents 0 or 1.

In Step B1 a compound of formula (VIII) is prepared by reducing the nitro group of a compound of formula (VII) to give an amino group. This may be effected by a conventional reducing method for reducing a nitro group to an amino group. One such method is catalytic reduction using a precious metal catalyst.

Examples of catalysts which are preferably employed include such conventional catalysts as palladium-on-carbon, palladium-on-barium sulphate and platinum oxide.

The reaction is normally and preferably effected in the presence of a solvent, and there is no particular restriction on the nature of the solvent to be employed, provided that it has no adverse effect on the reaction or on the reagents involved. Examples of suitable solvents include: alcohols, such as methanol or ethanol; ethers, such as tetrahydrofuran or dioxane; and esters, such as ethyl acetate.

The reaction can take place over a wide range of temperatures, and the precise reaction temperature is not critical to the invention. In general, we find it convenient to carry out the reaction at a temperature from 10°C to 80°C. The time required for the reaction may also vary widely, depending on many factors, notably the reaction temperature and the nature of the reagents. However, provided that the reaction is effected under the preferred conditions outlined above, a period of from 10 minutes to 5 hours will usually suffice.

An alternative preferred reducing method is reduction with zinc powder in acetic acid. This reaction is preferably carried out at a temperature ranging from 0°C to room temperature, and the reaction time is usually in the range of from 10 minutes to 2 hours.

After completion of the reaction, the reaction product can easily be recovered from the reaction mixture by conventional means. For example, the reaction mixture may be diluted with a water-immiscible organic solvent, and the insoluble materials, if necessary, removed by filtration. The filtrate may then be washed with water, and the solvent may be removed by distillation to afford the desired product. The product may, if required, be further purified by such conventional techniques as recrystallization or the various chromatography techniques, notably column chromatography.

In Step B2 a compound of formula (IX) is prepared by reacting the compound of formula (VIII) with a reagent that is reactive with the amino group, to introduce the group of formula $R^6$-$(Y')_n$-C(=Y')-NH-.

The nature of the reagent to be employed will, of course, be dictated by the nature of the group which it is desired to introduce. However, in general, it may be a reactive derivative of a carboxylic acid of the type commonly used as an acylating agent such as an acid halide, an acid anhydride, a mixed acid anhydride, a reactive ester or a reactive amide. Alternatively, it may be: a chloroformate, such as methyl chloroformate or benzyl chloroformate; a thiochloroformate, such as ethyl chlorothioformate; a sulphonyl chloride, such as methanesulphonyl chloride or benzenesulphonyl chloride; an isocyanate; a thioisocyanate; or an imino ether. Alternatively, a carboxylic acid may be used as such, provided that it is activated, for example with dicyclohexylcarbodiimide.

When a halide, such as an acid halide, is employed as the reagent, it is usually preferred to carry out the reaction in the presence of an organic base, such as triethylamine, N,N-diethylaniline, pyridine, 4-dimethylaminopyridine or 1,8-diazabicyclo[5.4.0]undecene, as an acid-binding agent.

The reaction can take place over a wide range of temperatures, and the precise reaction temperature is not critical to the invention. In general, we find it convenient to carry out the reaction at a temperature from 0°C to 80°C, preferably from 0°C to room temperature. The time required for the reaction may also vary widely, depending on many factors, notably the reaction temperature and the nature of the reagents. However, provided that the reaction is effected under the preferred conditions outlined above, a period of from 10 minutes to 10 hours will usually suffice.

After completion of the reaction, the reaction product can easily be recovered from the reaction mixture by conventional means. For example, the reaction mixture may be diluted with a water-immiscible organic solvent, and the insoluble materials may then be removed, if required, by filtration and washed with water, after which the solvent may be removed by distillation to afford the desired product. The product may, if required, by further purified by such conventional techniques as recrystallization or the various chromatography techniques, notably column chromatography.

The compound of formula (III), which is used as the starting material can advantageously be synthesized from 13-hydroxy-5-oxomilbemycin, which is represented by the general formula (X), as illustrated in the following Reaction Scheme C:

Reaction Scheme C:

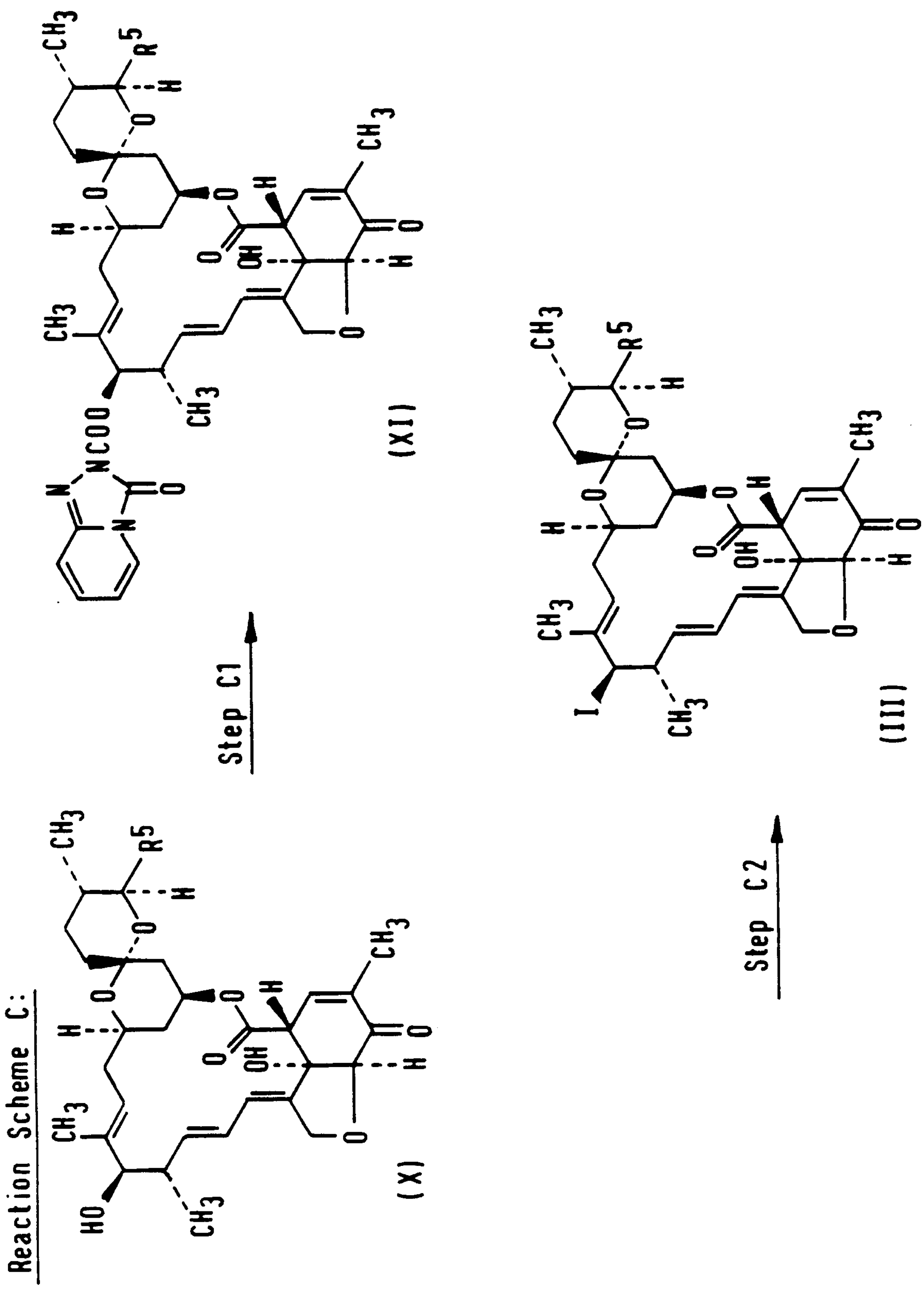


(X) (XI) (III)

In the above formulae, $R^5$ is as defined above.

In Step C1 a compound of formula (XI) is prepared by reacting the compound of formula (X) with 2-chloroformyl-1,2,4-triazolo[4.3a]pyridin-3-one in the presence of an acid-binding agent.

There is no particular limitation on the nature of the acid-binding agent to be employed provided that it has the ability to bind to and, hence, effectively eliminate from the reaction system any acid produced. For

example, an organic amine, such as triethylamine, N,N-diethylaniline, pyridine, 4-dimethylaminopyridine or 1,8-diazabicyclo[5.4.0]undecene, may be used.

The reaction is also preferably effected in the presence of an inert solvent, the nature of which is not critical, provided that it has no adverse effect on the reaction or on the reagents involved. Examples of suitable solvents include: hydrocarbons, which may be aliphatic, aromatic or cycloaliphatic, such as hexane, benzene, toluene or xylene; halogenated hydrocarbons, especially halogenated aliphatic hydrocarbons, such as methylene chloride, 1,2-dichloroethane or chloroform; esters, such as ethyl acetate or propyl acetate; and ethers, such as diethyl ether, tetrahydrofuran, dioxane or dimethoxyethane.

The reaction can take place over a wide range of temperatures, and the precise reaction temperature is not critical to the invention. In general, we find it convenient to carry out the reaction at a temperature from 0°C to 50°C. The time required for the reaction may also vary widely, depending on many factors, notably the reaction temperature and the nature of the reagents. However, provided that the reaction is effected under the preferred conditions outlined above, a period of from 5 minutes to 2 hours will usually suffice.

After completion of the reaction, the reaction product can easily be recovered from the reaction mixture by conventional means. For example, the reaction mixture may be diluted with a water-immiscible organic solvent, the insoluble materials may then be removed, if required, by filtration and washed successively with an aqueous solution of potassium iodide, an acid and water, after which the solvent may be removed by distillation to afford the desired product.

In Step C2 13-iodomilbemycin, which is represented by formula (III), is prepared by reacting the compound of formula (XI) with zinc iodide.

This reaction is usually carried out in a solvent. There is no particular restriction on the nature of the solvent to be employed, provided that it has no adverse effect on the reaction or on the reagents involved. Examples of suitable solvents include: hydrocarbons, which may be aliphatic, aromatic or cycloaliphatic, such as hexane, benzene, toluene or xylene; halogenated hydrocarbons, especially halogenated aliphatic hydrocarbons, such as methylene chloride, 1,2-dichloroethane or chloroform; esters, such as ethyl acetate or propyl acetate; and ethers, such as diethyl ether, tetrahydrofuran, dioxane or dimethoxyethane.

The reaction can take place over a wide range of temperatures, and the precise reaction temperature is not critical to the invention. In general, we find it convenient to carry out the reaction at a temperature from 0°C to room temperature. The time required for the reaction may also vary widely, depending on many factors, notably the reaction temperature and the nature of the reagents. However, provided that the reaction is effected under the preferred conditions outlined above, a period of from 10 minutes to 2 hours will usually suffice.

After completion of the reaction, the reaction product can easily be recovered from the reaction mixture by conventional means. For example, the insoluble materials may be removed by filtration and the filtrate washed with water, after which the solvent may be removed by distillation to afford the desired product. The product may, if required, be further purified by such conventional techniques as recrystallization or the various chromatography techniques, notably column chromatography.

The compound of formula (X), which is, therefore, the ultimate starting material for the above sequence of reactions, can be prepared from the natural or semisynthetic milbemycins or avermectins by the method disclosed in Japanese Patent Application Kokai No. Sho 61-103884.

The milbemycins and analogous natural products are generally obtained as mixtures at various ratios of related compounds, and they may be reacted after being separated into the various fractions or they may be used as mixtures, whether the natural mixture or an artificially produced mixture. Therefore, the compound used in each step of the above reactions may be either a single compound or a mixture of compounds. Accordingly, the compound of formula (I) may be prepared as a single compound or as a mixture of compounds, for example as a mixture of derivatives of milbemycins $A_3$ and $A_4$.

The compounds of the invention have a strong acaricidal activity against, for example, adults, imagos and eggs of Tetranychus, Panonychus (e.g. Panonychus ulmi and Panonychus citri), Aculopa pelekassi and rust mites, which are parasitic to fruit trees, vegetables and flowers. They are also active against Ixodidae, Dermanyssidae and Sarcoptidae, which are parasitic to animals. Surprisingly, they have a strong activity even against acarids resistant to the known acaricides, which have recently started to become a great problem. Further, they are active against: ectoparasites, such as Oestrus, Lucilia, Hypoderma, Gautrophilus, lice and fleas, which are parasitic to animals and birds, particularly livestock and poultry; domestic insects, such as cockroaches and houseflies; and various harmful insects in agricultural and horticultural areas, such as aphids and larval Lepidoptera. They are also effective against Meloidogyne in the soil, Bursaphelenchus and Rhizoglyphus, and against insects of the orders Coleoptera, Homoptera, Heteroptera, Diptera, Thysanoptera, Orthoptera, Anoplura, Siphonaptera, Mallophage, Thysanura, Isoptera, Psocoptera, and Hymenoptera.

The compounds of the invention equally can be used to control other plant-damaging insects, particularly insects that damage plants by eating them. The compounds can be used to protect both ornamental plants and productive plants, particularly cotton (e.g. against Spodoptera littoralis and Heliothis virescens), as well as vegetable crops (e.g. against Leptinotarsa decemlineata and Myzus persicae) and rice crops (e.g. against Chilo suppressalis and Laodelphax).

Accordingly, the compounds of the invention can be used to treat all manner of plants (as well as the seeds from which such plants are grown and the environment, whether for growth or storage, containing such plants) to protect them from insects such as those exemplified above. Such plants include cereals (e.g. maize or rice), vegetables (e.g. potatoes or soybeans), fruits and other plants (e.g. cotton).

The compounds of the invention can similarly be used to protect animals from a variety of ectoparasites, by applying the compounds to the animals or to the animals' environment, e.g. livestock housing, animal boxes, abattoirs, pasture land and other grasslands, as well as to any other places liable to be infested. The compounds may also be applied to external parts of the animals, preferably before they are infested.

Moreover, the compounds of the invention are effective against various parasitical helminths. These parasites can attack livestock, poultry and pet animals (such as pigs, sheep, goats, cows, horses, dogs, cats and fowl) and can cause grave economic damage. Among the helminths, the nematodes in particular often cause serious infection. Typical genera of nematodes which are parasitic on these animals and against which the compounds of the invention are effective include:

Haemonchus,
Trichostrongylus,
Ostertagia,
Nematodirus,
Cooperia,
Ascaris,
Bunostomum,
Oesophagostomum,
Chabertia,
Trichuris,
Strongylus,
Trichonema,
Dictyocaulus,
Capillaria,
Heterakis,
Toxocara,
Ascaridia,
Oxyuris,
Ancylostoma,
Uncinaria,
Toxascaris and
Parascaris.

Certain parasitical species of the genera Nematodirus, Cooperia and Oesophagostomum attack the intestines, while certain species of the genera Haemonchus and Ostertagia parasitize the stomach, and parasites belonging to the genus Dictyocaulus are found in the lungs. Parasites belonging to the families Filariidae and Setariidae are found in internal tissues and organs, for example, the heart, the blood vessels, the subcutaneous tissues and the lymphatic vessels. The compounds of the invention are active against all these parasites.

The compounds of the invention are also effective against parasites which infect humans. Typical of the parasites which may most commonly be found in the digestive tracts of human beings are parasites of the genera:

Ancylostoma,
Necator,
Ascaris,
Strongyloides,
Trichinella,
Capillaria,
Trichuris and
Enterobius.

The compounds are also active against parasites of the genera Wuchereria, Brugia, Onchocerca and Loa of the family Filariidae (which are found in blood, tissues and organs other than the digestive tract and are medically important), parasites of the genus Dracunculus of the family Dracunculidae and parasites of the genera Strongyloides and Trichinella, which in a particular state may parasitize outside the intestinal tract, although they are essentially intestinal parasites.

The form of compositions of the invention and the nature of the carriers or diluents employed in them will vary depending upon the intended use of the composition. For example, where the compounds of the invention are to be employed as anthelmintics, they are preferably administered orally, parenterally or topically and the form of the composition chosen will be appropriate to the intended route of administration.

For oral administration, the composition of the invention is preferably in the form of a liquid drink comprising a non-toxic solution, suspension or dispersion of the active compound in admixture with a suspending agent (such as bentonite), a wetting agent or other diluents, preferably in water or another non-toxic solvent. The drink, in general, also contains an anti-foaming agent. The active compound would normally be present in the drink in an amount of from 0.01 to 0.5% by weight, more preferably from 0.01 to 0.1% by weight.

Compositions for oral administration may also be in the form of dry solids, preferably in unit dosage form, such as capsules, pills or tablets containing the desired amount of the active compound. These compositions may be prepared by mixing the active compound uniformly with suitable diluents, fillers, disintegrators and/or binding agents, for example starch, lactose, talc, magnesium stearate and vegetable gum. The weight and contents of the preparation will vary widely, depending upon the nature of the animal to be treated, the degree of infection, the nature of the parasite and the body weight of the animal to be treated.

The compounds may also be administered as an additive to animal feedstuffs, in which case they may be dispersed uniformly in the feedstuffs, used as a top dressing or used in the form of pellets. The content of active compound in the feedstuff is preferably from 0.0001 to 0.02%, in order to achieve the desired anthelmintic activity.

For parenteral administration, the compound of the invention is preferably dissolved or suspended in a liquid vehicle, preferably a vegetable oil, such as peanut oil or cottonseed oil. Where the compound is a salt of a compound of formula (I), the liquid vehicle may be water or another aqueous medium. Depending upon the animal to be treated, the injection may be subcutaneous or into the proventriculus, a muscle or the trachea. Such preparations would normally contain the active compound at a concentration of from 0.05 to 50% by weight.

The compounds of the invention may also be administered topically in admixture with a suitable carrier, such as dimethyl sulphoxide or a hydrocarbon solvent. Such preparations would be applied directly to the outside of the animal by spraying (e.g. by a hand spray or in spray races), by dipping (e.g. in a plunge dip), by a pour-on solution or by manual methods (e.g. hand-dressing).

The dose of active compound may be varied, depending upon the nature of the animal to be treated, and the nature and degree of parasitic infection. However, best results for oral administration are achieved when the dose is from 0.01 to 100 mg, more preferably from 0.5 to 50 mg, per 1 kg body weight. The compound may be administered in a single dose or in divided doses for a relatively short period, such as from 1 to 5 days.

Where the composition of the invention is intended for agricultural or horticultural use, a variety of forms and formulations is possible. For example, the composition may be formulated as dusts, coarse dusts, soluble powders, microgranules, fine microgranules, wettable powders, dilute emulsions, emulsifiable concentrates, aqueous or oily suspensions, dispersions or solutions (which may be directly sprayable or for dilution), aerosols or capsules in, for example, polymeric substances. The carrier employed may be natural or synthetic and organic or inorganic; it is generally employed to assist the active compound to reach the substrate to be treated, and to make it easier to store, transport or handle the active compound. Solid, liquid and gaseous carriers may be employed, chosen from carriers well known in the art for use with compositions of this type.

Such formulations may be prepared by conventional means, e.g. by intimate mixing and/or grinding of the active ingredient(s) with the carrier or diluent, e.g. solvent, solid carrier or, optionally, surface-active agent.

Suitable solvents include: aromatic hydrocarbons, preferably the $C_8$ to $C_{12}$ fractions from petroleum distillation, such as xylene mixtures or substituted naphthalenes; esters of phthalic acid, such as dibutyl or dioctyl phthalate; aliphatic hydrocarbons, such as cyclohexane or the paraffins; alcohols and glycols or esters thereof, such as ethanol, ethylene glycol, ethylene glycol monomethyl ether or ethylene glycol monoethyl ether; ketones, such as cyclohexanone; strongly polar solvents, such as N-methyl-2-pyrrolidone,

dimethyl sulphoxide or N,N-dimethylformamide; optionally epoxidized vegetable oils, such as epoxidized coconut oil or soybean oil; and water.

Solid carriers, which may be used, for example, in dusts and dispersible powders, include natural mineral fillers, such as calcite, talc, kaolin, montmorillonite or attapulgite. In order to improve the physical properties of the composition, it is also possible to add highly dispersed silicic acid or highly dispersed absorbent polymers. Suitable granulated adsorptive carriers may be porous (such as pumice, ground brick, sepiolite or bentonite) or non-porous (such as calcite or sand). A wide variety of pregranulated materials, organic or inorganic, may also be used; examples include dolomite and ground plant residues.

Surface-active agents which may be used are well known in the art and may be non-ionic, cationic or anionic agents having good emulsifying, dispersing and wetting properties. Mixtures of such agents may also be used.

Compositions may also contain stabilizers, anti-foaming agents, viscosity regulators, binders or adhesives or any combination thereof, as well as fertilizers or other active substances to achieve special effects.

Pesticidal compositions will generally contain: from 0.01 to 99%, more preferably from 0.1 to 95%, by weight of the active compound; from 1 to 99.99% of a solid or liquid additive; and from 0 to 25%, more preferably from 0.1 to 25%, of a surface-active agent. Whereas commercial products are generally sold as concentrated compositions, they are generally diluted by the end-user to a concentration of from 0.001 to 0.0001% by weight (from 10 to 1 ppm).

The invention is further illustrated by the following Examples, which illustrate the preparation of the compounds of the present invention, and the subsequent Preparation, which illustrates the preparation of one of the starting materials used in these Examples. In the following Examples, all Nuclear Magnetic Resonance Spectra were measured at 270 MHz, unless otherwise stated.

## EXAMPLE 1

### 13-Phenethyloxymilbemcin $A_4$

#### Step A

0.333 g of 13-iodo-5-oxomilbemycin $A_4$ (III) (prepared as described in Preparation 1) was dissolved in 2.50 ml of 1,2-dichloroethane, and then 0.610 g of β-phenethyl alcohol and 1.000 g of silver oxide were added to the resulting solution. The mixture was then stirred at room temperature for 30 minutes. At the end of this time, 30 ml of ethyl acetate were added to the reaction mixture, insoluble materials were removed by filtration using a Celite (trade mark) filter aid. The filtrate was then washed with a 10% w/v aqueous solution of sodium thiosulphate and with water, in that order, after which it was dried over anhydrous sodium sulphate, and the solvent was removed by distillation to dryness under reduced pressure. The residue was purified by column chromatography through silica gel, eluted with a 3 : 7 by volume mixture of ethyl acetate and cyclohexane, to afford 0.282 g of 5-oxo-13-phenethyloxymilbemycin $A_4$.

#### Step B

0.140 g of 5-oxo-13-phenethyloxymilbemycin $A_4$ (prepared as described in Step A) was dissolved in 6 ml of methanol, and 0.009 g of sodium borohydride was added to the resulting solution, whilst ice-cooling. The mixture was then stirred for 30 minutes. At the end of this time, 30 ml of ethyl acetate were added to the reaction mixture, and the mixture was washed twice with water and dried over anhydrous sodium sulphate. The solvent was then removed by distillation to dryness under reduced pressure. The residue was purified by column chromatography through silica gel, eluted with a 3 : 7 by volume mixture of ethyl acetate and cyclohexane. The isomer substituted at the 15-position was then separated by reverse phase chromatography (ODS: eluted with 85% v/v aqueous acetonitrile) to obtain 0.076 g of the title compound. "ODS" is octadecylsilane.

Mass Spectrum m/e: 662 ($M^+$, $C_{40}H_{54}O_8$).

Nuclear Magnetic Resonance Spectrum ($CDCl_3$) δ ppm:

1.87 (3H, singlet);
3.22 (1H, doublet, J = 10.0 Hz);
3.95 (1H, doublet, J = 6.2 Hz).

EXAMPLE 2

13-Phenethyloxymilbemycin $A_4$ 5-oxime

0.85 ml of water, 1.7 ml of dioxane and 0.150 g of hydroxylamine hydrochloride were added to a solution of 0.140 g of 5-oxo-13-phenethyloxymilbemycin $A_4$ (prepared as described in Step A of Example 1) in 1.7 ml of methanol. The mixture was then stirred at 35 °C for 3 hours. At the end of this time, the reaction mixture was diluted with 20 ml of ethyl acetate, washed twice with water and dried over anhydrous sodium sulphate. The solvent was then removed by distillation to dryness under reduced pressure. The residue was purified by column chromatography through silica gel, eluted with a 3 : 7 by volume mixture of ethyl acetate and cyclohexane. The isomer substituted at the 15-position was then separated by reverse phase chromatography (ODS: eluted with 85% v/v aqueous acetonitrile) to obtain 0.080 g of the title compound.
Mass Spectrum m/e: 675 ($M^+$, $C_{40}H_{53}NO_8$).
Nuclear Magnetic Resonance Spectrum ($CDCl_3$) $\delta$ ppm:
1.94 (3H, singlet);
3.22 (1H, doublet, J = 9.9 Hz);
4.66 (1H, singlet).

EXAMPLE 3

13-[2-(4-Nitrophenyl)ethoxy]milbemycin-$A_4$

Step A

1.250 g of 4-nitrophenethyl alcohol, 0.300 g of anhydrous calcium carbonate and 0.815 g of mercuric iodide were added to a solution of 1.000 g of 13-iodo-5-oxomilbemycin $A_4$ (III) (prepared as described in Preparation 1) in 6.0 ml of 1,2-dichloroethane, and the mixture was stirred at room temperature for 2 hours. At the end of this time, 30 ml of ethyl acetate were added to the reaction mixture, insoluble materials were removed by filtration, and the filtrate was washed with a 20% w/v aqueous solution of potassium iodide (twice), with a 10% w/v aqueous solution of sodium thiosulphate and with water, in that order, after which it was dried over anhydrous sodium sulphate. The solvent was then removed by distillation to dryness under reduced pressure. The residue was purified by column chromatography through silica gel, eluted with a 15 : 85 by volume mixture of ethyl acetate and cyclohexane, to afford 0.744 g of 5-oxo-13-[2-(4-nitrophenyl)-ethoxy]milbemycin $A_4$.

Step B

0.037 g of sodium borohydride was added to a solution of 0.710 g of 5-oxo-13-[2-(4-nitrophenyl)ethoxy]-milbemycin $A_4$ (prepared as described in the preceding Step A) in 27 ml of methanol, whilst ice-cooling, and the mixture was stirred for 20 minutes. At the end of this time, 100 ml of ethyl acetate were added to the reaction mixture, the mixture was washed twice with water and dried over anhydrous sodium sulphate. The solvent was then removed by distillation to dryness under reduced pressure. The residue was purified by column chromatography through silica gel, eluted with a 2 : 8 by volume mixture of ethyl acetate and cyclohexane, to afford 0.693 g of the title compound.
Mass Spectrum m/e: 707 ($M^+$, $C_{40}H_{53}NO_{10}$).
Nuclear Magnetic Resonance Spectrum ($CDCl_3$) $\delta$ ppm:
1.87 (3H, singlet);
3.24 (1H, doublet, J = 9.5 Hz);
3.95 (1H, doublet, J = 6.2 Hz).

The compounds of Examples 4 to 21 were prepared by the same method as described in Example 3.

EXAMPLE 4

13-(1-Methyl-2-phenylethoxy)milbemycin $A_4$

Mass Spectrum (m/e) 676 ($M^+$, $C_{41}H_{56}O_8$).
Nuclear Magnetic Resonance Spectrum ($CDCl_3$) $\delta$ ppm:
1.87 (3H, singlet);

3.27 (0.5H, doublet, J = 10.0 Hz);
3.33 (0.5H, doublet, J = 9.9 Hz);
3.955 (0.5H, doublet, J = 6.2 Hz);
3.950 (0.5H, doublet, J = 6.2 Hz).

EXAMPLE 5

13-[2-(4-Chlorophenyl)ethoxy]milbemycin $A_4$

Mass Spectrum (m/e): 696 ($M^+$, $C_{40}H_{53}C\ell O_8$).
Nuclear Magnetic Resonance Spectrum ($CDC\ell_3$) $\delta$ ppm:
1.83 (3H, singlet);
3.19 (1H, doublet, J = 9.9 Hz);
4.00 (1H, doublet, J = 6.9 Hz).

EXAMPLE 6

13-[2-(4-Methoxyphenyl)ethoxy]milbemycin $A_4$

Mass Spectrum (m/e): 692 ($M^+$, $C_{41}H_{56}O_9$).
Nuclear Magnetic Resonance Spectrum ($CDC\ell_3$) $\delta$ ppm:
1.87 (3H, singlet);
3.21 (1H, doublet, J = 9.9 Hz);
3.96 (1H, doublet, J = 6.2 Hz).

EXAMPLE 7

13-(2-Phenylpropoxy)milbemycin $A_4$

Mass Spectrum (m/e): 676 ($M^+$, $C_{41}H_{56}O_8$).
Nuclear Magnetic Resonance Spectrum ($CDC\ell_3$) $\delta$ ppm:
1.83 (3H, singlet);
3.20 (1H, doublet, J = 9.9 Hz);
3.95 (1H, doublet, J = 6.2 Hz).

EXAMPLE 8

13-(2-Methoxy-2-phenylethoxy)milbemycin $A_4$

Mass Spectrum (m/e): 692 ($M^+$, $C_{41}H_{56}O_9$).
Nuclear Magnetic Resonance Spectrum ($CDC\ell_3$) $\delta$ ppm:
1.87 (3H, singlet);
3.28 (3H, singlet);
3.31 (1H, doublet, J = 9.9 Hz);
3.96 (1H, doublet, J = 6.2 Hz).

EXAMPLE 9

13-[2-(3,4-Dimethoxyphenyl)ethoxy]milbemycin $A_4$

Mass Spectrum (m/e): 722 ($M^+$, $C_{42}H_{58}O_{10}$).
Nuclear Magnetic Resonance Spectrum ($CDC\ell_3$) $\delta$ ppm:
1.87 (3H, singlet);
3.22 (1H, doublet, J = 9.5 Hz);
3.22 (3H, singlet);
3.88 (3H, singlet);
3.95 (1H, doublet, J = 6.2 Hz).

EXAMPLE 10

13-[2-(4-Methoxy-3-nitrophenyl)ethoxy]milbemycin $A_4$

Mass Spectrum (m/e): 737 ($M^+$, $C_{41}H_{55}NO_{10}$).
Nuclear Magnetic Resonance Spectrum ($CDCl_3$) $\delta$ ppm:
1.87 (3H, singlet);
3.19 (1H, doublet, J=9.9 Hz);
3.95 (1H, doublet, J=6.2 Hz);
3.94 (3H, singlet).

EXAMPLE 11

13-[2-(3-Methoxy-4-nitrophenyl)ethoxy]milbemycin $A_4$

Mass Spectrum (m/e): 737 ($M^+$, $C_{41}H_{55}NO_{10}$).
Nuclear Magnetic Resonance Spectrum ($CDCl_3$) $\delta$ ppm:
1.88 (3H, singlet);
3.20 (1H, doublet, J=9.9 Hz);
3.95 (3H, singlet).

EXAMPLE 12

13-[2-(2,4-Dimethylphenyl)ethoxy]milbemycin $A_4$

Mass Spectrum (m/e): 690 ($M^+$, $C_{42}H_{58}O_8$).
Nuclear Magnetic Resonance Spectrum ($CDCl_3$) $\delta$ ppm:
1.88 (3H, singlet);
3.95 (1H, doublet, J=6.2 Hz).

EXAMPLE 13

13-[2-(4-Fluorophenyl)ethoxy]milbemycin $A_4$

Mass Spectrum (m/e): 680 ($M^+$, $C_{40}H_{53}FO_8$).
Nuclear Magnetic Resonance Spectrum ($CDCl_3$) $\delta$ ppm:
1.87 (3H, singlet);
3.19 (1H, doublet, J=9.9 Hz);
3.95 (1H, doublet, J=6.2 Hz).

EXAMPLE 14

13-[2-(3,4-Dichlorophenyl)ethoxy]milbemycin $A_4$

Mass Spectrum (m/e): 731 ($M^+$, $C_{40}H_{52}Cl_2O_8$).
Nuclear Magnetic Resonance Spectrum ($CDCl_3$) $\delta$ ppm:
1.87 (3H, singlet);
3.19 (1H, doublet, J=9.9 Hz);
3.95 (1H, doublet, J=6.2 Hz).

EXAMPLE 15

13-[2-(2,5-Dimethoxyphenyl)ethoxy]milbemycin $A_4$

Mass Spectrum (m/e): 722 ($M^+$, $C_{40}H_{58}O_{10}$).
Nuclear Magnetic Resonance Spectrum ($CDCl_3$) $\delta$ ppm:
1.87 (3H, singlet);
3.24 (1H, doublet, J=9.5 Hz);

3.95 (1H, doublet, J = 6.2 Hz).

EXAMPLE 16

13-[2-(4-Ethoxy-3-methoxyphenyl)ethoxy]milbemycin $A_4$

Mass Spectrum (m/e): 736 ($M^+$, $C_{43}H_{60}O_{10}$).
Nuclear Magnetic Resonance Spectrum ($CDCl_3$) δ ppm:
1.87 (3H, singlet);
3.22 (1H, doublet, J = 9.9 Hz);
3.96 (1H, doublet, J = 6.2 Hz).

EXAMPLE 17

13-[2-(2,6-Difluorophenyl)ethoxy]milbemycin $A_4$

Mass Spectrum (m/e): 698 ($M^+$, $C_{40}H_{52}F_2O_8$).
Nuclear Magnetic Resonance Spectrum ($CDCl_3$) δ ppm:
1.87 (3H, singlet);
3.21 (1H, doublet, J = 9.5 Hz);
3.95 (1H, doublet, J = 6.2 Hz).

EXAMPLE 18

13-[2-(2-Nitrophenyl)ethoxy]milbemycin $A_4$

Mass Spectrum (m/e): 707 ($M^+$, $C_{40}H_{53}NO_{10}$).
Nuclear Magnetic Resonance Spectrum ($CDCl_3$) δ ppm:
1.87 (3H, singlet);
3.96 (1H, doublet, J = 6.2 Hz).

EXAMPLE 19

13-[2-(3-Nitrophenyl)ethoxy]milbemycin $A_4$

Mass Spectrum (m/e): 707 ($M^+$, $C_{40}H_{53}NO_{10}$).
Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz).

EXAMPLE 20

13-[2-(3-Methoxy-4-methoxymethoxyphenyl)ethoxy]milbemycin $A_4$

Mass Spectrum (m/e): 752 ($M^+$, $C_{43}H_{60}O_{11}$).
Nuclear Magnetic Resonance Spectrum ($CDCl_3$) δ ppm:
1.87 (3H, singlet);
3.21 (1H, doublet, J = 9.9 Hz);
3.95 (1H, doublet, J = 6.2 Hz).

EXAMPLE 21

13-[2-(3,4-Dimethoxyphenyl)propoxy]milbemycin $A_4$

Mass Spectrum (m/e): 736 ($M^+$, $C_{43}H_{60}O_{10}$).
Nuclear Magnetic Resonance Spectrum ($CDCl_3$) δ ppm:
1.87 (3H, singlet);

3.87 (3H, singlet);
3.88 (3H, singlet);
3.97 (1H, doublet, J = 6.2 Hz).

EXAMPLE 22

13-[2-(4-Nitrophenyl)ethoxy]milbemycin $A_4$5-oxime

0.6 ml of water, 1.2 ml of dioxane and 0.110 g of hydroxylamine hydrochloride were added to a solution of 0.106 g of 5-oxo-13-[2-(4-nitrophenyl)ethoxy]milbemycin $A_4$ (prepared as described in Step A of Example 3) in 1.2 ml of methanol, and the mixture was stirred at 40 °C for 2.5 hours. At the end of this time, 20 ml of ethyl acetate were added to the reaction mixture, and the mixture was washed twice with water. It was then dried over anhydrous sodium sulphate, and the solvent was removed by distillation to dryness under reduced pressure. The residue was purified by column chromatography through silica gel, eluted with a 2 : 8 by volume mixture of ethyl acetate and cyclohexane, to afford 0.090 g of the title compound.
Mass Spectrum m/e: 720 ($M^+$, $C_{40}H_{52}N_2O_{10}$).
Nuclear Magnetic Resonance Spectrum ($CDCl_3$) δ ppm:
1.94 (3H, singlet);
3.20 (1H, doublet, J = 9.9 Hz);
4.66 (1H, singlet).

The compounds of Examples 23 - 34 were prepared by a similar procedure to that described in Step A of Example 3 and in Example 22.

EXAMPLE 23

13-(1-Methyl-2-phenylethoxy)milbemycin $A_4$oxime

Mass Spectrum (m/e) 689 ($M^+$, $C_{41}H_{55}NO_8$).
Nuclear Magnetic Resonance Spectrum ($CDCl_3$) δ ppm:
1.94 (3H, singlet);
3.28 (0.5H, doublet, J = 10.3 Hz);
3.34 (0.5H, doublet, J = 9.9 Hz);
4.65 (0.5H, singlet);
4.66 (0.5H, singlet).

EXAMPLE 24

13-[2-(4-Chlorophenyl)ethoxy]milbemycin $A_4$oxime

Mass Spectrum (m/e): 709 ($M^+$, $C_{41}H_{52}ClNO_8$).
Nuclear Magnetic Resonance Spectrum ($CDCl_3$) δ ppm:
1.94 (3H, singlet);
3.20 (1H, doublet, J = 9.9 Hz);
4.66 (1H, singlet).

EXAMPLE 25

13-(2-Phenylpropoxy)milbemycin $A_4$oxime

Mass Spectrum (m/e): 689 ($M^+$, $C_{41}H_{55}NO_8$).
Nuclear Magnetic Resonance Spectrum ($CDCl_3$) δ ppm:
1.93 (3H, singlet);
3.20 (1H, doublet, J = 10.2 Hz);
4.66 (1H, singlet).

EXAMPLE 26

13-[2-(3,4-Dimethoxyphenyl)propoxy]milbemycin $A_4$oxime

Mass Spectrum (m/e): 749 ($M^+$, $C_{43}H_{59}NO_{10}$).
Nuclear Magnetic Resonance Spectrum ($CDCl_3$) δ ppm:
1.94 (3H, singlet);
3.16 (1H, doublet, J = 9.9 Hz);
4.66 (1H, singlet).

EXAMPLE 27

13-(4-Methoxyphenethyloxy)milbemycin $A_4$oxime

Mass Spectrum (m/e): 705 ($M^+$, $C_{41}H_{55}NO_9$).
Nuclear Magnetic Resonance Spectrum ($CDCl_3$) δ ppm:
1.94 (3H, singlet);
3.21 (1H, doublet, J = 9.9 Hz);
4.66 (1H, singlet).

EXAMPLE 28

13-(4-Fluorophenethyloxy)milbemycin $A_4$oxime

Mass Spectrum (m/e): 693 ($M^+$, $C_{40}H_{52}FNO_8$).
Nuclear Magnetic Resonance Spectrum ($CDCl_3$) δ ppm:
1.94 (3H, singlet);
3.19 (1H, doublet, J = 9.9 Hz);
4.66 (1H, singlet).

EXAMPLE 29

13-(3,4-Dichlorophenethyloxy)milbemycin $A_4$oxime

Mass Spectrum (m/e): 743 ($M^+$, $C_{40}H_{52}Cl_2NO_8$).
Nuclear Magnetic Resonance Spectrum ($CDCl_3$) δ ppm:
1.94 (3H, singlet);
3.19 (1H, doublet, J = 9.5 Hz);
4.66 (1H, singlet).

EXAMPLE 30

13-(2,5-Dimethoxyphenethyloxy)milbemycin $A_4$oxime

Mass Spectrum (m/e): 735 ($M^+$, $C_{42}H_{57}NO_{10}$).
Nuclear Magnetic Resonance Spectrum ($CDCl_3$) δ ppm:
1.94 (3H, singlet);
3.24 (1H, doublet, J = 9.9 Hz);
4.66 (1H, singlet).

EXAMPLE 31

13-(4-Ethoxy-3-methoxyphenethyloxy)milbemycin $A_4$oxime

Mass Spectrum (m/e): 749 ($M^+$, $C_{43}H_{59}NO_{10}$).
Nuclear Magnetic Resonance Spectrum ($CDCl_3$) δ ppm:
1.94 (3H, singlet);
3.22 (1H, doublet, J = 9.9 Hz);

4.66 (1H, singlet).

EXAMPLE 32

13-(2,6-Difluorophenethyloxy)milbemycin $A_4$oxime

Mass Spectrum (m/e): 711 ($M^+$, $C_{40}H_{51}F_2NO_8$).
Nuclear Magnetic Resonance Spectrum ($CDCl_3$) δ ppm:
1.93 (3H, singlet);
3.22 (1H, doublet, J = 9.9 Hz);
4.66 (1H, singlet).

EXAMPLE 33

13-(3-Nitrophenethyloxy)milbemycin $A_4$oxime

Mass Spectrum (m/e): 720 ($M^+$, $C_{40}H_{52}N_2O_{10}$).
Nuclear Magnetic Resonance Spectrum ($CDCl_3$) δ ppm:
1.94 (3H, singlet);
3.20 (1H, doublet, J = 9.9 Hz);
4.66 (1H, singlet).

EXAMPLE 34

13-(3-Methoxy-4-methoxymethoxyphenethyloxy)milbemycin $A_4$oxime

Mass Spectrum (m/e): 765 ($M^+$, $C_{43}H_{59}NO_{11}$).
Nuclear Magnetic Resonance Spectrum ($CDCl_3$) δ ppm:
1.94 (3H, singlet);
3.22 (1H, doublet, J = 9.5 Hz);
4.66 (1H, singlet).

EXAMPLE 35

13-[2-(4-Aminophenyl)ethoxy]milbemycin $A_4$

0.70 g of zinc powder was added to a solution of 0.693 g of 13-[2-(4-nitrophenyl)ethoxy]milbemycin $A_4$ - (prepared as described in Example 3) in 7 ml of 90% v/v aqueous acetic acid, whilst cooling with water, and the mixture was stirred for 20 minutes. At the end of this time, 50 ml of ethyl acetate were added to the reaction mixture, insoluble materials were removed by filtration, and the filtrate was washed three times with water. It was then dried over anhydrous sodium sulphate, and the solvent was removed by distillation to dryness under reduced pressure. The residue was purified by column chromatography through silica gel (ODS treated), eluted with 75% v/v aqueous acetonitrile), to afford 0.620 g of the title compound.
Mass Spectrum m/e: 677 ($M^+$, $C_{40}H_{55}NO_8$),
Nuclear Magnetic Resonance Spectrum ($CDCl_3$) δ ppm:
1.87 (3H, singlet);
3.21 (1H, doublet, J = 9.9 Hz);
3.95 (1H, doublet, J = 6.2 Hz).

The compounds of Examples 36 and 37 were prepared by a similar procedure to that described in Example 35 above.

EXAMPLE 36

13-[2-(3-Amino-4-methoxyphenyl)ethoxy]milbemycin $A_4$

Mass Spectrum m/e: 707 ($M^+$, $C_{41}H_{57}NO_9$).
Nuclear Magnetic Resonance Spectrum ($CDCl_3$) δ ppm:
1.87 (3H, singlet);

3.21 (1H, doublet, J = 9.9 Hz);
3.84 (3H, singlet);
3.95 (1H, doublet, J = 6.2 Hz).

EXAMPLE 37

13-[2-(4-Amino-3-methoxyphenyl)ethoxy]milbemycin $A_4$

Mass Spectrum m/e: 707 ($M^+$, $C_{41}H_{57}NO_9$).
Nuclear Magnetic Resonance Spectrum ($CDCl_3$) $\delta$ ppm:
1.87 (3H, singlet);
3.22 (1H, doublet, J = 9.9 Hz);
3.85 (3H, singlet);
3.95 (1H, doublet, J = 6.2 Hz).

EXAMPLE 38

13-[2-(4-Benzoylaminophenyl)ethoxy]milbemycin $A_4$

0.024 ml of pyridine and 0.035 ml of benzoyl chloride were added to a solution of 0.200 g of 13-[2-(4-aminophenyl)ethoxy]milbemycin $A_4$ (prepared as described in Example 35) in 3.0 ml of methylene chloride, and the mixture was stirred for 15 minutes. At the end of this time, the reaction mixture was poured into ice-water and extracted with methylene chloride. The extract was washed with 0.1 N aqueous hydrochloric acid, with water, with a 4% w/v aqueous solution of sodium bicarbonate and with water, in that order. It was then dried over anhydrous sodium sulphate, and the solvent was removed by distillation to dryness under reduced pressure. The residue was purified by column chromatography through silica gel (ODS treated, eluted with 70% v/v aqueous acetonitrile), to afford 0.188 g of the title compound.
Mass Spectrum m/e: 763 ($M^+$ - $H_2O$, $C_{47}H_{57}NO_8$).
Nuclear Magnetic Resonance Spectrum ($CDCl_3$) $\delta$ ppm:
1.87 (3H, singlet);
3.22 (1H, doublet, J = 9.9 Hz);
3.95 (1H, doublet, J = 5.9 Hz).

The compounds of Examples 39 - 47 were prepared by a similar procedure to that described in Example 38 above.

EXAMPLE 39

13-[2-(4-Acetamidophenyl)ethoxy]milbemycin $A_4$

Nuclear Magnetic Resonance Spectrum ($CDCl_3$) $\delta$ ppm:
1.87 (3H, singlet);
2.18 (3H, singlet);
3.20 (1H, doublet, J = 9.9 Hz);
3.95 (1H, doublet, J = 6.2 Hz).

EXAMPLE 40

13-{2-[4-(3,4-Dimethoxybenzoylamino)phenyl]ethoxy}milbemycin $A_4$

Nuclear Magnetic Resonance Spectrum ($CDCl_3$) $\delta$ ppm:
1.87 (3H, singlet);
3.22 (1H, doublet, J = 9.9 Hz);
3.95 (3H, singlet);
3.96 (3H, singlet);
3.98 (1H, doublet, J = 6.2 Hz).

EXAMPLE 41

13-[2-(4-Methoxycarbonylaminophenyl)ethoxy]milbemycin $A_4$

Mass Spectrum m/e: 735 ($M^+$, $C_{42}H_{57}NO_{10}$).
Nuclear Magnetic Resonance Spectrum ($CDC\ell_3$) $\delta$ ppm:
1.87 (3H, singlet);
3.20 (1H, doublet, J=9.5 Hz);
3.95 (1H, doublet, J=6.2 Hz).

EXAMPLE 42

13-[2-(4-Ethoxycarbonylaminophenyl)ethoxy]milbemycin $A_4$

Mass Spectrum m/e: 749 ($M^+$, $C_{43}H_{59}NO_{10}$).
Nuclear Magnetic Resonance Spectrum ($CDC\ell_3$) $\delta$ ppm:
1.87 (3H, singlet);
3.20 (1H, doublet, J=9.9 Hz);
3.95 (1H, doublet, J=6.2 Hz).

EXAMPLE 43

13-[2-(4-Butoxycarbonylaminophenyl)ethoxy]milbemycin $A_4$

Mass Spectrum m/e: 777 ($M^+$, $C_{45}H_{63}NO_{10}$).
Nuclear Magnetic Resonance Spectrum ($CDC\ell_3$) $\delta$ ppm:
1.87 (3H, singlet);
3.20 (1H, doublet, J=9.9 Hz);
3.95 (1H, doublet, J=6.2 Hz).

EXAMPLE 44

13-[2-(4-Isobutoxycarbonylaminophenyl)ethoxy]milbemycin $A_4$

Mass Spectrum m/e: 777 ($M^+$, $C_{45}H_{63}NO_{10}$).
Nuclear Magnetic Resonance Spectrum ($CDC\ell_3$) $\delta$ ppm:
1.87 (3H, singlet);
3.20 (1H, doublet, J=9.9 Hz);
3.95 (1H, doublet, J=6.2 Hz).

EXAMPLE 45

13-[2-(4-Benzyloxycarbonylaminophenyl)ethoxy]milbemycin $A_4$

Nuclear Magnetic Resonance Spectrum ($CDC\ell_3$) $\delta$ ppm:
1.87 (3H, singlet);
3.20 (1H, doublet, J=9.9 Hz);
3.95 (1H, doublet, J=6.2 Hz);
5.19 (2H, singlet).

EXAMPLE 46

13-[2-(4-Methanesulphonylaminophenyl)ethoxy]milbemycin $A_4$

Nuclear Magnetic Resonance Spectrum ($CDC\ell_3$) $\delta$ ppm:
1.87 (3H, singlet);
2.97 (3H, singlet);
3.20 (1H, doublet, J=9.9 Hz);

3.95 (1H, doublet, J = 6.2 Hz);
6.39 (1H, singlet).

EXAMPLE 47

13-[2-(4-Benzenesulphonylaminophenyl)ethoxy]milbemycin $A_4$

Nuclear Magnetic Resonance Spectrum ($CDCl_3$) δ ppm:
1.87 (3H, singlet);
3.16 (1H, doublet, J = 9.9 Hz);
3.95 (1H, doublet, J = 6.2 Hz);
6.49 (1H, singlet).

EXAMPLE 48

13-{2-[4-(3-Phenylureido)phenyl]ethoxy}milbemycin $A_4$

0.039 ml of phenylisocyanate was added to a solution of 0.200 g of 13-[2-(4-aminophenyl)ethoxy]-milbemycin $A_4$ (prepared as described in Example 35) in 2.0 ml of tetrahydrofuran, and the mixture was stirred for 2 hours. At the end of this time, the reaction mixture was diluted with 20 ml of ethyl acetate, washed with water and dried over anhydrous sodium sulphate. The solvent was then removed by distillation to dryness under reduced pressure. The residue was purified by column chromatography (ODS treated, eluted with 80% v/v aqueous acetonitrile), to afford 0.207 g of the title compound.
Nuclear Magnetic Resonance Spectrum ($CDCl_3$, 270 MHz) δ ppm:
1.87 (3H, singlet);
3.21 (1H, doublet, J = 9.9 Hz);
3.95 (1H, doublet, J = 6.2 Hz);
6.56 (1H, singlet);
6.61 (1H, singlet).

The compounds of Examples 49 - 56 were prepared by a similar procedure to that described above.

EXAMPLE 49

13-{2-[4-(3-Methylureido)phenyl]ethoxy}milbemycin $A_4$

Nuclear Magnetic Resonance Spectrum ($CDCl_3$) δ ppm:
1.87 (3H, singlet);
2.82 (3H, doublet, J = 4.8 Hz);
3.21 (1H, doublet, J = 9.9 Hz);
3.95 (1H, doublet, J = 6.2 Hz).

EXAMPLE 50

13-{2-[4-(3-Cyclohexylureido)phenyl]ethoxy}milbemycin $A_4$

Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz);
4.53 (1H, doublet, J = 8.1 Hz);
6.12 (1H, singlet).

EXAMPLE 51

13-{2-[4-(3-o-Fluorophenylureido)phenyl]ethoxy}milbemycin $A_4$

Nuclear Magnetic Resonance Spectrum ($CDCl_3$) δ ppm:
1.87 (3H, singlet);

3.21 (1H, doublet, J = 9.9 Hz);
3.96 (1H, doublet, J = 6.2 Hz);
6.63 (1H, singlet).

EXAMPLE 52

13-{2-[4-(3-o-Methylphenylureido)phenyl]ethoxy}milbemycin $A_4$

Nuclear Magnetic Resonance Spectrum ($CDCl_3$) δ ppm:
1.87 (3H, singlet);
3.21 (1H, doublet, J = 9.9 Hz);
3.95 (1H, doublet, J = 6.2 Hz);
6.48 (1H, singlet);
6.52 (1H, singlet).

EXAMPLE 53

13-{2-[4-(3-α-Naphthylureido)phenyl]ethoxy}milbemyin $A_4$

Nuclear Magnetic Resonance Spectrum ($CDCl_3$) δ ppm:
1.87 (3H, singlet);
3.19 (1H, doublet, J = 9.9 Hz);
3.95 (1H, doublet, J = 6.2 Hz);
6.53 (1H, singlet);
6.71 (1H, singlet).

EXAMPLE 54

13-{2-[4-(3-p-Methoxyphenylureido)phenyl]ethoxy}milbemycin $A_4$

Nuclear Magnetic Resonance Spectrum ($CDCl_3$) δ ppm:
1.87 (3H, singlet);
3.21 (1H, doublet, J = 9.5 Hz);
3.95 (1H, doublet, J = 6.2 Hz);
6.36 (1H, singlet);
6.43 (1H, singlet).

EXAMPLE 55

13-{2-[4-(3-p-Nitrophenylureido)phenyl]ethoxy}milbemycin $A_4$

Nuclear Magnetic Resonance Spectrum ($CDCl_3$) δ ppm:
1.87 (3H, singlet);
3.21 (1H, doublet, J = 9.5 Hz);
3.96 (1H, doublet, J = 6.2 Hz);
6.83 (1H, singlet).

EXAMPLE 56

13-{2-[4-(3-p-Chlorophenylureido)phenyl]ethoxy}milbemycin $A_4$

Nuclear Magnetic Resonance Spectrum ($CDCl_3$) δ ppm:
1.87 (3H, singlet);
3.21 (1H, doublet, J = 9.9 Hz);
3.95 (1H, doublet, J = 6.2 Hz);
6.55 (1H, singlet);
6.65 (1H, singlet).

EXAMPLE 57

13-{2-[4-(3-phenylthioureido)phenyl]ethoxy}milbemycin $A_4$

0.043 ml of phenylisothiocyanate was added to a solution of 0.200 g of 13-[2-(4-aminophenyl)ethoxy]-milbemycin $A_4$ (prepared as described in Example 35) in 1.0 ml of tetrahydrofuran, and the mixture was stirred for 5 hours. At the end of this time, the reaction mixture was poured into ice-water and extracted with 20 ml of ethyl acetate. The extract was dried over anhydrous sodium sulphate and the solvent was removed by distillation to dryness under reduced pressure. The residue was purified by column chromatography through silica gel (ODS treated, eluted with 80% v/v aqueous acetonitrile) to afford 0.253 g of the title compound.

Nuclear Magnetic Resonance Spectrum ($CDCl_3$) δ ppm:

1.87 (3H, singlet);
3.21 (1H, doublet, J = 9.5 Hz);
3.95 (1H, doublet, J = 6.1 Hz);
7.67 (2H, singlet).

EXAMPLE 58

13-{2-[4-(3-m-Fluorophenylthioureido)phenyl]ethoxy}milbemycin $A_4$

The title compound was synthesized following a similar procedure to that described in Example 57, but using 3-fluorophenylisothiocyanate instead of the phenyliosthiocyanate

Nuclear Magnetic Resonance Spectrum ($CDCl_3$) δ ppm:

1.87 (3H, singlet);
3.21 (1H, doublet, J = 9.5 Hz);
3.95 (1H, doublet, J = 6.2 Hz);
7.60 (1H, singlet);
7.75 (1H, singlet).

EXAMPLE 59

13-[2-(4-Propionylaminophenyl)ethoxy]milbemycin $A_4$

Step 1

4.96 g of 13-[2-(4-nitrophenyl)ethoxy]milbemycin $A_4$ (prepared by the same procedure as that described in Example 3) were dissolved in 20 ml of dimethylformamide. 0.571 g of imidazole and 1.270 g of t-butyldimethylsilyl chloride were added to the resulting solution, and then the mixture was stirred at room temperature for 2.5 hours. At the end of this time, the reaction mixture was diluted with 200 ml of ethyl acetate, washed with water four times and dried over anhydrous sodium sulphate. The solvent was then removed by evaporation to dryness under reduced pressure, and the residue was purified by column chromatography through silica gel, eluted with a 85 : 15 by volume mixture of cyclohexane and ethyl acetate, to give 5.08 g of the 5-silylated ether. The whole of this product was dissolved in 50 ml of 90% acetic acid, 5.00 g of zinc powder were added, and then the mixture was stirred for 20 minutes, whilst cooling with water. The mixture was then diluted with 250 ml of ethyl acetate and the insoluble matter was filtered off. The filtrate was washed with water (three times), with a 4% w/v aqueous solution of sodium bicarbonate and with water, in that order, after which it was dried over anhydrous sodium sulphate. The solvent was then removed by evaporation to dryness under reduced pressure, and the residue was purified by column chromatography through silica gel (ODS treated), eluted with 90% v/v aqueous actonitrile, to give 4.370 g of 13-[2-(4-aminophenyl)ethoxy]-5-O-t-butyldimethylsilylmilbemycin $A_4$.

Mass Spectrum m/e: 791 ($M^+$, $C_{46}H_{69}NO_8Si$).

Nuclear Magnetic Resonance Spectrum ($CDCl_3$) δ ppm:

1.87 (3H, singlet);
3.21 (1H, doublet, J = 9.9 Hz);
3.80 (1H, doublet, J = 5.5 Hz);
6.61 (2H, doublet, J = 8.4 Hz);
6.99 (2H, doublet, J = 8.4 Hz).

Step 2

0.200 g of 13-[2-(4-aminophenyl)ethoxy]-5-O-t-butyldimethylsilylmilbemycin $A_4$ (prepared as described in Step 1) was dissolved in 2.0 ml of 1,2-dichloroethane. 0.024 ml of pyridine and 0.026 ml of propionyl chloride were then added, whilst ice-cooling, to the resulting solution, and then the mixture was stirred for 30 minutes. At the end of this time, the reaction mixture was diluted with 15 ml of ethyl acetate and washed, in turn, with 0.5N aqueous hydrochloric acid, with water, with a 4% w/v aqueous solution of sodium bicarbonate and with water. After the mixture had been dried over anhydrous sodium sulphate, the solvent was removed by evaporation to dryness under reduced pressure. The residue was dissolved in 2.0 ml of methanol and stirred at room temperature for 30 minutes in the presence of a catalytic amount of p-toluenesulphonic acid monohydrate. The reaction mixture was then diluted with 15 ml of ethyl acetate, washed, in turn, with a 4% w/v aqueous solution of sodium bicarbonate and with water and dried over anhydrous sodium sulphate. The solvent was then removed by evaporation to dryness under reduced pressure, and the residue was purified by column chromatography through silica gel (ODS treated), eluted with 80% v/v aqueous acetonitrile, to give 0.187 g of the title compound.

Nuclear Magnetic Resonance Spectrum ($CDCℓ_3$) δ ppm:
1.87 (3H, singlet);
3.20 (1H, doublet, J = 9.9 Hz);
3.95 (1H, doublet, J = 6.2 Hz);
7.08 (1H, singlet).

Following a similar procedure to that described in Example 59, the compounds of Examples 60 to 63 were obtained.

EXAMPLE 60

13-[2-(4-Chloroacetamidophenyl)ethoxy]milbemycin $A_4$

Mass Spectrum m/e: 753 ($M^+$, $C_{42}H_{56}CℓNO_9$).
Nuclear Magnetic Resonance Spectrum ($CDCℓ_3$) δ ppm:
1.87 (3H, singlet);
3.20 (1H, doublet, J = 9.9 Hz);
3.96 (1H, doublet, J = 6.2 Hz);
4.19 (2H, singlet);
8.18 (1H, singlet).

EXAMPLE 61

13-[2-(4-Isonicotinoylaminophenyl)ethoxy]milbemycin $A_4$

Nuclear Magnetic Resonance Spectrum ($CDCℓ_3$) δ ppm:
1.79 (3H, singlet);
3.21 (1H, doublet, J = 9.5 Hz);
3.80 (1H, doublet, J = 5.5 Hz);
7.70 (2H, doublet, J = 6.2 Hz);
7.80 (1H, singlet);
8.80 (2H, doublet, J = 6.2 Hz).

EXAMPLE 62

13-[2-(4-Ethanesulphonylaminophenyl)ethoxy]milbemycin $A_4$

Mass Spectrum m/e: 769 ($M^+$, $C_{42}H_{59}NO_{10}S$).
Nuclear Magnetic Resonance Spectrum ($CDCℓ_3$) δ ppm:
1.87 (3H, singlet);
3.10 (2H, quartet, J = 7.5 Hz);
3.20 (1H, doublet, J = 9.9 Hz);
3.95 (1H, doublet, J = 6.2 Hz);
6.37 (1H, singlet).

EXAMPLE 63

13-[2-(4-Propanesulphonylaminophenyl)ethoxy]milbemycin $A_4$

Mass Spectrum m/e: 783 ($M^+$, $C_{43}H_{61}NO_{10}S$).
Nuclear Magnetic Resonance Spectrum ($CDCl_3$) $\delta$ ppm:
1.87 (3H, singlet);
3.20 (1H, doublet, J = 9.9 Hz);
3.96 (1H, doublet, J = 6.2 Hz);
6.34 (1H, singlet).

EXAMPLE 64

13-[2-(4-Methoxyacetamidophenyl)ethoxy]milbemycin $A_4$

0.130 g of 13-[2-(4-aminophenyl)ethoxy]milbemycin $A_4$ (prepared by a similar procedure to that described in Example 35) was dissolved in 1.1 ml of 1,2-dichloroethane. 0.024 ml of pyridine and 0.024 g of methoxyacetyl chloride were added, while ice-cooling, to the resulting solution, and then the mixture was stirred for 30 minutes. At the end of this time, the reaction mixture was diluted with 15 ml of ethyl acetate and washed with 0.5N hydrochloric acid, with water, with a 4% w/v aqueous solution of sodium bicarbonate and with water, in that order. After the mixture had been dried over anhydrous sodium sulphate, the solvent was removed by evaporation to dryness under reduced pressure. The residue was purified by column chromatography through silica gel (ODS treated), eluted with 80% v/v aqueous acetonitrile) to give 0.123 g of the title compound.
Mass Spectrum m/e: 749 ($M^+$, $C_{43}H_{59}NO_{10}$).
Nuclear Magnetic Resonance Spectrum ($CDCl_3$) $\delta$ ppm:
1.87 (3H, singlet);
3.21 (1H, doublet, J = 9.9 Hz);
3.50 (3H, singlet);
3.95 (1H, doublet, J = 6.2 Hz);
4.01 (2H, singlet);
8.19 (1H, singlet).

Following a similar procedure to that described in Example 64, the compounds of Examples 65 to 83 were obtained and have the properties shown below.

EXAMPLE 65

13-[2-(4-Phenoxyacetamidophenyl)ethoxy]milbemycin $A_4$

Mass Spectrum (m/e): 811 ($M^+$, $C_{48}H_{61}NO_{10}$).
Nuclear Magnetic Resonance Spectrum ($CDCl_3$) $\delta$ ppm:
1.83 (3H, singlet);
3.21 (1H, doublet, J = 9.9 Hz);
3.95 (1H, doublet, J = 6.2 Hz);
4.61 (2H, singlet);
8.23 (2H, singlet).

EXAMPLE 66

13-{2-[4-(2-Furoyl)aminophenyl]ethoxy}milbemycin $A_4$

Mass Spectrum (m/e): 771 ($M^+$, $C_{45}H_{57}NO_{10}$).
Nuclear Magnetic Resonance Spectrum ($CDCl_3$) $\delta$ ppm:
1.87 (3H, singlet);
3.21 (1H, doublet, J = 9.9 Hz);
3.95 (1H, doublet, J = 6.2 Hz);
8.03 (1H, singlet).

EXAMPLE 67

13-{2-[4-(2-Thenoyl)aminophenyl]ethoxy}milbemycin $A_4$

Nuclear Magnetic Resonance Spectrum ($CDCl_3$) δ ppm:
1.87 (3H, singlet);
3.21 (1H, doublet, J = 9.9 Hz);
3.95 (1H, doublet, J = 6.2 Hz);
7.13, 7.54 & 7.61 (1H x 3, multiplet);
7.62 (1H, singlet).

EXAMPLE 68

13-[2-(4-Cyclobutylcarbonylaminophenyl)ethoxy]milbemycin $A_4$

Mass Spectrum (m/e): 759 ($M^+$, $C_{45}H_{61}NO_9$).
Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz);
6.95 (1H, singlet).

EXAMPLE 69

13-{2-[4-(Methoxalylamino)phenyl]ethoxy}milbemycin $A_4$

Mass Spectrum (m/e): 763 ($M^+$, $C_{43}H_{57}NO_{11}$).
Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz);
3.97 (1H, singlet);
8.80 (1H, singlet).

EXAMPLE 70

13-{2-[4-(Ethoxycarbonylacetamido)phenyl]ethoxy}milbemycin $A_4$,

Nuclear Magnetic Resonance Spectrum ($CDCl_3$) δ ppm:
1.87 (3H, singlet);
3.20 (1H, doublet, J = 9.5 Hz);
3.46 (2H, singlet);
3.95 (1H, doublet, J = 6.2 Hz);
4.26 (2H, quartet, J = 7.0 Hz);
9.15 (1H, singlet).

EXAMPLE 71

13-[2-(4-Cyclopropylcarbonylaminophenyl)ethoxy]milbemycin $A_4$

Mass Spectrum (m/e): 745 ($M^+$, $C_{45}H_{59}NO_9$).
Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz);
7.29 (1H, singlet).

EXAMPLE 72

13-[2-(4-Butyramidophenyl)ethoxy]milbemycin $A_4$

Mass Spectrum (m/e): 747 ($M^+$, $C_{44}H_{61}NO_9$).
Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz);
7.06 (1H, singlet).

EXAMPLE 73

13-[2-(4-Crotonoylaminophenyl)ethoxy]milbemycin $A_4$

Mass Spectrum (m/e): 745 ($M^+$, $C_{45}H_{59}NO_9$).
Nuclear Magnetic Resonance Spectrum ($CDCl_3$) δ ppm:
1.87 (3H, singlet);
3.21 (1H, doublet, J = 9.5 Hz);
3.95 (1H, doublet, J = 6.2 Hz);
7.05 (1H, singlet).

EXAMPLE 74

13-{2-[4-(4-Cyanobenzamido)phenyl]ethoxy}milbemycin $A_4$,

Mass Spectrum (m/e): 770 ($M^+$ - 36).
Nuclear Magnetic Resonance Spectrum ($CDCl_3$) δ ppm:
1.87 (3H, singlet);
3.22 (1H, doublet, J = 9.5 Hz);
3.95 (1H, doublet, J = 6.2 Hz);
7.79 (1H, singlet).

EXAMPLE 75

13-{2-[4-(4-Methoxycarbonylbenzamido)phenyl]ethoxy}milbemycin $A_4$,

Mass Spectrum (m/e): 803 ($M^+$ - 36).
Nuclear Magnetic Resonance Spectrum ($CDCl_3$) δ ppm:
1.87 (3H, singlet);
3.22 (1H, doublet, J = 9.5 Hz);
3.95 (1H, doublet, J = 6.2 Hz);
3.96 (3H, singlet);
7.79 (1H, singlet).

EXAMPLE 76

13-[2-(4-Trifluoroacetamidophenyl)ethoxy]milbemycin $A_4$

Mass Spectrum (m/e): 773 ($M^+$, $C_{42}H_{54}F_3NO_9$).
Nuclear Magnetic Resonance Spectrum ($CDCl_3$) δ ppm:
1.87 (3H, singlet);
3.20 (1H, doublet, J = 9.8 Hz);
3.95 (1H, doublet, J = 5.9 Hz);
9.86 (1H, singlet).

EXAMPLE 77

13-[2-(4-Fluoroacetamidophenyl)ethoxy]milbemycin $A_4$

Mass Spectrum (m/e): 737 ($M^+$, $C_{42}H_{56}FNO_9$).
Nuclear Magnetic Resonance Spectrum ($CDCl_3$) $\delta$ ppm:
1.87 (3H, singlet);
3.21 (1H, doublet, J = 9.8 Hz);
3.95 (1H, doublet, J = 5.9 Hz);
4.92 (2H, doublet, J = 47.4 Hz).

EXAMPLE 78

13-[2-(4-Cyanomethanesulphonylaminophenyl)ethoxy]milbemycin $A_4$

Nuclear Magnetic Resonance Spectrum ($CDCl_3$) $\delta$ ppm:
1.87 (3H, singlet);
3.20 (1H, doublet, J = 9.9 Hz);
3.96 (2H, singlet);
3.96 (1H, doublet, J = 6.2 Hz).

EXAMPLE 79

13-{2-[4-(4-Methoxybenzenesulphonylamino)phenyl]ethoxy}milbemycin $A_4$,

Mass Spectrum (m/e): 847 ($M^+$, $C_{47}H_{61}NO_{11}S$).
Nuclear Magnetic Resonance Spectrum ($CDCl_3$) $\delta$ ppm:
1.87 (3H, singlet);
3.16 (1H, doublet, J = 9.9 Hz);
3.83 (3H, singlet);
3.96 (1H, doublet, J = 6.2 Hz);
6.39 (1H, singlet).

EXAMPLE 80

13-[2-(4-Isopropoxycarbonylaminophenyl)ethoxy]milbemycin $A_4$

Mass Spectrum (m/e): 764 ($M^+$ + 1).
Nuclear Magnetic Resonance Spectrum ($CDCl_3$) $\delta$ ppm:
1.87 (3H, singlet);
3.21 (1H, doublet, J = 9.9 Hz);
3.96 (1H, doublet, J = 6.2 Hz);
5.01 (1H, multiplet);
6.47 (1H, singlet).

EXAMPLE 81

13-[2-(4-Vinyloxycarbonylaminophenyl)ethoxy]milbemycin $A_4$

Nuclear Magnetic Resonance Spectrum ($CDCl_3$) $\delta$ ppm:
1.87 (3H, singlet);
3.20 (1H, doublet, J = 9.9 Hz);
3.95 (1H, doublet, J = 6.2 Hz);
4.53 (1H, doublet of doublets, J = 1.8 & 6.2 Hz);
4.83 (1H, doublet of doublets, J = 1.8 & 13.9 Hz);
6.65 (1H, singlet).

EXAMPLE 82

13-[2-(4-Allyloxycarbonylaminophenyl)ethoxy]milbemycin $A_4$

Nuclear Magnetic Resonance Spectrum ($CDCl_3$) δ ppm:
1.87 (3H, singlet);
3.20 (1H, doublet, J = 9.9 Hz);
3.95 (1H, doublet, J = 6.2 Hz);
4.66 (2H, multiplet);
5.26 (1H, multiplet);
5.36 (1H, multiplet);
5.97 (1H, multiplet);
6.57 (1H, singlet).

EXAMPLE 83

13-[2-(4-Ethoxycarbonylamino-3-methoxyphenyl)ethoxy]milbemycin $A_4$

Mass Spectrum (m/e): 779 ($M^+$, $C_{44}H_{61}NO_{11}$).
Nuclear Magnetic Resonance Spectrum ($CDCl_3$) δ ppm:
1.87 (3H, singlet);
3.21 (1H, doublet, J = 9.9 Hz);
3.85 (3H, singlet);
3.96 (1H, doublet, J = 6.2 Hz);
4.22 (1H, quartet, J = 7.0 Hz).

EXAMPLE 84

13-[2-(4-Cyanoacetamidophenyl)ethoxy]milbemycin $A_4$

0.050 ml of pyridine and 0.100 g of 2-chloroformyl-1,2,4-triazolo[4,3-a]pyridin-3-one were added to a solution of 0.0425 g of cyanoacetic acid in 2.5 ml of methylene chloride, and then the mixture was stirred at room temperature for 30 minutes. At the end of this time, 0.203 g of 13-[2-(4-aminophenyl)ethoxy]-milbemycin $A_4$ (prepared by a similar procedure to that described in Example 35) was added to the mixture, and then the whole mixture was stirred at room temperature for a further 1 hour. At the end of this time, the reaction mixture was diluted with 15 ml of ethyl acetate and filtered. The filtrate was washed, in turn, with 0.5N aqueous hydrochloric acid, with water, with a 4% w/v aqueous solution of sodium bicarbonate and again with water, after which it was dried over anhydrous sodium sulphate. The solvent was then removed by evaporation to dryness under reduced pressure, and the residue was purified by column chromatography through silica gel (ODS treated), eluted with 80% v/v aqueous acetonitrile, to give 0.190 g of the title compound.
Nuclear Magnetic Resonance Spectrum ($CDCl_3$) δ ppm:
1.87 (3H, singlet);
3.20 (1H, doublet, J = 9.9 Hz);
3.54 (2H, singlet);
3.95 (1H, doublet, J = 6.2 Hz);
7.70 (1H, singlet).

Following a similar procedure to that described in Example 84, the compounds of Examples 85 to 95 were obtained and had the properties shown below.

EXAMPLE 85

13-[2-(4-Methoxycarbonylacetamidophenyl)ethoxy]milbemycin $A_4$

Nuclear Magnetic Resonance Spectrum ($CDCl_3$) δ ppm:
1.87 (3H, singlet);
3.21 (1H, doublet, J = 9.5 Hz);
3.48 (2H, singlet);

3.81 (3H, singlet);
3.95 (1H, doublet, J = 6.2 Hz);
9.09 (1H, singlet).

EXAMPLE 86

13-[2-(4-Difluoroacetamidophenyl)ethoxy]milbemycin $A_4$

Mass Spectrum (m/e): 755 ($M^+$, $C_{42}H_{55}F_2NO_9$).
Nuclear Magnetic Resonance Spectrum ($CDCl_3$) δ ppm:
1.87 (3H, singlet);
3.20 (1H, doublet, J = 9.8 Hz);
3.95 (1H, doublet, J = 5.9 Hz);
6.01 (1H, triplet, J = 54.4 Hz);
7.85 (1H, singlet).

EXAMPLE 87

13-{2-[4-(2-Cyanopropionamido)phenyl]ethoxy}milbemycin $A_4$

Mass Spectrum (m/e): 758 ($M^+$, $C_{44}H_{58}N_2O_9$).
Nuclear Magnetic Resonance Spectrum ($CDCl_3$) δ ppm:
1.87 (3H, singlet);
3.22 (1H, doublet, J = 9.5 Hz);
3.95 (1H, doublet, J = 5.9 Hz);
3.57 (1H, quartet, J = 7.3 Hz);
7.71 (1H, singlet).

EXAMPLE 88

13-[2-(4-Cyanomethylthioacetamidophenyl)ethoxy]milbemycin $A_4$

Nuclear Magnetic Resonance Spectrum ($CDCl_3$) δ ppm:
1.87 (3H, singlet);
3.20 (1H, doublet, J = 9.5 Hz);
3.50 & 3.55 (2H x 2, each singlet);
3.95 (1H, doublet, J = 6.2 Hz);
7.87 (1H, singlet).

EXAMPLE 89

13-[2-(4-Acetylcarbonylaminophenyl)ethoxy]milbemycin $A_4$

Mass Spectrum (m/e): 747 ($M^+$, $C_{43}H_{57}NO_{10}$).
Nuclear Magnetic Resonance Spectrum ($CDCl_3$) δ ppm:
1.87 (3H, singlet);
2.57 (3H, singlet);
3.20 (1H, doublet, J = 9.9 Hz);
3.95 (1H, doublet, J = 6.2 Hz);
8.68 (1H, singlet).

EXAMPLE 90

13-{2-[4-(1-t-Butoxycarbonylpiperidine-4-carbonylamino)phenyl]ethoxy}milbemycin $A_4$

Nuclear Magnetic Resonance Spectrum ($CDCl_3$) δ ppm:
1.59 (9H, singlet);
1.87 (3H, singlet);

3.20 (1H, doublet, J = 9.5 Hz);
3.95 (1H, doublet, J = 6.3 Hz);
7.17 (1H, singlet).

EXAMPLE 91

13-[2-(4-Acryloylaminophenyl)ethoxy]milbemycin $A_4$

Mass Spectrum (m/e): 731 ($M^+$, $C_{43}H_{57}NO_9$).
Nuclear Magnetic Resonance Spectrum ($CDC\ell_3$) δ ppm:
1.87 (3H, singlet);
3.21 (1H, doublet, J = 9.9 Hz);
3.95 (1H, doublet, J = 6.2 Hz);
6.22 (1H, doublet of doublets, J = 10.3 & 16.9 Hz);
6.42 (1H, doublet of doublets, J = 1.5 & 16.9 Hz);
8.68 (1H, singlet).

EXAMPLE 92

13-{2-[4-(2-Butynoylamino)phenyl]ethoxy}milbemycin $A_4$

Mass Spectrum (m/e): 743 ($M^+$, $C_{44}H_{57}NO_9$).
Nuclear Magnetic Resonance Spectrum ($CDC\ell_3$) δ ppm:
1.87 (3H, singlet);
2.00 (3H, singlet);
3.20 (1H, doublet, J = 9.9 Hz);
3.95 (1H, doublet, J = 6.2 Hz);
7.32 (1H, singlet).

EXAMPLE 93

13-{2-[4-(Pyrazin-2-ylcarbonylamino)phenyl]ethoxy}milbemycin $A_4$

Mass Spectrum (m/e): 783 ($M^+$, $C_{45}H_{57}N_3O_9$).
Nuclear Magnetic Resonance Spectrum ($CDC\ell_3$) δ ppm:
1.87 (3H, singlet);
3.22 (1H, doublet, J = 9.9 Hz);
3.95 (1H, doublet, J = 6.2 Hz);
8.59 (1H, doublet of doublets, J = 1.5 & 2.6 Hz);
8.81 (1H, doublet, J = 2.6 Hz);
9.52 (1H, doublet, J = 1.5 Hz);
9.63 (1H, singlet).

EXAMPLE 94

13-{2-[4-(3,4-dihydropyran-2-ylcarbonylamino)phenyl]ethoxy}milbemycin $A_4$ Mass Spectrum (m/e): 787 ($M^+$, $C_{46}H_{61}NO_{10}$).
Nuclear Magnetic Resonance Spectrum ($CDC\ell_3$) δ ppm:
1.87 (3H, singlet);
3.21 (1H, doublet, J = 9.9 Hz);
3.95 (1H, doublet, J = 6.2 Hz);
4.87 (1H, multiplet);
6.23 (1H, multiplet);
8.17 (1H, singlet).

EXAMPLE 95

13-[2-(4-Cinnamoylaminophenyl)ethoxy]milbemycin $A_4$ Mass Spectrum (m/e): 789 ($M^+$ - 18).
Nuclear Magnetic Resonance Spectrum ($CDCl_3$) $\delta$ ppm:
1.87 (3H, singlet);
3.22 (1H, doublet, J = 9.5 Hz);
3.95 (1H, doublet, J = 5.9 Hz);
6.53 (1H, doublet, J = 15.6 Hz);
7.28 (1H, doublet, J = 15.6 Hz).

EXAMPLE 96

13-[2-(4-Formamidophenyl)ethoxy]-5-O-formylmilbemycin $A_4$

0.360 ml of pyridine and 0.420 ml of acetic anhydride were added, whilst ice-cooling, to a solution of 0.170 ml of formic acid in 1.0 ml of methylene chloride, and then the mixture was stirred for 15 minutes. At the end of this time, 0.150 g of 13-[2-(4-aminophenyl)ethoxy]milbemycin $A_4$ (prepared by a similar procedure to that described in Example 35) was added, and then the whole mixture was stirred at room temperature for a further 20 minutes. At the end of this time, the reaction mixture was diluted with 15 ml of ethyl acetate and washed with 0.5N aqueous hydrochloric acid, with water, with a 4% w/v aqueous solution of sodium bicarbonate and again with water, in that order, after which it was dried over anhydrous sodium sulphate. The solvent was then removed by evaporation to dryness under reduced pressure, and the resulting residue was purified by column chromatography through silica gel, eluted with a 3 : 1 by volume mixture of cyclohexane and ethyl acetate, to give 0.119 g of the title compound.
Mass Spectrum m/e: 705 ($M^+$ - 28).
Nuclear Magnetic Resonance Spectrum ($CDCl_3$) $\delta$ ppm:
1.87 (3H, singlet);
3.20 (1H, doublet, J = 9.9 Hz);
4.08 (1H, singlet);
5.67 (1H, doublet, J = 6.2 Hz).

EXAMPLE 97

13-[2-(4-Glycylaminophenyl)ethoxy]milbemycin $A_4$

0.420 ml of triethylamine and 0.600 g of 2-chloroformyl-1,2,4-triazolo[4,3-a]pyridin-3-one were added to a mixture of 0.751 g of N-trichloroethoxycarbonylglycine and 3.0 ml of 1,2-dichloroethane, and then the mixture was stirred at room temperature for 30 minutes. At the end of this time, 1.000 g of 13-[2-(4-aminophenyl)ethoxy]milbemycin $A_4$ (prepared by a similar procedure to that described in Example 35) was added, and then the whole mixture was stirred at room temperature for a further 1 hour. The reaction mixture was diluted with 25 ml of ethyl acetate and filtered. The filtrate was washed with 0.5N aqueous hydrochloric acid, with water, with a 4% w/v aqueous solution of sodium bicarbonate and again with water, in that order, after which it was dried over anhydrous sodium sulphate. The solvent was then removed by evaporation to dryness under reduced pressure, and the resulting residue was purified by column chromatography through silica gel, eluted with a 3 : 1 by volume mixture of cyclohexane and ethyl acetate, to give 1.530 g of 13-[2-(4-trichloroethoxycarbonylglycylaminophenyl)ethoxy]milbemycin $A_4$. The whole of this product was dissolved in 6.0 ml of 90% acetic acid, 1.53 g of zinc powder were added, and then the mixture was stirred for 20 minutes, whilst cooling with water. The mixture was then diluted with 250 ml of ethyl acetate and the insoluble matter was filtered off. The filtrate was washed with water, and the white crystals which precipitated were collected by filtration to give 0.445 g of the title compound in the form of the hydrochloride. The filtrate was then subjected to column chromatography through silica gel (ODS treated), eluted with 90% v/v aqueous acetonitrile containing 1% by volume acetic acid, to give a further 0.054 g of the title compound as the hydrochloride.

EXAMPLE 98

13-[2-(4-N-Acetylglycylaminophenyl)ethoxy]milbemycin $A_4$

0.100 g of 13-[2-(4-glycylaminophenyl)ethoxy]milbemycin $A_4$ (prepared as described in Example 97) was suspended in 1.0 ml of tetrahydrofuran. 0.042 ml of triethylamine and 0.014 ml of acetic anhydride were then added, whilst ice-cooling, to the suspension, and then the mixture was stirred for 30 minutes. The reaction mixture was then diluted with 15 ml of ethyl acetate and washed with 0.5N aqueous hydrochloric acid, with water, with a 4% w/v aqueous solution of sodium bicarbonate and again with water, in that order, after which it was dried over anhydrous sodium sulphate. The solvent was then removed by evaporation to dryness under reduced pressure, and the resulting residue was purified by column chromatography through silica gel (ODS treated), eluted with 80% v/v aqueous acetonitrile, to give 0.079 g of the title compound.
Mass Spectrum m/e: 776 ($M^+$, $C_{44}H_{60}N_2O_{10}$).
Nuclear Magnetic Resonance Spectrum ($CDC\ell_3$) δ ppm:
1.87 (3H, singlet);
3.20 (1H, doublet, J = 9.9 Hz);
3.95 (1H, doublet, J = 6.2 Hz);
4.08 (2H, doublet, J = 5.5 Hz);
6.44 (1H, singlet).

Following a similar procedure to that described in Example 98, the compounds of Examples 99 to 101 were obtained.

EXAMPLE 99

13-[2-(4-N-Methoxycarbonylglycylaminophenyl)ethoxy]milbemycin $A_4$

Nuclear Magnetic Resonance Spectrum ($CDC\ell_3$) δ ppm:
1.87 (3H, singlet);
3.20 (1H, doublet, J = 9.5 Hz);
3.75 (3H, singlet);
4.00 (2H, singlet);
7.83 (1H, singlet).

EXAMPLE 100

13-{2-[4-(3-Methylureidoacetamido)phenyl]ethoxy -milbemycin $A_4$

Nuclear Magnetic Resonance Spectrum ($CDC\ell_3$) δ ppm:
1.87 (3H, singlet);
2.78 (3H, doublet, J = 4.8 Hz);
3.20 (1H, doublet, J = 9.9 Hz);
3.95 (1H, doublet, J = 6.2 Hz);
4.04 (2H, doublet, J = 5.1 Hz);
4.83 (1H, quartet, J = 4.8 Hz);
5.59 (1H, triplet, J = 5.1 Hz).

EXAMPLE 101

13-{2-[4-(3-Phenylureidoacetamido)phenyl]ethoxy -milbemycin $A_4$

Nuclear Magnetic Resonance Spectrum ($CDC\ell_3$) δ ppm:
1.86 (3H, singlet);
3.20 (1H, doublet, J = 9.9 Hz);
3.95 (1H, doublet, J = 5.9 Hz);
4.13 (2H, doublet, J = 5.4 Hz);
6.38 (1H, triplet, J = 5.4 Hz);
6.36 & 8.83 (1H x 2, singlet).

EXAMPLE 102

13-{2-[4-(1,2,4-Triazolo[4.3-a]pyridin-3-on-2-ylcarbonylamino)phenyl]ethoxy milbemycin $A_4$

0.150 ml of pyridine and 0.300 g of 2-chloroformyl-1,2,4-triazolo[4,3-a]pyridin-3-one were added, whilst ice-cooling, to a solution of 1.020 g of 13-[2-(4-aminophenyl)ethoxy]milbemycin $A_4$ (prepared by a similar procedure to that described in Example 35) in 10.0 ml of 1,2-dichloroethane, and then the mixture was stirred at room temperature for 30 minutes. At the end of this time, the reaction mixture was diluted with 50 ml of ethyl acetate and filtered. The filtrate was washed, in turn, with 0.5N aqueous hydrochloric acid, with water, with a 4% w/v aqueous solution of sodium bicarbonate and with water, after which it was dried over anhydrous sodium sulphate. The solvent was then removed by evaporation to dryness under reduced pressure, and the resulting residue was purified by column chromatography through silica gel (ODS treated), eluted with 80% v/v aqueous acetonitrile, to give 1.136 g of the title compound.
Nuclear Magnetic Resonance Spectrum ($CDC\ell_3$) $\delta$ ppm:

1.87 (3H, singlet);
3.22 (1H, doublet, J = 9.9 Hz);
3.96 (1H, doublet, J = 6.2 Hz);
6.60 (1H, multiplet);
7.80 (1H, doublet of doublets, J = 1.1 & 7.3 Hz);
10.10 (1H, singlet).

EXAMPLE 103

13-{2-[p-3-(2-Hydroxyethyl)ureidophenyl]ethoxy -milbemycin $A_4$,

0.135 g of 13-{2-[4-(1,2,4-triazolo[4.3-a]pyridin-3-on-2-ylcarbonylamino)phenyl]ethoxy milbemycin $A_4$ - (prepared as described in Example 102) was dissolved in 1.0 ml of dimethylformamide. 0.020 g of ethanolamine was added, and then the mixture was stirred at room temperature for 20 minutes. At the end of this time, the reaction mixture was diluted with 15 ml of ethyl acetate and washed, in turn, with 0.5N aqueous hydrochloric acid, with water, with a 4% w/v aqueous solution of sodium bicarbonate and with water, after which it was dried over anhydrous sodium sulphate. The solvent was then removed by evaporation to dryness under reduced pressure, and the resulting residue was purified by column chromatography through silica gel (ODS treated), eluted with 80% v/v aqueous acetonitrile, to give 0.106 g of the title compound. Mass Spectrum m/e: 677 ($M^+$ - 87).
Nuclear Magnetic Resonance Spectrum ($CDC\ell_3$) $\delta$ ppm:

1.87 (3H, singlet);
2.40 (1H, doublet, J = 8.5 Hz);
3.20 (1H, doublet, J = 9.9 Hz);
3.4 (2H, multiplet);
3.7 (2H, multiplet);
3.95 (1H, doublet, J = 5.9 Hz);
6.66 (1H, singlet).

Following a similar procedure to that described in Example 103, the compounds of Examples 104 to 120 were obtained and had the properties shown below.

EXAMPLE 104

13-[2-(p-Ureidophenyl)ethoxy]milbemycin $A_4$

Mass Spectrum m/e: 677 ($M^+$ - 43).
Nuclear Magnetic Resonance Spectrum ($CDC\ell_3$) $\delta$ ppm:

1.87 (3H, singlet);
2.38 (1H, doublet, J = 8.1 Hz);
3.21 (1H, doublet, J = 9.5 Hz);
3.96 (1H, doublet, J = 6.2 Hz);
4.68 (2H, singlet);
6.45 (1H, singlet).

EXAMPLE 105

13-{2-[4-(3,3-Dimethylureido)phenyl]ethoxy}milbemycin $A_4$

Mass Spectrum m/e: 677 ($M^+$ - 71).
Nuclear Magnetic Resonance Spectrum ($CDCl_3$) δ ppm:
1.87 (3H, singlet);
3.06 (6H, singlet);
3.21 (1H, doublet, J = 9.8 Hz);
3.96 (1H, doublet, J = 6.3 Hz);
6.24 (1H, singlet).

EXAMPLE 106

13-{2-[p-3-(2-Thioethyl)ureidophenyl]ethoxy}milbemycin $A_4$

Mass Spectrum m/e: 758 ($M^+$, $C_{44}H_{58}N_2O_9$).
Nuclear Magnetic Resonance Spectrum ($CDCl_3$) δ ppm:
1.87 (3H, singlet);
2.85 (2H, multiplet);
3.20 (1H, doublet, J = 9.8 Hz);
3.95 (1H, doublet, J = 6.3 Hz);
6.56 % 7.26 (1H x 2, each singlet).

EXAMPLE 107

13-{2-[4-(3-Cyclopropylureido)phenyl]ethoxy}milbemycin $A_4$

Mass Spectrum m/e: 677 ($M^+$ - 83).
Nuclear Magnetic Resonance Spectrum ($CDCl_3$) δ ppm:
1.87 (3H, singlet);
2.59 (1H, multiplet);
3.21 (1H, doublet, J = 9.8 Hz);
3.96 (1H, doublet, J = 5.9 Hz);
4.84 (1H, singlet);
6.77 (1H, singlet).

EXAMPLE 108

13-{2-[p-3-(2-Pyridyl)ureidophenyl]ethoxy}milbemycin $A_4$

Nuclear Magnetic Resonance Spectrum ($CDCl_3$) δ ppm:
1.86 (3H, singlet);
3.23 (1H, doublet, J = 9.8 Hz);
3.96 (1H, doublet, J = 6.3 Hz);
6.83 (1H, doublet, J = 9.3 Hz);
6.94 & 7.64 (2H, multiplet);
8.26 (1H, doublet of doublets, J = 1.5 & 4.9 Hz);
7.92 (1H, singlet);
11.64 (1H, singlet).

EXAMPLE 109

13-{2-[p-3-(2-Thiazolinyl)ureidophenyl]ethoxy}milbemycin $A_4$

Mass Spectrum m/e: 677 ($M^+$ - 128).
Nuclear Magnetic Resonance Spectrum ($CDCl_3$) δ ppm:
1.87 (3H, singlet);

3.21 (1H, doublet, J = 9.9 Hz);
3.3 (2H, multiplet);
3.9 (2H, multiplet);
3.96 (1H, doublet, J = 6.2 Hz).

EXAMPLE 110

13-{2-[p-3-(2-Thiazolyl)ureidophenyl]ethoxy}milbemycin $A_4$

Mass Spectrum m/e: 677 ($M^+$ - 126).
Nuclear Magnetic Resonance Spectrum ($CDCl_3$) δ ppm:
1.79 (3H, singlet);
3.23 (1H, doublet, J = 9.9 Hz);
3.98 (1H, doublet, J = 6.2 Hz);
6.86 & 7.34 (1H x 2, each doublet, J = 3.7 Hz).

EXAMPLE 111

13-{2-[4-(Morpholinocarbonylamino)phenyl]ethoxy}milbemycin $A_4$

Mass Spectrum m/e: 677 ($M^+$ - 113).
Nuclear Magnetic Resonance Spectrum ($CDCl_3$) δ ppm:
1.79 (3H, singlet);
3.21 (1H, doublet, J = 9.5 Hz);
3.47 & 3.74 (4H x 2, multiplet);
3.95 (1H, doublet, J = 6.2 Hz);
6.26 (1H, singlet).

EXAMPLE 112

13-[2-(4-Carbazoylaminophenyl)ethoxy]milbemycin $A_4$

Mass Spectrum m/e: 703 ($M^+$ - 32), 677 ($M^+$ - 58).
Nuclear Magnetic Resonance Spectrum ($CDCl_3$) δ ppm:
1.87 (3H, singlet);
3.21 (1H, doublet, J = 9.9 Hz);
3.84 (2H, broad singlet);
3.96 (1H, doublet, J = 6.2 Hz);
5.96 & 8.07 (1H x 2, each singlet).

EXAMPLE 113

13-{2-[4-(2-Methylcarbazoylamino)phenyl]ethoxy}milbemycin $A_4$

Mass Spectrum m/e: 677 ($M^+$ - 72).
Nuclear Magnetic Resonance Spectrum ($CDCl_3$) δ ppm:
1.87 (3H, singlet);
3.21 (1H, doublet, J = 9.5 Hz);
3.23 (3H, singlet);
3.70 (2H, singlet);
3.96 (1H, doublet, J = 6.2 Hz);
8.52 (1H, singlet).

EXAMPLE 114

13-{2-[4-(3,3-Dimethylcarbazoylamino)phenyl]ethoxy}milbemycin $A_4$

Mass Spectrum m/e: 677 ($M^+$ - 86).
Nuclear Magnetic Resonance Spectrum ($CDCl_3$) $\delta$ ppm:
1.87 (3H, singlet);
2.59 (6H, singlet);
3.21 (1H, doublet, J = 9.9 Hz);
3.96 (1H, doublet, J = 6.2 Hz);
5.13 & 8.06 (1H x 2, each singlet).

EXAMPLE 115

13-{2-[4-(3-Perhydroazepinylureido)phenyl]ethoxy}milbemycin $A_4$

Nuclear Magnetic Resonance Spectrum ($CDCl_3$) $\delta$ ppm:
1.87 (3H, singlet);
3.21 (1H, doublet, J = 9.9 Hz);
3.96 (1H, doublet, J = 6.2 Hz);
5.60 & 8.17 (1H x 2, each singlet).

EXAMPLE 116

13-{2-[4-(3-Morpholinoureido)phenyl]ethoxy}milbemycin $A_4$

Nuclear Magnetic Resonance Spectrum ($CDCl_3$) $\delta$ ppm:
1.87 (3H, singlet);
3.21 (1H, doublet, J = 9.9 Hz);
3.95 (1H, doublet, J = 6.2 Hz);
5.40 & 7.97 (1H x 2, each singlet).

EXAMPLE 117

13-{2-[4-(3-Phenylcarbazoylamino)phenyl]ethoxy}milbemycin $A_4$

Mass Spectrum m/e: 677 ($M^+$ - 134).
Nuclear Magnetic Resonance Spectrum ($CDCl_3$) $\delta$ ppm:
1.87 (3H, singlet);
3.21 (1H, doublet, J = 9.9 Hz);
3.95 (1H, doublet, J = 5.9 Hz);
6.07 & 7.99 (1H x 2, each singlet).

EXAMPLE 118

13-{2-[4-(3-Pyrid-2'-ylcarbazoylamino)phenyl]ethoxy}milbemycin $A_4$

Nuclear Magnetic Resonance Spectrum ($CDCl_3$) $\delta$ ppm:
1.87 (3H, singlet);
3.21 (1H, doublet, J = 9.9 Hz);
3.96 (1H, doublet, J = 6.2 Hz);
6.38, 6.53 & 7.71 (1H x 3, each singlet);
7.64 & 8.21 (1H x 2, each singlet).

EXAMPLE 119

13-{2-[4-(3-Acetylcarbazoylamino)phenyl]ethoxy}milbemycin $A_4$

Nuclear Magnetic Resonance Spectrum ($CDCl_3$) δ ppm:
1.87 (3H, singlet);
1.99 (3H, singlet);
2.47 (1H, doublet, J = 8.1 Hz);
3.20 (1H, doublet, J = 9.5 Hz);
3.95 (1H, doublet, J = 6.2 Hz);
7.90, 7.98 & 8.90 (1H x 3, each singlet).

EXAMPLE 120

13-{2-[4-(3-Benzoylcarbazoylamino)phenyl]ethoxy}milbemycin $A_4$

Mass Spectrum m/e: 677 ($M^+$ - 152).
Nuclear Magnetic Resonance Spectrum ($CDCl_3$) δ ppm:
1.86 (3H, singlet);
2.44 (1H, doublet, J = 8.1 Hz);
3.20 (1H, doublet, J = 9.9 Hz);
3.95 (1H, doublet, J = 6.2 Hz);
7.99, 8.28 & 9.30 (1H x 3, each singlet).

EXAMPLE 121

13-{2-[4-(3-p-Fluorophenylureido)phenyl]ethoxy milbemycin $A_4$

0.020 ml of 4-fluorophenylisocyanate was added to a solution of 0.100 g of 13-[2-(4-aminophenyl)-ethoxy]milbemycin $A_4$ (prepared by a similar procedure to that described in Example 35) in 2.0 ml of tetrahydrofuran, and then the mixture was stirred at room temperature for 20 minutes. At the end of this time, the reaction mixture was diluted with 15 ml of ethyl acetate, washed with water and dried over anhydrous sodium sulphate. The solvent was then removed by evaporation to dryness under reduced pressure, and the resulting residue was purified by column chromatography through silica gel (ODS treated), eluted with 80% v/v aqueous acetonitrile, to give 0.118 g of the title compound.
Mass Spectrum m/e: 677 ($M^+$ - 137).
Nuclear Magnetic Resonance Spectrum ($CDCl_3$) δ ppm:
1.87 (3H, singlet);
3.21 (1H, doublet, J = 9.9 Hz);
3.96 (1H, doublet, J = 6.2 Hz);
6.51 & 6.58 (1H x 2, singlet).

Following a similar procedure to that described in Example 121, the compounds of Examples 122 to 130 were obtained and had the properties shown below.

EXAMPLE 122

13-{2-[4-(3-Ethylureido)phenyl]ethoxy milbemycin $A_4$

Nuclear Magnetic Resonance Spectrum ($CDCl_3$) δ ppm:
1.87 (3H, singlet);
3.21 (1H, doublet, J = 9.9 Hz);
3.96 (1H, doublet, J = 6.2 Hz);
4.60 (1H, triplet, J = 6.0 Hz);
6.14 (1H, singlet);
7.17 (4H, multiplet).

EXAMPLE 123

13-{2-[4-(3-Propylureido)phenyl]ethoxy milbemycin $A_4$

Mass Spectrum m/e: 677 ($M^+$ - 85).
Nuclear Magnetic Resonance Spectrum ($CDC\ell_3$) δ ppm:
1.87 (3H, singlet);
3.21 (1H, doublet, J = 9.9 Hz);
3.96 (1H, doublet, J = 6.2 Hz);
6.16 (1H, singlet);
7.17 (4H, multiplet).

EXAMPLE 124

13-{2-[4-(3-2'-Chloroethylureido)phenyl]ethoxy milbemycin $A_4$

Mass Spectrum m/e: 677 ($M^+$ - 105).
Nuclear Magnetic Resonance Spectrum ($CDC\ell_3$) δ ppm:
1.87 (3H, singlet);
3.21 (1H, doublet, J = 9.9 Hz);
3.96 (1H, doublet, J = 6.2 Hz);
5.14 (1H, triplet, J = 5.5 Hz);
6.30 (1H, singlet);
7.19 (4H, multiplet).

EXAMPLE 125

13-{2-[4-(3-Allylureido)phenyl]ethoxy milbemycin $A_4$

Nuclear Magnetic Resonance Spectrum ($CDC\ell_3$) δ ppm:
1.87 (3H, singlet);
3.21 (1H, doublet, J = 9.9 Hz);
3.86 (2H, multiplet);
3.96 (1H, doublet, J = 6.2 Hz);
4.75 (1H, triplet, J = 5.9 Hz);
5.13, 5.19 & 5.80 (1H x 3, multiplet);
6.26 (1H, singlet).

EXAMPLE 126

13-{2-[4-(3-Propionylureido)phenyl]ethoxy milbemycin $A_4$

Mass Spectrum m/e: 677 ($M^+$ - 99).
Nuclear Magnetic Resonance Spectrum ($CDC\ell_3$) δ ppm:
1.83 (3H, singlet);
2.44 (2H, quartet, J = 7.3 Hz);
3.21 (1H, doublet, J = 9.8 Hz);
3.96 (1H, doublet, J = 6.3 Hz);
8.49 & 10.48 (1H x 2, each singlet).

EXAMPLE 127

13-{2-[4-(3-Benzoylureido)phenyl]ethoxy milbemycin $A_4$

Mass Spectrum m/e: 677 ($M^+$ - 147). Nuclear Magnetic Resonance Spectrum ($CDC\ell_3$) δ ppm:
1.87 (3H, singlet);
3.22 (1H, doublet, J = 9.8 Hz);
3.96 (1H, doublet, J = 6.3 Hz);

9.05 & 10.79 (1H x 2, each singlet).

EXAMPLE 128

13-{2-[4-(3-Methanesulphonylureido)phenyl]ethoxy -milbemycin $A_4$

Mass Spectrum m/e: 677 ($M^+$ - 121).
Nuclear Magnetic Resonance Spectrum ($CDCl_3$) δ ppm:
1.83 (3H, singlet);
3.21 (1H, doublet, J = 9.5 Hz);
3.29 (3H, singlet);
3.95 (1H, doublet, J = 6.2 Hz);
8.11 (1H, singlet).

EXAMPLE 129

13-{2-[4-3'-Methyl(thioureido)phenyl]ethoxy milbemycin $A_4$

Mass Spectrum m/e: 677 ($M^+$ - 73).
Nuclear Magnetic Resonance Spectrum ($CDCl_3$) δ ppm:
1.87 (3H, singlet);
3.13 (3H, doublet, J = 4.8 Hz);
3.21 (1H, doublet, J = 9.9 Hz);
3.96 (1H, doublet, J = 6.2 Hz);
5.94 (1H, broad singlet);
7.57 (1H, singlet).

EXAMPLE 130

13-{2-[4-3'-Ethyl(thioureido)phenyl]ethoxy milbemycin $A_4$

Mass Spectrum m/e: 677 ($M^+$ - 87).
Nuclear Magnetic Resonance Spectrum ($CDCl_3$) δ ppm:
1.87 (3H, singlet);
3.20 (1H, doublet, J = 9.9 Hz);
3.65 (2H, multiplet);
3.95 (1H, doublet, J = 6.2 Hz);
5.77 (1H, multiplet);
7.50 (1H, singlet).

EXAMPLE 131

13-[2-(4-Formimidoylaminophenyl)ethoxy]milbemycin $A_4$

0.066 g of ethyl formimidate hydrochloride was added to a solution of 0.306 g of 13-[2-(4-aminophenyl)-ethoxy]milbemycin $A_4$ (prepared by a similar procedure to that described in Example 35) in 2.0 ml of methanol, and then the mixture was stirred at room temperature for 30 minutes. At the end of this time, the solvent was removed by evaporation to dryness under reduced pressure, and the resulting residue was purified by column chromatography through silica gel (ODS treated), eluted with 55% v/v aqueous acetonitrile, to give 0.076 g of the title compound. Mass Spectrum m/e: 677 ($M^+$ - 27).
Nuclear Magnetic Resonance Spectrum ($CDCl_3$) δ ppm:
1.86 (3H, singlet);
3.19 (1H, doublet, J = 9.5 Hz);
3.95 (1H, doublet, J = 5.9 Hz);
8.25 (1H, singlet).

EXAMPLE 132

13-[2-(4-Benzimidoylaminophenyl)ethoxy]milbemycin $A_4$

0.170 g of methyl thiobenzimidate hydroiodide was added to a solution of 0.306 g of 13-[2-(4-aminophenyl)ethoxy]milbemycin $A_4$ (prepared by a similar procedure to that described in Example 35) in 2.0 ml of methanol, and then the mixture was stirred at room temperature for 40 minutes. At the end of this time, the solvent was removed by evaporation to dryness under reduced pressure. The resulting residue was diluted with ethyl acetate and was washed, in turn, with a 4% v/v aqueous solution of sodium bicarbonate and with water, after which it was dried over anhydrous sodium sulphate. The solvent was then removed by evaporation to dryness under reduced pressure, and the resulting residue was purified by column chromatography through silica gel (ODS treated), eluted with 65% v/v aqueous acetonitrile, to give 0.310 g of the title compound.

Mass Spectrum m/e: 780 ($M^+$, $C_{47}H_{60}N_2O_8$).

Nuclear Magnetic Resonance Spectrum ($CDC\ell_3$) $\delta$ ppm:

1.87 (3H, singlet);
3.21 (1H, doublet, J = 9.5 Hz);
3.96 (1H, doublet, J = 6.2 Hz);
7.83 (2H, broad singlet).

EXAMPLE 133

13-{2-[4-(3-Methoxycarbonylquanidino)phenyl]ethoxy -milbemycin $A_4$

0.041 g of N-methoxycarbonyl-S-methyisothiourea and two drops of acetic acid were added to a solution of 0.204 g of 13-[2-(4-aminophenyl)ethoxy]milbemycin $A_4$ (prepared by a similar procedure to that described in Example 35) in 2.0 ml of methanol, and then the mixture was stirred at room temperature for 1.5 hours. At the end of this time, the solvent was removed by evaporation to dryness under reduced pressure, and the resulting residue was purified by column chromatography through silica gel (ODS treated), eluted with 55% v/v aqueous acetonitrile, to give 0.112 g of the title compound.

Mass Spectrum m/e: 751 ($M^+$ - 16).

Nuclear Magnetic Resonance Spectrum ($CDC\ell_3$) $\delta$ ppm:

1.87 (3H, singlet);
3.21 (1H, doublet, J = 9.9 Hz);
3.71 (3H, singlet);
3.96 (1H, doublet, J = 6.2 Hz).

Following a similar procedure to that described in Example 133, the compound of Example 134 was obtained.

EXAMPLE 134

13-{2-[p-2,3-Bis(methoxycarbonyl)quanidinophenyl]ethoxy -milbemycin $A_4$

Nuclear Magnetic Resonance Spectrum ($CDC\ell_3$) $\delta$ ppm:

1.87 (3H, singlet);
3.21 (1H, doublet, J = 9.8 Hz);
3.73 & 3.86 (3H x 2, singlet);
3.96 (1H, doublet, J = 5.9 Hz);
10.17 & 11.88 (1H x 2, singlet).

EXAMPLE 135

13-[2-(4-Benzenesulphinylaminophenyl)ethoxy]milbemycin $A_4$

0.049 ml of pyridine and 0.107 g of benzenesulphinyl chloride were added to a solution of 0.340 g of 13-[2-(4-aminophenyl)ethoxy]milbemycin $A_4$ (prepared by a similar procedure to that described in Example 35) in 3.5 ml of 1,2-dichloroethane, and then the mixture was stirred at room temperature for 30 minutes. At the end of this time, the reaction mixture was diluted with ethyl acetate and washed, in turn, with 0.5N

aqueous hydrochloric acid, with water, with a 4% w/v aqueous solution of sodium bicarbonate and with water, after which it was dried over anhydrous sodium sulphate. The solvent was then removed by evaporation to dryness under reduced pressure, and the resulting residue was purified by column chromatography through silica gel, eluted with a 55 : 45 by volume mixture of cyclohexane and ethyl acetate, to give 0.105 g of the title compound.
Mass Spectrum m/e: 677 ($M^+$ - 124).
Nuclear Magnetic Resonance Spectrum ($CDCl_3$) $\delta$ ppm:
1.87 (3H, singlet);
3.21 (1H, doublet, J = 9.9 Hz);
3.96 (1H, doublet, J = 6.3 Hz);
5.96 (1H, singlet);
7.0 - 7.9 (9H, multiplet).

EXAMPLE 136

13-Deoxy-13-[2-(4-aminophenyl)ethoxy]-22,23-dihydroavermectin $B_1$a aglycone

1.460 g of 13-deoxy-13-[2-(4-nitrophenyl)ethoxy]-22,23-dihydroavermectin $B_1$a aglycone (which had been prepared by treating 22,23-dihydroavermectin $B_1$a aglycone by similar procedures to those described in Preparation 1 and Example 3) was dissolved in 13.0 ml of 90% v/v aqueous acetic acid. 1.30 g of zinc powder was added to the resulting solution, and then the mixture was stirred for 20 minutes whilst cooling with water. The reaction mixture was then worked up by a similar procedure to that described in Example 35 to give 1.143 g of the title compound.
Mass Spectrum m/e: 705 ($M^+$, $C_{42}H_{59}NO_8$).
Nuclear Magnetic Resonance Spectrum ($CDCl_3$) $\delta$ ppm:
1.87 (3H, singlet);
3.21 (1H, doublet, J = 9.8 Hz);
3.53 (2H, broad singlet);
3.96 (1H, doublet, J = 6.3 Hz).

EXAMPLE 137

13-[2-(4-Aminophenyl)ethoxy]milbemycin D

1.630 g of 13-[2-(4-nitrophenyl)ethoxy]milbemycin D (which had been prepared by treating milbemycin D by similar procedures to those described in Preparation 1 and Example 3) was dissolved in 15.0 ml of 90% v/v aqueous acetic acid. 1.50 g of zinc powder was added to the resulting solution, and then the mixture was stirred for 20 minutes, whilst cooling with water. The reaction mixture was then worked up using similar procedures to those described in Example 35, to give 1.130 g of the title compound.
Mass Spectrum m/e: 691 ($M^+$, $C_{41}H_{57}NO_8$).
Nuclear Magnetic Resonance Spectrum ($CDCl_3$) $\delta$ ppm:
1.87 (3H, singlet);
3.21 (1H, doublet, J = 9.8 Hz);
3.56 (2H, broad singlet);
3.96 (1H, doublet, J = 5.9 Hz);

EXAMPLE 138

13-Deoxy-13-{2-[4-(3-methylureido)phenyl]ethoxy -22,23-dihydroavermectin $B_1$a aglycone

0.212 g of 13-deoxy-13-[2-(4-aminophenyl)ethoxy]-22,23-dihydroavermectin $B_1$a aglycone (prepared as described in Example 136) was dissolved in 2.0 ml of 1,2-dichloroethane. 2 drops of methyl isocyanate were added, and then the mixture was stirred at room temperature for 2 hours. At the end of this time, the solvent was removed by evaporation to dryness under reduced pressure, and the resulting residue was purified by column chromatography through silica gel (ODS treated), eluted with 80% v/v aqueous acetonitrile, to give 0.218 g of the title compound.
Mass Spectrum m/e: 705 ($M^+$ - 57).
Nuclear Magnetic Resonance Spectrum ($CDCl_3$) $\delta$ ppm:

1.87 (3H, singlet);
2.82 (3H, doublet, J = 3.7 Hz);
3.21 (1H, doublet, J = 9.5 Hz);
3.96 (1H, doublet, J = 5.9 Hz);
6.28 (1H, singlet).

EXAMPLE 139

13-{2-[4-(3-Methylureido)phenyl]ethoxy milbemycin D

0.208 g of 13-[2-(4-aminophenyl)ethoxy]milbemycin D (prepared as described in Example 137) was treated by a similar procedure to that that described in Example 138 to give 0.216 g of the title compound.
Mass Spectrum m/e: 691 ($M^+$ - 57).
Nuclear Magnetic Resonance Spectrum ($CDCl_3$) δ ppm:
1.87 (3H, singlet);
2.82 (3H, doublet, J = 4.9 Hz);
3.21 (1H, doublet, J = 9.8 Hz);
3.96 (1H, doublet, J = 5.9 Hz);
6.27 (1H, singlet).

EXAMPLE 140

13-Deoxy-13-[2-(4-methanesulphonylaminophenyl)ethoxy]-22,23-dihydroavermectin $B_1$a aglycone

0.212 g of 13-deoxy-13-[2-(4-aminophenyl)ethoxy]-22,23-dihydroavermectin $B_1$a aglycone (prepared as described in Example 136) was dissolved in 2.0 ml of 1,2-dichloroethane. 0.032 ml of pyridine and 0.046 g of methanesulphonyl chloride were added to the resulting solution, and then the mixture was stirred at room temperature for 2.5 hours. At the end of this time, the reaction mixture was diluted with 15 ml of ethyl acetate and washed, in turn, with 0.1N aqueous hydrochloric acid, with water, with a 4% v/v aqueous solution of sodium bicarbonate and again with water, after which it was dried over anhydrous sodium sulphate. The solvent was then removed by evaporation to dryness under reduced pressure, and the resulting residue was purified by column chromatography through silica gel (ODS treated), eluted with 80% v/v aqueous acetonitrile, to give 0.221 g of the title compound.
Mass Spectrum m/e: 783 ($M^+$, $C_{43}H_{61}NO_{10}S$).
Nuclear Magnetic Resonance Spectrum ($CDCl_3$) δ ppm:
1.87 (3H, singlet);
2.93 (3H, singlet);
3.20 (1H, doublet, J = 9.5 Hz);
3.96 (1H, doublet, J = 6.3 Hz);
6.37 (1H, singlet).

EXAMPLE 141

13-[2-(4-Methanesulphonylaminophenyl)ethoxy]milbemycin D

0.208 g of 13-[2-(4-aminophenyl)ethoxy]milbemycin D (prepared as described in Example 137) was treated by a similar procedure to that described in Example 140 to give 0.219 g of the title compound.
Mass Spectrum m/e: 769 ($M^+$, $C_{42}H_{59}NO_{10}S$).
Nuclear Magnetic Resonance Spectrum ($CDCl_3$) δ ppm:
1.87 (3H, singlet);
2.97 (3H, singlet);
3.21 (1H, doublet, J = 9.8 Hz);
3.96 (1H, doublet, J = 5.9 Hz);
6.39 (1H, singlet).

EXAMPLE 142

13-Deoxy-13-[2-(4-ethoxycarbonylaminophenyl)ethoxy]-22,23-dihydroavermectin $B_1$a aglycone

0.212 g of 13-deoxy-13-[2-(4-aminophenyl)ethoxy]-22,23-dihydroavermectin $B_1$a aglycone (prepared as described in Example 136) was dissolved in 2.0 ml of 1,2-dichloroethane. 0.032 ml of pyridine and 0.060 g of ethyl chlorocarbonate were added to the resulting solution, whilst ice-cooling, and then the mixture was stirred for 30 minutes. At the end of this time, the reaction mixture was diluted with 15 ml of ethyl acetate and washed, in turn, with 0.5N aqueous hydrochloric acid, with water, with a 4% w/v aqueous solution of sodium bicarbonate and again with water, after which it was dried over anhydrous sodium sulphate. The solvent was then removed by evaporation to dryness under reduced pressure, and the resulting residue was purified by column chromatography through silica gel (ODS treated), eluted with 80% v/v aqueous acetonitrile, to give 0.168 g of the title compound.
Mass Spectrum m/e: 778 ($M^+$ + 1).
Nuclear Magnetic Resonance Spectrum ($CDCl_3$) δ ppm:
1.87 (3H, singlet);
3.21 (1H, doublet, J = 9.5 Hz);
3.96 (1H, doublet, J = 5.9 Hz);
4.22 (2H, quartet, J = 7.3 Hz).

EXAMPLE 143

13-[2-(4-Ethoxycarbonylaminophenyl)ethoxy]milbemycin D

0.208 g of 13-[2-(4-aminophenyl)ethoxy]milbemycin D (prepared as described in Example 137) was treated by a similar procedure to that described in Example 142 to give 0.167 g of the title compound.
Mass Spectrum m/e: 745 ($M^+$ - 18).
Nuclear Magnetic Resonance Spectrum ($CDCl_3$) δ ppm:
1.87 (3H, singlet);
3.21 (1H, doublet, J = 9.8 Hz);
3.96 (1H, doublet, J = 6.3 Hz);
4.21 (2H, quartet, J = 7.0 Hz);
6.52 (1H, singlet).

EXAMPLE 144

13-[2-(N-Ethoxycarbonyl-N-methyl-4-aminophenyl)ethoxy]milbemycin $A_4$

0.682 g of N-ethoxycarbonyl-N-methyl-4-aminophenethyl alcohol and 0.300 g of mercuric iodide were added to a solution of 0.325 g of 13-iodo-5-oxo-milbemycin $A_4$ in 5 ml of 1,2-dichloroethane, and then the mixture was stirred at room temperature for 2.5 hours. At the end of this time, the reaction mixture was worked up by a similar procedure to that described in Example 3 to give 0.138 g of the title compound.
Mass Spectrum m/e: 763 ($M^+$, $C_{44}H_{61}NO_{10}$).
Nuclear Magnetic Resonance Spectrum ($CDCl_3$) δ ppm:
1.87 (3H, singlet);
3.21 (1H, doublet, J = 9.9 Hz);
3.27 (3H, singlet);
3.95 (1H, doublet, J = 6.2 Hz);
4.15 (2H, quartet, J = 7.0 Hz).

EXAMPLE 145

13-{2-[4-(3-Methylureidomethyl)phenyl]ethoxy milbemycin $A_4$

Step 1

4.900 g of 4-(2,2,2-trichloroethoxycarbonylaminomethyl)phenethyl alcohol and 2.060 g of mercuric iodide were added to a solution of 2.000 g of 13-iodo-5-oxo-milbemycin $A_4$ in 10 ml of 1,2-dichloroethane,

and then the mixture was stirred at room temperature for 2.5 hours. At the end of this time, the reaction mixture was worked up by a similar procedure to that described in Example 3 to give 1.610 g of 13-{2-[4-(2,2,2-trichloroethoxycarobonylaminomethyl)phenyl]ethoxy}milbemycin $A_4$.

Step 2

All of the product obtained in Step 1 was dissolved in 15.0 ml of 90% v/v aqueous acetic acid, 2.0 g of zinc powder were added to the resulting solution, and then the mixture was stirred for 60 minutes whilst cooling with water. The mixture was then diluted with 50 ml of ethyl acetate and the insoluble matter was filtered off. The filtrate was washed with a saturated aqueous solution of sodium chloride and dried over anhydrous sodium sulphate. The solvent was then removed by evaporation under reduced pressure, and the resulting residue was purified by column chromatography through silica gel (ODS treated), eluted with 65% v/v aqueous acetonitrile. The eluate was concentrated by evaporation under reduced pressure, and the product was dissolved in 20 ml of ethyl acetate. The resulting solution was then washed with a 4% v/v aqueous solution of sodium bicarbonate and with water, in that order, after which the solvent was removed by evaporation under reduced pressure to give 0.738 g of 13-{2-[4-(aminomethyl)phenyl]ethoxy milbemycin $A_4$.

Step 3

0.138 g of the product obtained in Step 2 was dissolved in 1.5 ml of 1,2-dichloroethane. 2 drops of methyl isocyanate were added, and then the mixture was stirred at room temperature for 30 minutes. The solvent was then removed by evaporation to dryness under reduced pressure, and the resulting residue was purified by column chromatography through silica gel (ODS treated), eluted with 80% v/v aqueous acetonitrile, to give 0.143 g of the title compound.

Mass spectrum m/e: 731 ($M^+$ - 17).

Nuclear Magnetic Resonance Spectrum ($CDC\ell_3$) $\delta$ ppm:

1.87 (3H, singlet);
2.77 (3H, doublet, J = 4.8 Hz);
3.21 (1H, doublet, J = 9.9 Hz);
3.95 (1H, doublet, J = 6.2 Hz);
4.33 (2H, doublet, J = 4.9 Hz);
4.68 (1H, doublet, J = 4.9 Hz).

EXAMPLE 146

13-{2-[4-(Ethoxycarbonylaminomethyl)phenyl]ethoxy -milbemycin $A_4$

0.138 g of 13-{2-[4-(aminomethyl)phenyl]ethoxy - milbemycin $A_4$ (obtained as described in Step 2 of Example 145) was treated by a similar procedure to that described in Example 142 to give 0.082 g of the title compound.

Mass Spectrum m/e: 763 ($M^+$, $C_{44}H_{61}NO_{10}$).

Nuclear Magnetic Resonance Spectrum ($CDC\ell_3$) $\delta$ ppm:

1.87 (3H, singlet);
3.21 (1H, doublet, J = 9.9 Hz);
3.95 (1H, doublet, J = 6.2 Hz);
4.15 (2H, quartet, J = 7.0 Hz);
4.33 (2H, doublet, J = 5.9 Hz);
4.89 (1H, broad singlet).

EXAMPLE 147

13-{2-[4-(Methanesulphonylaminomethyl)phenyl]ethoxy -milbemycin $A_4$

0.138 g of 13-{2-[4-(aminomethyl)phenyl]ethoxy - milbemycin $A_4$ (obtained as described in Step 2 of Example 145) was treated by a similar procedure to that described in Example 140 to give 0.105 g of the title compound.

Mass Spectrum m/e: 769 ($M^+$, $C_{42}H_{59}NO_{10}S$).

Nuclear Magnetic Resonance Spectrum ($CDCl_3$) δ ppm:
1.87 (3H, singlet);
2.87 (3H, singlet);
3.21 (1H, doublet, J = 9.9 Hz);
3.95 (1H, doublet, J = 6.2 Hz);
4.29 (2H, doublet, J = 5.9 Hz);
4.54 (1H, triplet, J = 5.9 Hz).

EXAMPLE 148

13-{2-[4-(N-p-Methylphenylcarbamoyl)phenyl]ethoxy -milbemycin $A_4$

0.510 g of 4-[N-(4-methylphenyl)carbamoyl]phenethyl alcohol, 0.300 g of mercuric iodide and 0.120 ml of 2,6-lutidine were added to a solution of 0.335 g of 13-iodo-5-oxomilbemycin $A_4$ in 5.0 ml of 1,2-dichloroethane, and then the mixture was stirred at 30 °C for 3.5 hours. At the end of this time, the reaction mixture was worked up by a similar procedure to that described in Example 3 to give 0.115 g of the title compound.
Mass Spectrum m/e: 777 ($M^+$ - 18).
Nuclear Magnetic Resonance Spectrum ($CDCl_3$) δ ppm:
1.87 (3H, singlet);
2.34 (3H, singlet);
3.21 (1H, doublet, J = 9.5 Hz);
3.95 (1H, doublet, J = 6.2 Hz);
7.72 (1H, singlet).

EXAMPLE 149

13-[2-(4-Methanesulphonylaminophenyl)ethoxy]milbemycin $A_4$5-oxime

Step 1

0.714 g of 13-[2-(4-methanesulphonylaminophenyl)ethoxy]milbemycin $A_4$ (prepared as described in Example 46) was dissolved in 5.0 ml of methylene chloride. 1.90 g of manganese dioxide was added to the resulting solution, whilst ice-cooling, and then the mixture was stirred for 3.5 hours. At the end of this time, the reaction mixture was filtered using a Cellite (trade name) filter aid, and then the filtrate was concentrated under reduction pressure to dryness, to give 0.672 g of 5-oxo-13-[2-(4-methanesulphonylaminophenyl)-ethoxy]milbemycin $A_4$.

Step 2

0.226 g of the product obtained in Step 1 was dissolved in 2.4 ml of methanol. 1.2 ml of water, 2.4 ml of dioxane and 0.220 g of hydroxylamine hydrochloride were added to the resulting solution, and then the mixture was stirred at 40 °C for 2.5 hours. The reaction mixture was then diluted with 20 ml of ethyl acetate, washed twice with water and dried over anhydrous sodium sulphate. The solvent was then removed by evaporation under reduced pressure, and the resulting residue was purified by column chromatography through silica gel (ODS treated), eluted with 80% v/v aqueous acetonitrile, to give 0.196 g of the title compound.
Mass spectrum m/e: 735 ($M^+$ - 33).
Nuclear Magnetic Resonance Spectrum ($CDCl_3$) δ ppm:
1.93 (3H, singlet);
2.97 (3H, singlet);
3.21 (1H, doublet, J = 9.9 Hz);
4.66 (1H, singlet);
6.35 (1H, singlet);
8.05 (1H, singlet).

PREPARATION 1

5-Oxo-13-iodomilbemycin $A_4$(III)

6.00 g of 2-chloroformyl-1,2,4-triazolo[4.3a]pyridin-3-one and 2.42 ml of pyridine were added, whilst ice-cooling, to a solution of 16.60 g of 5-oxo-13-hydroxymilbemycin $A_4$ in 75 ml of methylene chloride, and the mixture was stirred for 30 minutes. At the end of this time, the reaction mixture was filtered and the filtrate was washed with a 0.5M aqueous solution of citric acid, with water, with a 4% v/v aqueous solution of sodium bicarbonate and again with water, in that order. The solution was then dried over anhydrous sodium sulphate, after which the solvent was removed by distillation to dryness under reduced pressure. The residue was dissolved in 300 ml of 1,2-dichloroethane, and 51.4 g of zinc iodide were added to the solution. The mixture was then stirred at room temperature for 25 minutes. At the end of this time, the excess of zinc iodide was removed by filtration and the filtrate was washed with a 10% v/v aqueous solution of sodium thiosulphate and with water, in that order. The solution was then dried over anhydrous sodium sulphate and the solvent was removed by distillation to dryness under reduced pressure. The residue was purified by column chromatography through 300 g of silica gel, eluted with a 9 : 1 by volume mixture of methylene chloride and hexane, and the eluate was crystallized with a mixture of diethyl ether and hexane to afford 15.3 g of the title compound.

Nuclear Magnetic Resonance Spectrum ($CDC\ell_3$) $\delta$ ppm:

3.85 (1H, singlet);
3.99 (1H, singlet);
4.58 (1H, doublet, J = 11.0 Hz).

BIOLOGICAL ACTIVITY

The anthelmintic activity against Nippostongylus brasiliensis, a nematode parasitic to rats, was examined with groups each containing 3 Wistar strain rats of body weight in the range from 40 to 60 g.

The rats were infested percutaneously with about 100 larvae of the nematode for each rat. Solutions containing the test compound at various concentrations were administered orally 3 days after infection. Each solution was prepared by dissolving 1.0 mg of the test compound in 0.1 ml of dimethylformamide, and then adding 10 ml of polyethylene glycol (PEG 400) to the solution. The solution was then adjusted by the addition of PEG 400 to achieve a concentration of 0.250 or 0.125 mg/kg.

The rats were killed 4 days after infection, and the number of parasites in the small intestine was counted. The results obtained are summarized in Table 1.

In the Table, the mortality rate was calculated by the following formula:-

$$\text{Mortality rate (\%)} = \frac{\text{Number of parasites in untreated group} - \text{Number of parasites in treated group}}{\text{Number of parasites in untreated group}}$$

Table 1 Activity on Oral Administration

| Cpd. of Example No. | Mortality rate (%) 0.250 mg/kg | 0.125 mg/kg |
|---|---|---|
| 1 | 73.5 | 44.3 |
| 7 | 98.4 | 81.6 |
| 9 | 98.6 | 98.1 |
| 35 | 98.5 | 92.3 |
| 38 | 99.8 | 99.8 |
| 39 | 96.7 | 68.1 |
| 42 | 99.6 | 99.6 |
| 46 | 99.7 | 100 |
| 47 | 100 | 50.7 |
| 48 | 99.5 | 100 |
| 49 | 100 | 98.6 |
| 50 | 99.5 | 98.2 |
| 57 | 99.8 | 99.4 |
| 61 | - | 99.1 |
| 64 | - | 92.8 |
| 71 | - | 100 |
| 81 | - | 99.7 |
| 84 | - | 100 |
| 86 | 99.5 | 94.4 |
| 87 | - | 100 |
| 88 | - | 97.4 |

...Cont'd

| | | |
|---|---|---|
| 109 | - | 98.9 |
| 111 | - | 93.5 |
| 114 | - | 100 |
| 117 | - | 97.9 |
| 126 | - | 95.9 |
| 129 | - | 100 |
| 131 | - | 97.6 |
| 132 | 89.5 | 77.0 |
| 134 | 98.6 | 92.7 |
| 140 | - | 93.2 |
| 141 | 99.8 | 98.7 |
| 145 | - | 99.2 |
| 13-Methoxymilbemycin $A_4$* | 44.0 | 49.5 |
| Milbemycin $A_4$ | 24.8 | - |

*Compound disclosed in US Patent No. 4 696 945.

In the above Table, a dash means that the compound was not tested at the particular concentration.

**Claims**

**Claims for the following Contracting States : GB, DE, FR, IT, CH, BE, NL, SE, LU, AT**

**1.** Compounds of formula (I):

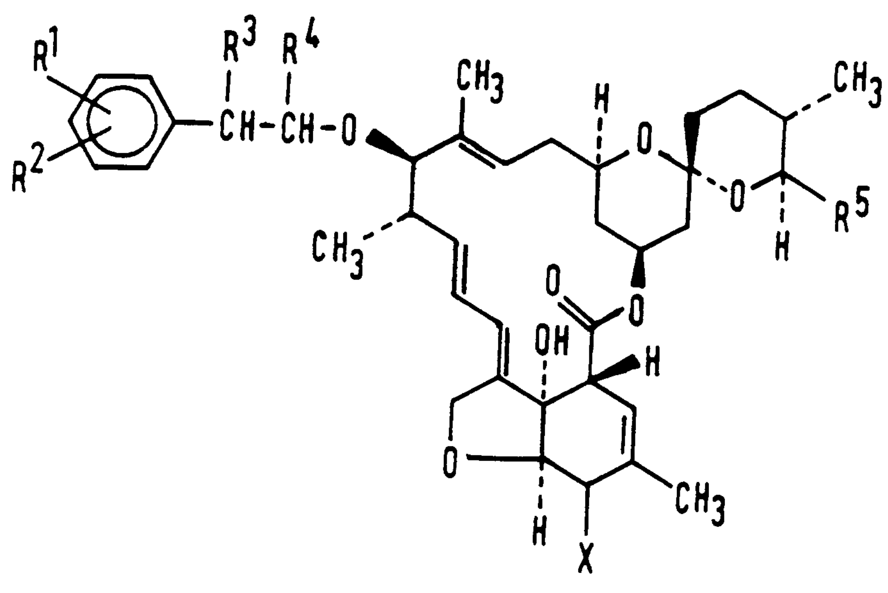

(I)

in which:

$R^1$ and $R^2$ are the same or different and each represents: a hydrogen atom; a halogen atom; a cyano group; a nitro group; a $C_1$ - $C_4$ alkyl group; a substituted $C_1$ - $C_4$ alkyl group having at least one of substituents (a), defined below; a $C_1$ - $C_4$ alkoxy group; a $C_2$ - $C_6$ alkoxyalkoxy group; a group of formula $-(CH_2)_nNHR^9$,

in which: n represents 0 or the integer 1 or 2, and $R^9$ represents a hydrogen atom or a $C_1$ - $C_4$ alkyl group;

a group of formula $-(CH_2)_nNR^9C(=O)R^6$,

in which:

n and $R^9$ are as defined above, and

$R^6$ represents: a hydrogen atom; a $C_1$ - $C_4$ alkyl group; a substituted $C_1$ - $C_4$ alkyl group having at least one of substituents (b), defined below; a $C_2$ - $C_8$ aliphatic hydrocarbon group having one or two ethylenically unsaturated carbon-carbon double bonds, said group being unsubstituted or having at least one of substituents (b), defined below; a $C_2$ - $C_8$ alkynyl group; a substituted $C_2$ - $C_8$ alkynyl group having at least one of substituents (b), defined below; a $C_3$ - $C_8$ cycloalkyl group; a substituted $C_3$ - $C_8$ cycloalkyl group having at least one of substituents (c), defined below; a carbocyclic aryl group having from 6 to 14 ring carbon atoms and being unsubstituted or having at least one of substituents (c), defined below; or a heterocyclic group having from 3 to 6 ring atoms of which at least one is a nitrogen and/or oxygen and/or sulphur hetero-atom, said heterocyclic group being monocyclic or being fused to one or two benzene rings and being unsubstituted or having at least one of substituents (c), defined below;

a group of formula $-(CH_2)_nNR^9COCOR^6$

in which n, $R^6$ and $R^9$ are as defined above;

a group of formula $-(CH_2)_nR^9COCOOR^7$

in which n and $R^9$ are as defined above and $R^7$ represents a $C_1$ - $C_4$ alkyl group, a $C_3$ - $C_8$ cycloalkyl group or an aralkyl group as defined below;

a group of formula $-(CH_2)_nNR^9CHR^6NHCOR^6$

in which n, $R^6$ and $R^9$ are as defined above;

a group of formula $-(CH_2)_nNR^9CHR^6NHCONHR^6$

in which n, $R^6$ and $R^9$ are as defined above;

a group of formula $-(CH_2)_nNR^9CHR^6NHCOOR^7$

in which n, $R^6$, $R^7$ and $R^9$ are as defined above;

a group of formula $-(CH_2)_nNR^9C(=Y)YR^6$

in which n, $R^6$ and $R^9$ are as defined above and the two symbols Y are the same or different and each represents an oxygen atom or a sulphur atom;

groups of formula $-(CH_2)_nNR^9C(=Y)NR^{6'}R^{6'}$

in which n, Y and $R^9$ are as defined above, and the two symbols $R^{6'}$ are the same or different and each represents one of the groups or atoms represented by $R^6$, or the two, together with the nitrogen atom to which they are attached, form a heterocyclic group having from 3 to 7 ring atoms of which one is said nitrogen atom and 0 or 1 is an additional nitrogen and/or oxygen and/or sulphur hetero-atom;

a group of formula $-(CH_2)_nNR^9C(=Y)NR^{6''}NR^{6''}R^{6''}$

in which n, Y and $R^9$ are as defined above, and each of the symbols $R^{6''}$ is the same or different and each represents one of the groups or atoms represented by $R^6$, or any two of the symbols $R^{6''}$, together with the nitrogen atom to which each is attached, forms a heterocyclic group having from 3 to 7 ring atoms of which one or two is said nitrogen atom or atoms and 0 or 1 is an additional nitrogen and/or oxygen and/or sulphur hetero-atom;

a group of formula $-(CH_2)_nNR^9C(=Y)NR^6NHZ$

in which n, Y, $R^6$ and $R^9$ are as defined above and Z represents

a group of formula $-COOR^7$, in which $R^7$ is as defined above,

a group of formula $-COR^6$, in which $R^6$ is as defined above, or

a group of formula $-SO_2R^6$, in which $R^6$ is as defined above;

a group of formula $-(CH_2)_nNR^9C(=NR^{10})NHR^{10}$

in which n and $R^9$ are as defined above and the two symbols $R^{10}$ are the same or different and each represents one of the groups or atoms represented by $R^6$, or a cyano group, a nitro group, a group of formfula $-COOR^7$, in which $R^7$ is as defined above, or a group of formula $-COR^6$, in which $R^6$ is as defined above;

a group of formula $-(CH_2)_nNR^9C(=NR^{10})R^6$

in which n, $R^6$, $R^9$ and $R^{10}$ are as defined above;

a group of formula $-(CH_2)_nNR^9SO_mR^6$

in which n, $R^6$ and $R^9$ are as defined above and m is 1 or 2;
a group of formula $-CONHR^6$
in which $R^6$ is as defined above; and
a group of formula $-COOR^7$
in which $R^7$ is as defined above;
$R^3$ and $R^4$ are the same or different and each represents a hydrogen atom, a $C_1$ - $C_4$ alkyl group or a $C_1$ - $C_4$ alkoxy group;
$R^5$ represents a methyl group, an ethyl group, an isopropyl group or a sec-butyl group; and
X represents a hydroxy group, a $C_1$ - $C_5$ alkanoyloxy group, a substituted $C_1$ - $C_5$ alkanoyloxy group having at least one of substituents (d), defined below, or a hydroxyimino group;
said aralkyl groups have from 1 to 4 carbon atoms in the alkyl part and from 6 to 10 ring atoms in the aryl part, which is a carbocyclic aryl group which is unsubstituted or has at least one of substituents (c), defined below;
substituents (a):
halogen atoms, $C_1$ - $C_4$ alkoxy groups, $C_1$ - $C_4$ alkylthio groups and $C_1$ - $C_5$ alkanoyloxy groups;
substituents (b):
$C_3$ - $c_8$ cycloalkyl groups; $C_1$ - $C_4$ alkoxy groups; $C_1$ - $C_4$ alkylthio groups; $C_2$ - $C_5$ cyanoalkylthio groups; $C_2$ - $C_5$ alkoxycarbonyl groups; halogen atoms; cyano groups; nitro groups; amino groups; carbocyclic aryl groups having from 6 to 10 carbon atoms and being unsubstituted or having at least one of substituents (c), defined below; aromatic heterocyclic groups having from 5 to 8 ring atoms of which from 1 to 4 are nitrogen and/or oxygen and/or sulphur hetero-atoms, said heterocyclic group being monocyclic or being fused either to a benzene ring or to a heterocyclic group which has 5 or 6 ring atoms of which from 1 to 3 are nitrogen hetero-atoms and being unsubstituted or having at least one of substituents (c), defined below; and aryloxy and arylthio groups in which the aryl part has from 6 to 10 carbon atoms and is unsubstituted or has at least one of substituents (c), defined below;
substituents (c):
$C_1$ - $C_4$ alkyl groups, $C_1$ - $C_4$ alkoxy groups, $C_1$ - $C_4$ alkylthio groups, $C_1$ - $C_5$ alkanoyloxy groups, $C_2$ - $C_5$ alkoxycarbonyl groups, halogen atoms, cyano groups, nitro groups, amino groups, mono- and di-alkylamino groups in which the or each alkyl part is $C_1$ - $C_4$, carbamoyl groups, mono- and di-alkylcarbamoyl groups in which the or each alkyl part is $C_1$ - $C_4$, and $C_1$ - $C_5$ alkanoylamino groups;
substituents (d):
halogen atoms, $C_1$ - $C_4$ alkoxy groups, $C_2$ - $C_5$ alkoxycarbonyl groups and carboxy groups;
and salts thereof.

**2.** A compound according to Claim 1, in which:
$R^1$ represents a hydrogen atom; and
$R^2$ represents a hydrogen atom, a $C_1$ - $C_3$ alkyl group, a $C_1$ - $C_3$ alkoxy group, a fluorine or chlorine atom, a nitro group or an amino group.

**3.** A compound according to Claim 1, in which:
$R^1$ represents a hydrogen atom; and
$R^2$ represents a group of formula $-(CH_2)_nNR^{9a}COR^{6a}$
in which n is 0, $R^{9a}$ represents a hydrogen atom or a methyl group, and $R^{6a}$ represents: a $C_1$ - $C_4$ alkyl group; a $C_3$ - $C_5$ cycloalkyl group; a $C_1$ - $C_3$ alkyl group substituted with a halogen, cyano, $C_1$ - $C_3$ alkoxy, $C_1$ - $C_3$ alkylthio, cyanomethylthio or phenoxy substituent; an alkenyl group; an unsubstituted phenyl group; a phenyl group substituted with a $C_1$ - $C_3$ alkyl, $C_1$ - $C_3$ alkoxy, halogen or nitro substituent; a pyridyl group; a pyrimidyl group; a pyrazyl group; a furyl group; or a thienyl group.

**4.** A compound according to Claim 1, in which:
$R^1$ represents a hydrogen atom; and
$R^2$ represents a group of formula $-(CH_2)_nNR^{9a}COCOR^{6b}$
in which n is 0, $R^{9a}$ represents a hydrogen atom or a methyl group, and $R^{6b}$ represents a hydrogen atom, a $C_1$ - $C_4$ alkyl group, a $C_3$ - $C_5$ cycloalkyl group, an alkenyl group, an unsubstituted phenyl group, or a phenyl group substituted with a $C_1$ - $C_3$ alkyl, $C_1$ - $C_3$ alkoxy, halogen or nitro substituent.

**5.** A compound according to Claim 1, in which:
$R^1$ represents a hydrogen atom; and
$R^2$ represents a group of formula $-(CH_2)_nNR^{9a}C(=Y)YR^{6c}$

in which n is 0, $R^{9a}$ represents a hydrogen atom or a methyl group, both Y are oxygen atoms and $R^{6c}$ represents a $C_1$ - $C_4$ alkyl group, a $C_1$ - $C_4$ alkyl group substituted with a halogen or $C_1$ - $C_3$ alkoxy substituent, an alkenyl group, a benzyl group, a methoxybenzyl group, a nitrobenzyl group, a furfuryl group, a thenyl group or a phenyl group.

**6.** A compound according to Claim 1, in which:
$R^1$ represents a hydrogen atom; and
$R^2$ represents a group of formula $-(CH_2)_nNR^{9a}C(=Y)NR^{6d}R^{6e}$
in which n is 0, $R^{9a}$ represents a hydrogen atom or a methyl group, Y represents an oxygen atom or a sulphur atom, and $R^{6d}$ and $R^{6e}$ are the same or different and each represents a $C_1$ - $C_4$ alkyl group, a $C_3$ - $C_6$ cycloalkyl group, unsubstituted phenyl group, or a phenyl group substituted with a $C_1$ - $C_3$ alkyl, $C_1$ - $C_3$ alkoxy, halogen or nitro substituent, or $R^{6d}$ and $R^{6e}$, together with the nitrogen atom to which they are attached, represent a piperidino, piperazino, morpholino, pyrrolidino or aziridino group.

**7.** A compound according to Claim 1, in which:
$R^1$ represents a hydrogen atom; and
$R^2$ represents a group of formula $-(CH_2)_nNR^{9a}C(=Y)NR^{6f}NR^{6g}R^{6h}$
in which n is 0, $R^{9a}$ represents a hydrogen atom or a methyl group, Y represents an oxygen atom or a sulphur atom, and $R^{6f}$, $R^{6g}$ and $R^{6h}$ are the same or different and each represents: a hydrogen atom; a $C_1$ - $C_4$ alkyl group, a $C_3$ - $C_6$ cycloalkyl group; an unsubstituted phenyl group, or a phenyl group substituted with a $C_1$ - $C_3$ alkyl, $C_1$ - $C_3$ alkoxy, halogen or nitro substituent; or $R^{6g}$ and $R^{6h}$, together with the nitrogen atom to which they are attached, represent a piperidino, piperazino, morpholino, pyrrolidino or aziridino group; or $R^{6f}$ and $R^{6g}$, together with the nitrogen atoms to which they are attached, represent a pyrazolidinyl or tetrahydropyridazinyl group.

**8.** A compound according to Claim 1, in which:
$R^1$ represents a hydrogen atom; and
$R^2$ represents a group of formula $-(CH_2)_nNR^{9a}C(=Y)NR^{6j}NHZ$
in which n is 0, $R^{9a}$ represents a hydrogen atom or a methyl group, Y represents an oxygen atom or a sulphur atom, $R^{6j}$ represents a hydrogen atom; a $C_1$ - $C_4$ alkyl group or a $C_3$ - $C_6$ cycloalkyl group; and Z represents a group of formula $-COOR^{7a}$
in which $R^{7a}$ represents a $C_1$ - $C_4$ alkyl group, a $C_3$ - $C_6$ cycloalkyl group or a benzyl group,
a group of formula $-COR^{6k}$
in which $R^{6k}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent or
a group of formula $-SO_2R^{6m}$
in which $R^{6m}$ represents a $C_1$ - $C_4$ alkyl group, an unsubstituted phenyl group or a phenyl group substituted with a $C_1$ - $C_3$ alkyl, $C_1$ - $C_3$ alkoxy, halogen or nitro substituent.

**9.** A compound according to Claim 1, in which:
$R^1$ represents a hydrogen atom; and $R^2$ represents a group of formula
$-(CH_2)_nNR^{9a}C(=NR^{10a})NHR^{10b}$
in which n is 0, $R^{9a}$ represents a hydrogen atom or a methyl group, and $R^{10a}$ and $R^{10b}$ are the same or different and each represents: a hydrogen atom; a $C_1$ - $C_4$ alkyl group; an unsubstituted phenyl group; a phenyl group substituted with a $C_1$ - $C_3$ alkyl, $C_1$ - $C_3$ alkoxy, halogen or nitro substituent; a group of formula $-COOR^{7b}$
in which $R^{7b}$ represents a $C_1$ - $C_4$ alkyl group, a $C_3$ - $C_6$ cycloalkyl group or a benzyl group, or
a group of formula $-COR^{6n}$
in which $R^{6n}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent.

**10.** A compound according to Claim 1, in which:
$R^1$ represents a hydrogen atom; and
$R^2$ represents a group of formula $-(CH_2)_nNR^{9a}C(=NR^{10c})R^{6p}$
in which n is 0; $R^{9a}$ represents a hydrogen atom or a methyl group; and $R^{10c}$ represents a hydrogen atom; a $C_1$ - $C_4$ alkyl group, an unsubstituted phenyl group, a phenyl group substituted with a $C_1$ - $C_3$ alkyl, $C_1$ - $C_3$ alkoxy, halogen or nitro substituent, a group of formula $-COOR^{7c}$

in which $R^{7c}$ represents a $C_1$ - $C_4$ alkyl group, a $C_3$ - $C_6$ cycloalkyl group or a benzyl group;
or a group of formula $-COR^{6q}$
in which $R^{6q}$ represents a $C_1$ - $C_4$ alkyl group, a $C_3$ - $C_6$ cycloalkyl group, an unsubstituted phenyl group; or a phenyl group substituted with a $C_1$ - $C_3$ alkyl, $C_1$ - $C_3$ alkoxy, halogen or nitro substituent and
$R^{6p}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent.

**11.** A compound according to Claim 1, in which:
$R^1$ represents a hydrogen atom; and
$R^2$ represents a group of formula $-(CH_2)_nNR^{9a}SO_mR^{6r}$
in which n is 0, $R^{9a}$ represents a hydrogen atom or a methyl group, m is 1 or 2, and $R^{6r}$ represents a $C_1$ - $C_4$ alkyl group, a $C_2$ - $C_4$ cyanoalkyl group, an unsubstituted phenyl group; a phenyl group substituted with a $C_1$ - $C_3$ alkyl, $C_1$ - $C_3$ alkoxy, halogen or nitro substituent.

**12.** A compound according to Claim 1, in which:
$R^1$ represents a hydrogen atom, and
$R^2$ represents a group of formula $-CONHR^{6s}$
in which $R^{6s}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent.

**13.** A compound according to Claim 1, in which:
$R^1$ represents a hydrogen atom, and
$R^2$ represents a group of formula $-COOR^{7d}$
in which $R^{7d}$ represents a methyl, ethyl or benzyl group.

**14.** A compound according to Claim 1, in which:
$R^1$ represents a methoxy group, and
$R^2$ represents a $C_1$ - $C_3$ alkoxy group or a $C_2$ - $C_4$ alkoxyalkoxy group.

**15.** A compound according to any one of the preceding Claims, in which $R^2$ is attached at the p-position of the benzene ring.

**16.** A compound according to any one of the preceding Claims, in which $R^5$ represents an ethyl group.

**17.** A composition comprising a mixture of compounds, each of which is as defined in any one of Claims 1 to 15, wherein, in one of said compounds, $R^5$ represents an ethyl group and, in the other of said compounds, $R^5$ represents a methyl group.

**18.** A compound according to any one of the preceding Claims, in which $R^3$ and $R^4$ are hydrogen atoms.

**19.** A compound according to any one of the preceding Claims, in which X represents a hydroxy group.

**20.** 13-[2-(4-Acetamidophenyl)ethoxy]milbemycin $A_4$.

**21.** 13-[2-(4-Cyanoacetamidophenyl)ethoxy]milbemycin $A_4$.

**22.** 13-{2-[4-Cyanopropionamido)phenyl]ethoxy}milbemycin $A_4$.

**23.** 13-[2-(4-Methoxyacetamidophenyl)ethoxy]milbemycin $A_4$.

**24.** 13-{2-[4-(Cyclopropylcarbonylamino)phenyl]ethoxy}milbemycin $A_4$.

**25.** 13-{2-[4-(Cyclobutylcarbonylamino)phenyl]ethoxy}milbemycin $A_4$.

**26.** 13-{2-[4-(4-Cyanobenzamido)phenyl]ethoxy}milbemycin $A_4$.

**27.** 13-[2-(4-Methoxycarbonylaminophenyl)ethoxy]milbemycin $A_4$.

**28.** 13-[2-(4-Vinyloxycarbonylaminophenyl)ethoxy]milbemycin $A_4$.

**29.** 13-{2-[4-(3-Methylureido)phenyl]ethoxy}milbemycin $A_4$.

**30.** 13-{2-[4-(3-Phenylureido)phenyl]ethoxy}milbemycin $A_4$.

**31.** 13-{2-[4-(3-Cyclohexylureido)phenyl]ethoxy}milbemycin $A_4$.

**32.** 13-[2-(4-Methanesulphonylaminophenyl)ethoxy]milbemycin $A_4$.

**33.** 13-[2-(4-Ethanesulphonylaminophenyl)ethoxy]milbemycin $A_4$.

**34.** 13-{2-[4-(3,3-Dimethylcarbazoylamino)phenyl]ethoxy}milbemycin $A_4$ and salts thereof.

**35.** 13-{2-[4-(3-o-Fluorophenylureido)phenyl]ethoxy}milbemycin $A_4$.

**36.** 13-{2-[4-(3-p-Fluorophenylureido)phenyl]ethoxy}milbemycin $A_4$.

**37.** 13-{2-[4-(3-p-Methoxyphenylureido)phenyl]ethoxy}milbemycin $A_4$.

**38.** An anthelmintic, acarcidal and insecticidal composition comprising an anthelmintic, acaricidal and insecticidal compound in admixture with a pharmaceutically, agriculturally, veterinarily or horticulturally acceptable carrier or diluent, in which said compound is at least one compound according to any one of the preceding Claims.

**39.** The use for the manufacture of a medicament for the treatment of infections by helminths, acarids or insects of at least one compound according to any one of Claims 1 to 37 or a composition according to Claim 38.

**40.** A method of protecting plants from damage by acarids, helminths or insects, which comprises applying an active compound to said plants or to seeds of said plants or to a locus including said plants or seeds, in which the active compound is at least one compound according to any one of Claims 1 to 37 or a composition according to Claim 38.

**41.** A process for preparing a compound according to any one of Claims 1 to 37, which process comprises the steps:
reacting a compound of formula (IV):

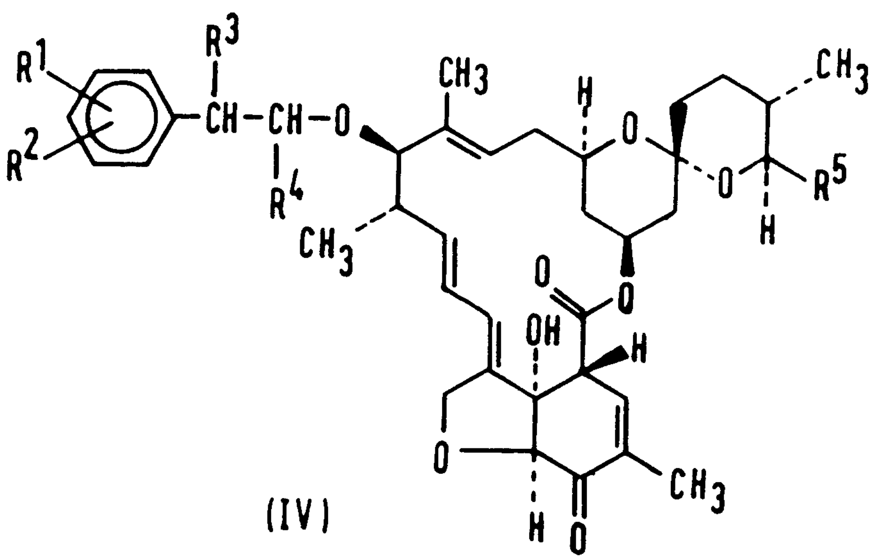

(IV)

(in which $R^1$, $R^2$, $R^3$, $R^4$ and $R^5$ are as defined in Claim 1) with a reducing agent to prepare a compound of formula (I) in which X represents a hydroxy group;
and, if required, acylating said compound in which X represents a hydroxy group to prepare a compound of formula (I) in which X represents said alkanoyloxy group;
or reacting said compound of formula (IV) with hydroxylamine or with a salt thereof to prepare a compound of formula (I) in which X represents a hydroxyimino group.

**42.** A process according to Claim 41, in which said compound of formula (IV) is prepared by reacting a compound of formula (III):

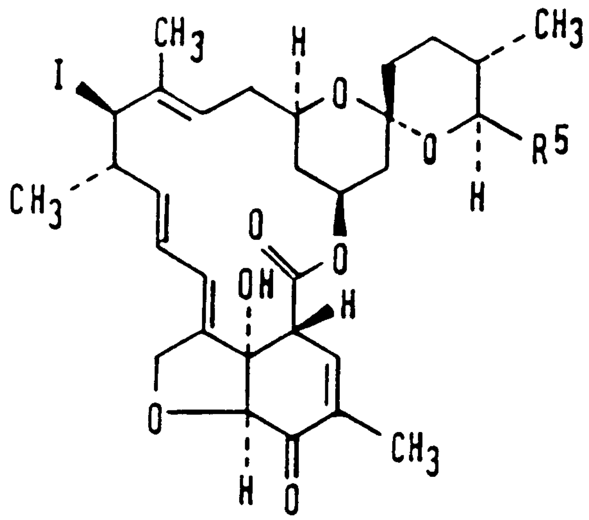

(III)

(in which $R^5$ is as defined in Claim 1) with a compound of formula (III'):

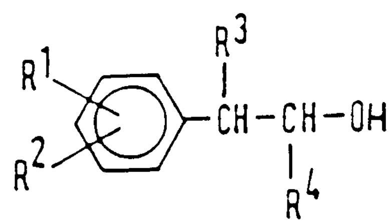

(III')

(in which $R^1$, $R^2$, $R^3$ and $R^4$ are as defined in Claim 1).

**Claims for the following Contracting States: ES, GR**

**1.** A process for preparing a compound of formula (I):

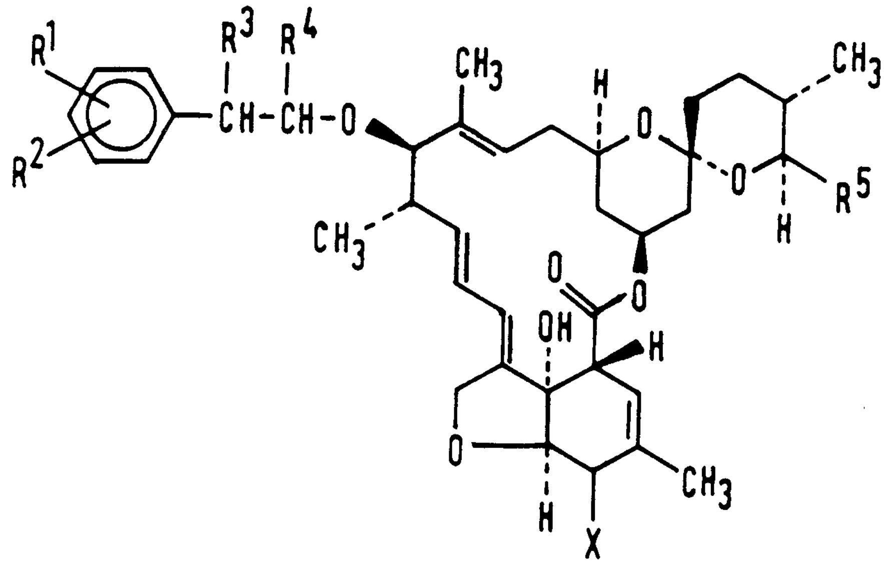

(I)

{in which:

$R^1$ and $R^2$ are the same or different and each represents: a hydrogen atom; a halogen atom; a cyano group; a nitro group; a $C_1$ - $C_4$ alkyl group; a substituted $C_1$ - $C_4$ alkyl group having at least one of substituents (a), defined below; a $C_1$ - $C_4$ alkoxy group; a $C_2$ - $C_6$ alkoxyalkoxy group; a group of formula $-(CH_2)_nNHR^9$,

in which: n represents 0 or the integer 1 or 2, and $R^9$ represents a hydrogen atom or a $C_1$ - $C_4$ alkyl group;

a group of formula $-(CH_2)_nNR^9C(=O)R^6$,

in which:

n and $R^9$ are as defined above, and

$R^6$ represents: a hydrogen atom; a $C_1$ - $C_4$ alkyl group; a substituted $C_1$ - $C_4$ alkyl group having at least one of substituents (b), defined below; a $C_2$ - $C_8$ aliphatic hydrocarbon group having one or two ethylenically unsaturated carbon-carbon double bonds, said group being unsubstituted or having at least one of substituents (b), defined below; a $C_2$ - $C_8$ alkynyl group; a substituted $C_2$ - $C_8$ alkynyl group having at least one of substituents (b), defined below; a $C_3$ - $C_8$ cycloalkyl group; a substituted $C_3$ - $C_8$ cycloalkyl group having at least one of substituents (c), defined below; a carbocyclic aryl group having from 6 to 14 ring carbon atoms and being unsubstituted or having at least one of substituents (c), defined below; or a heterocyclic group having from 3 to 6 ring atoms of which at least one is a nitrogen and/or oxygen and/or sulphur hetero-atom, said heterocyclic group being monocyclic or being fused to one or two benzene rings and being unsubstituted or having at least one of substituents (c), defined below;

a group of formula $-(CH_2)_nNR^9COCOR^6$

in which n, $R^6$ and $R^9$ are as defined above;

a group of formula $-(CH_2)_nR^9COCOOR^7$

in which n and $R^9$ are as defined above and $R^7$ represents a $C_1$ - $C_4$ alkyl group, a $C_3$ - $C_8$ cycloalkyl group or an aralkyl group as defined below;

a group of formula $-(CH_2)_nNR^9CHR^6NHCOR^6$

in which n, $R^6$ and $R^9$ are as defined above;

a group of formula $-(CH_2)_nNR^9CHR^6NHCONHR^6$

in which n, $R^6$ and $R^9$ are as defined above;

a group of formula $-(CH_2)_nNR^9CHR^6NHCOOR^7$

in which n, $R^6$, $R^7$ and $R^9$ are as defined above;

a group of formula $-(CH_2)_nNR^9C(=Y)YR^6$

in which n, $R^6$ and $R^9$ are as defined above and the two symbols Y are the same or different and

each represents an oxygen atom or a sulphur atom;
groups of formula $-(CH_2)_nNR^9C(=Y)NR^{6'}R^{6'}$
in which n, Y and $R^9$ are as defined above, and the two symbols $R^{6'}$ are the same or different and each represents one of the groups or atoms represented by $R^6$, or the two, together with the nitrogen atom to which they are attached, form a heterocyclic group having from 3 to 7 ring atoms of which one is said nitrogen atom and 0 or 1 is an additional nitrogen and/or oxygen and/or sulphur hetero-atom;
a group of formula $-(CH_2)_nNR^9C(=Y)NR^{6''}NR^{6''}$
in which n, Y and $R^9$ are as defined above, and each of the symbols $R^{6''}$ is the same or different and each represents one of the groups or atoms represented by $R^6$, or any two of the symbols $R^{6''}$, together with the nitrogen atom to which each is attached, forms a heterocyclic group having from 3 to 7 ring atoms of which one or two is said nitrogen atom or atoms and 0 or 1 is an additional nitrogen and/or oxygen and/or sulphur hetero-atom;
a group of formula $-(CH_2)_nNR^9C(=Y)NR^6NHZ$
in which n, Y, $R^6$ and $R^9$ are as defined above and Z represents
a group of formula $-COOR^7$, in which $R^7$ is as defined above,
a group of formula $-COR^6$, in which $R^6$ is as defined above, or
a group of formula $-SO_2R^6$, in which $R^6$ is as defined above;
a group of formula $-(CH_2)_nNR^9C(=NR^{10})NHR^{10}$
in which n and $R^9$ are as defined above and the two symbols $R^{10}$ are the same or different and each represents one of the groups or atoms represented by $R^6$, or a cyano group, a nitro group, a group of formula $-COOR^7$, in which $R^7$ is as defined above, or a group of formula $-COR^6$, in which $R^6$ is as defined above;
a group of formula $-(CH_2)_nNR^9C(=NR^{10})R^6$
in which n, $R^6$, $R^9$ and $R^{10}$ are as defined above;
a group of formula $-(CH_2)_nNR^9SO_mR^6$
in which n, $R^6$ and $R^9$ are as defined above and m is 1 or 2;
a group of formula $-CONHR^6$
in which $R^6$ is as defined above; and
a group of formula $-COOR^7$
in which $R^7$ is as defined above;
$R^3$ and $R^4$ are the same or different and each represents a hydrogen atom, a $C_1$ - $C_4$ alkyl group or a $C_1$ - $C_4$ alkoxy group;
$R^5$ represents a methyl group, an ethyl group, an isopropyl group or a sec-butyl group; and
X represents a hydroxy group, a $C_1$ - $C_5$ alkanoyloxy group, a substituted $C_1$ - $C_5$ alkanoyloxy group having at least one of substituents (d), defined below, or a hydroxyimino group;
said aralkyl groups have from 1 to 4 carbon atoms in the alkyl part and from 6 to 10 ring atoms in the aryl part, which is a carbocyclic aryl group which is unsubstituted or has at least one of substituents (c), defined below;
substituents (a):
halogen atoms, $C_1$ - $C_4$ alkoxy groups, $C_1$ - $C_4$ alkylthio groups and $C_1$ - $C_5$ alkanoyloxy groups;
substituents (b):
$C_3$ - $C_8$ cycloalkyl groups; $C_1$ - $C_4$ alkoxy groups; $C_1$ - $C_4$ alkylthio groups; $C_2$ - $C_5$ cyanoalkylthio groups; $C_2$ - $C_5$ alkoxycarbonyl groups; halogen atoms; cyano groups; nitro groups; amino groups; carbocyclic aryl groups having from 6 to 10 carbon atoms and being unsubstituted or having at least one of substituents (c), defined below; aromatic heterocyclic groups having from 5 to 8 ring atoms of which from 1 to 4 are nitrogen and/or oxygen and/or sulphur hetero-atoms, said heterocyclic group being monocyclic or being fused either to a benzene ring or to a heterocyclic group which has 5 or 6 ring atoms of which from 1 to 3 are nitrogen hetero-atoms and being unsubstituted or having at least one of substituents (c), defined below; and aryloxy and arylthio groups in which the aryl part has from 6 to 10 carbon atoms and is unsubstituted or has at least one of substituents (c), defined below;
substituents (c):
$C_1$ - $C_4$ alkyl groups, $C_1$ - $C_4$ alkoxy groups, $C_1$ - $C_4$ alkylthio groups, $C_1$ - $C_5$ alkanoyloxy groups, $C_2$ - $C_5$ alkoxycarbonyl groups, halogen atoms, cyano groups, nitro groups, amino groups, mono- and di-alkylamino groups in which the or each alkyl part is $C_1$ - $C_4$, carbamoyl groups, mono- and di-alkylcarbamoyl groups in which the or each alkyl part is $C_1$ - $C_4$, and $C_1$ - $C_5$ alkanoylamino groups;
substituents (d):
halogen atoms, $C_1$ - $C_4$ alkoxy groups, $C_2$ - $C_5$ alkoxycarbonyl groups and carboxy groups};
or a salt thereof,

which process comprises the steps:
reacting a compound of formula (IV):

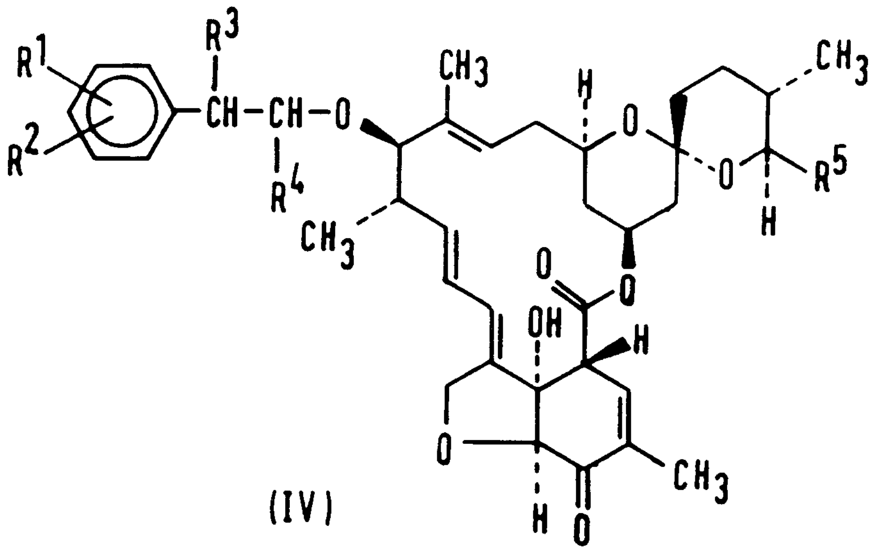

(IV)

(in which $R^1$, $R^2$, $R^3$, $R^4$ and $R^5$ are as defined above) with a reducing agent to prepare a compound of formula (I) in which X represents a hydroxy group;
and, if required, acylating said compound in which X represents a hydroxy group to prepare a compound of formula (I) in which X represents said alkanoyloxy group;
or reacting said compound of formula (IV) with hydroxylamine or with a salt thereof to prepare a compound of formula (I) in which X represents a hydroxyimino group.

**2.** A process according to Claim 1, in which said compound of formula (IV) is prepared by reacting a compound of formula (III):

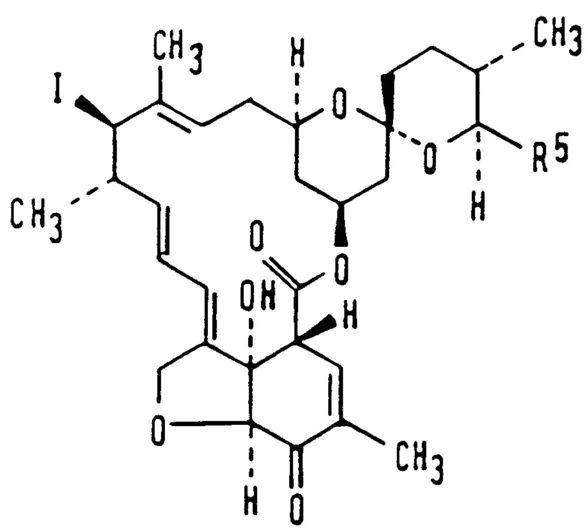

(III)

(in which $R^5$ is as defined in Claim 1) with a compound of formula (III'):

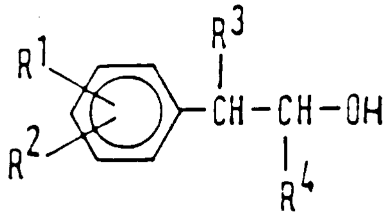

(III')

(in which $R^1$, $R^2$, $R^3$ and $R^4$ are as defined in Claim 1).

**3.** A process according to Claim 1 or Claim 2, in which the reagents and reaction conditions are so chosen as to prepare a compound of formula (I) or salt thereof in which:
$R^1$ represents a hydrogen atom; and
$R^2$ represents a hydrogen atom, a $C_1$ - $C_3$ alkyl group, a $C_1$ - $C_3$ alkoxy group, a fluorine or chlorine atom, a nitro group or an amino group.

**4.** A process according to Claim 1 or Claim 2, in which the reagents and reaction conditions are so chosen as to prepare a compound of formula (I) or salt thereof, in which:
$R^1$ represents a hydrogen atom; and
$R^2$ represents a group of formula $-(CH_2)_nNR^{9a}COR^{6a}$
in which n is 0, $R^{9a}$ represents a hydrogen atom or a methyl group, and $R^{6a}$ represents: a $C_1$ - $C_4$ alkyl group; a $C_3$ - $C_5$ cycloalkyl group; a $C_1$ - $C_3$ alkyl group substituted with a halogen, cyano, $C_1$ - $C_3$ alkoxy, $C_1$ - $C_3$ alkylthio, cyanomethylthio or phenoxy substituent; an alkenyl group; an unsubstituted phenyl group; a phenyl group substituted with a $C_1$ - $C_3$ alkyl, $C_1$ - $C_3$ alkoxy, halogen or nitro substituent; a pyridyl group; a pyrimidyl group; a pyrazyl group; a furyl group; or a thienyl group.

**5.** A process according to Claim 1 or Claim 2, in which the reagents and reaction conditions are so chosen as to prepare a compound of formula (I) or salt thereof, in which:
$R^1$ represents a hydrogen atom; and
$R^2$ represents a group of formula $-(CH_2)_nNR^{9a}COCOR^{6b}$
in which n is 0, $R^{9a}$ represents a hydrogen atom or a methyl group, and $R^{6b}$ represents a hydrogen atom, a $C_1$ - $C_4$ alkyl group, a $C_3$ - $C_5$ cycloalkyl group, an alkenyl group, an unsubstituted phenyl group, or a phenyl group substituted with a $C_1$ - $C_3$ alkyl, $C_1$ - $C_3$ alkoxy, halogen or nitro substituent.

**6.** A process according to Claim 1 or Claim 2, in which the reagents and reaction conditions are so chosen as to prepare a compound of formula (I) or salt thereof, in which:
$R^1$ represents a hydrogen atom; and
$R^2$ represents a group of formula $-(CH_2)_nNR^{9a}C(=Y)YR^{6c}$
in which n is 0, $R^{9a}$ represents a hydrogen atom or a methyl group, both Y are oxygen atoms and $R^{6c}$ represents a $C_1$ - $C_4$ alkyl group, a $C_1$ - $C_4$ alkyl group substituted with a halogen or $C_1$ - $C_3$ alkoxy substituent, an alkenyl group, a benzyl group, a methoxybenzyl group, a nitrobenzyl group, a furfuryl group, a thenyl group or a phenyl group.

**7.** A process according to Claim 1 or Claim 2, in which the reagents and reaction conditions are so chosen as to prepare a compound of formula (I) or salt thereof, in which:
$R^1$ represents a hydrogen atom; and
$R^2$ represents a group of formula $-(CH_2)_nNR^{9a}C(=Y)NR^{6d}R^{6e}$
in which n is 0, $R^{9a}$ represents a hydrogen atom or a methyl group, Y represents an oxygen atom or a sulphur atom, and $R^{6d}$ and $R^{6e}$ are the same or different and each represents a $C_1$ - $C_4$ alkyl group, a $C_3$ - $C_6$ cycloalkyl group, an unsubstituted phenyl group, or a phenyl group substituted with a $C_1$ - $C_3$ alkyl, $C_1$ - $C_3$ alkoxy, halogen or nitro substituent, or $R^{6d}$ and $R^{6e}$, together with the nitrogen atom to which they are attached, represent a piperidino, piperazino, morpholino, pyrrolidino or aziridino group.

**8.** A process according to Claim 1 or Claim 2, in which the reagents and reaction conditions are so chosen as to prepare a compound of formula (I) or salt thereof, in which:
$R^1$ represents a hydrogen atom; and

$R^2$ represents a group of formula $-(CH_2)_nNR^{9a}C(=Y)NR^{6f}NR^{6g}R^{6h}$

in which n is 0, $R^{9a}$ represents a hydrogen atom or a methyl group, Y represents an oxygen atom or a sulphur atom, and $R^{6f}$, $R^{6g}$ and $R^{6h}$ are the same or different and each represents: a hydrogen atom; a $C_1$ - $C_4$ alkyl group; a $C_3$ - $C_6$ cycloalkyl group; an unsubstituted phenyl group; or a phenyl group substituted with a $C_1$ - $C_3$ alkyl, $C_1$ - $C_3$ alkoxy, halogen or nitro substituent; or $R^{6g}$ and $R^{6h}$, together with the nitrogen atom to which they are attached, represent a piperidino, piperazino, morpholino, pyrrolidino or aziridino group; or $R^{6f}$ and $R^{6g}$, together with the nitrogen atoms to which they are attached, represent a pyrazolidinyl or tetrahydropyridazinyl group.

**9.** A process according to Claim 1 or Claim 2, in which the reagents and reaction conditions are so chosen as to prepare a compound of formula (I) or salt thereof, in which:

$R^1$ represents a hydrogen atom; and

$R^2$ represents a group of formula $-(CH_2)_nNR^{9a}C(=Y)NR^{6j}NHZ$

in which n is 0, $R^{9a}$ represents a hydrogen atom or a methyl group, Y represents an oxygen atom or a sulphur atom, $R^{6j}$ represents a hydrogen atom, a $C_1$ - $C_4$ alkyl group or a $C_3$ - $C_6$ cycloalkyl group; and Z represents a group of formula $-COOR^{7a}$

in which $R^{7a}$ represents a $C_1$ - $C_4$ alkyl group, a $C_3$ - $C_6$ cycloalkyl group or a benzyl group,

a group of formula $-COR^{6k}$

in which $R^{6k}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent or a group of formula $-SO_2R^{6m}$

in which $R^{6m}$ represents a $C_1$ - $C_4$ alkyl group, an unsubstituted phenyl group or a phenyl group substituted with a $C_1$ - $C_3$ alkyl, $C_1$ - $C_3$ alkoxy, halogen or nitro substituent.

**10.** A process according to Claim 1 or Claim 2, in which the reagents and reaction conditions are so chosen as to prepare a compound of formula (I) or salt thereof, in which:

$R^1$ represents a hydrogen atom; and

$R^2$ represents a group of formula $-(CH_2)_nNR^{9a}C(=NR^{10a})NHR^{10b}$

in which n is 0, $R^{9a}$ represents a hydrogen atom or a methyl group, and $R^{10a}$ and $R^{10b}$ are the same or different and each represents: a hydrogen atom; a $C_1$ - $C_4$ alkyl group; an unsubstituted phenyl group; a phenyl group substituted with a $C_1$ - $C_3$ alkyl, $C_1$ - $C_3$ alkoxy, halogen or nitro substituent; a group of formula $-COOR^{7b}$

in which $R^{7b}$ represents a $C_1$ - $C_4$ alkyl group, a $C_3$ - $C_6$ cycloalkyl group or a benzyl group; or

a group of formula $-COR^{6n}$

in which $R^{6n}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent.

**11.** A process according to Claim 1 or Claim 2, in which the reagents and reaction conditions are so chosen as to prepare a compound of formula (I) or salt thereof, in which:

$R^1$ represents a hydrogen atom; and

$R^2$ represents a group of formula $-(CH_2)_nNR^{9a}C(=NR^{10c})R^{6p}$

in which n is 0; $R^{9a}$ represents a hydrogen atom or a methyl group; $R^{10c}$ represents a hydrogen atom, a $C_1$ - $C_4$ alkyl group, an unsubstituted phenyl group, a phenyl group substituted with a $C_1$ - $C_3$ alkyl, $C_1$ - $C_3$ alkoxy, halogen or nitro substituent, a group of formula $-COOR^{7c}$

in which $R^{7c}$ represents a $C_1$ - $C_4$ alkyl group, a $C_3$ - $C_6$ cycloalkyl group or a benzyl group;

or a group of formula $-COR^{6q}$

in which $R^{6q}$ represents a $C_1$ - $C_4$ alkyl group, a $C_3$ - $C_6$ cycloalkyl group, an unsubstituted phenyl group; or a phenyl group substituted with a $C_1$ - $C_3$ alkyl, $C_1$ - $C_3$ alkoxy, halogen or nitro substituent and

$R^{6p}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent.

**12.** A process according to Claim 1 or Claim 2, in which the reagents and reaction conditions are so chosen as to prepare a compound of formula (I) or salt thereof, in which:

$R^1$ represents a hydrogen atom; and

$R^2$ represents a group of formula $-(CH_2)_nNR^{9a}SO_mR^{6r}$

in which n is 0, $R^{9a}$ represents a hydrogen atom or a methyl group, m is 1 or 2, and $R^{6r}$ represents a $C_1$ - $C_4$ alkyl group, a $C_2$ - $C_4$ cyanoalkyl group, an unsubstituted phenyl group, a phenyl group

substituted with a $C_1$ - $C_3$ alkyl, $C_1$ - $C_3$ alkoxy, halogen or nitro substituent.

**13.** A process according to Claim 1 or Claim 2, in which the reagents and reaction conditions are so chosen as to prepare a compound of formula (I) or salt thereof, in which:
$R^1$ represents a hydrogen atom, and
$R^2$ represents a group of formula $-CONHR^{6s}$
in which $R^{6s}$ represents a $C_1$ - $C_4$ alkyl group, a $C_3$ - $C_6$ cycloalkyl group, an unsubstituted phenyl group or a phenyl group substituted with a $C_1$ - $C_3$ alkyl, $C_1$ - $C_3$ alkoxy, halogen or nitro substituent.

**14.** A process according to Claim 1 or Claim 2, in which the reagents and reaction conditions are so chosen as to prepare a compound of formula (I) or salt thereof, in which:
$R^1$ represents a hydrogen atom, and
$R^2$ represents a group of formula $-COOR^{7d}$
in which $R^{7d}$ represents a methyl, ethyl or benzyl group.

**15.** A process according to Claim 1 or Claim 2, in which the reagents and reaction conditions are so chosen as to prepare a compound of formula (I) or salt thereof, in which:
$R^1$ represents a methoxy group, and
$R^2$ represents a $C_1$ - $C_3$ alkoxy group or a $C_2$ - $C_4$ alkoxyalkoxy group.

**16.** A process according to any one of the preceding Claims, in which the reagents and reaction conditions are so chosen as to prepare a compound of formula (I) or salt thereof in which $R^2$ is attached at the p-position of the benzene ring.

**17.** A process according to any one of the preceding Claims, in which the reagents and reaction conditions are so chosen as to prepare a compound of formula (I) or salt thereof in which $R^5$ represents an ethyl group.

**18.** A process according to any one of Claims 1 to 15, in which the reagents and reaction conditions are so chosen as to prepare a composition comprising a mixture of compounds of formula (I) or salts thereof in which, in one of said compounds, $R^5$ represents an ethyl group and, in the other of said compounds, $R^5$ represents a methyl group.

**19.** A process according to any one of the preceding Claims, in which the reagents and reaction conditions are so chosen as to prepare a compound of formula (I) or salts thereof in which $R^3$ and $R^4$ are hydrogen atoms.

**20.** A process according to any one of the preceding Claims, in which the reagents and reaction conditions are so chosen as to prepare a compound of formula (I) or salt thereof in which X represents a hydroxy group.

**21.** A process according to any one of the preceding Claims, in which the reagents and reaction conditions are so chosen as to prepare:
13-[2-(4-acetamidophenyl)ethoxy]milbemycin $A_4$;
13-[2-(4-cyanoacetamidophenyl)ethoxy]milbemycin $A_4$;
13-{2-[4-(2-cyanopropionamido)phenyl]ethoxy}milbemycin $A_4$;
13-[2-(4-methoxyacetamidophenyl)ethoxy]milbemycin $A_4$;
13-{2-[4-(cyclopropylcarbonylamino)phenyl]ethoxy}milbemycin $A_4$;
13-{2-[4-(cyclobutylcarbonylamino)phenyl]ethoxy}milbemycin $A_4$;
13-{2-[4-(4-cyanobenzamido)phenyl]ethoxy}milbemycin $A_4$;
13-[2-(4-methoxycarbonylaminophenyl)ethoxy]milbemycin $A_4$;
13-[2-(4-vinyloxycarbonylaminophenyl)ethoxy]milbemycin $A_4$;
13-{2-[4-(3-methylureido)phenyl]ethoxy}milbemycin $A_4$;
13-{2-[4-(3-phenylureido)phenyl]ethoxy}milbemycin $A_4$;
13-{2-[4-(3-cyclohexylureido)phenyl]ethoxy}milbemycin $A_4$;
13-[2-(4-methanesulphonylaminophenyl)ethoxy]milbemycin $A_4$;
13-[2-(4-ethanesulphonylaminophenyl)ethoxy]milbemycin $A_4$;
13-{2-[4-(3,3-dimethylcarbazoylamino)phenyl]ethoxy}milbemycin $A_4$;

13-{2-[4-(3-o-fluorophenylureido)phenyl]ethoxy}milbemycin $A_4$;
13-{2-[4-(3-p-fluorophenylureido)phenyl]ethoxy}milbemycin $A_4$;
13-{2-[4-(3-p-methoxyphenylureido)phenyl]ethoxy}milbemycin $A_4$;
or a salt thereof.

**22.** A process for preparing an anthelmintic, acaricidal and insecticidal composition comprising mixing an anthelmintic, acaricidal and insecticidal compound with a pharmaceutically, agriculturally, veterinarily or horticulturally acceptable carrier or diluent, in which said compound is at least one compound prepared according to any one of the preceding Claims.

**23.** The use for the manufacture of a medicament for the treatment of infections by helminths, acarids or insects of at least one compound of formula (I) as defined in any one of Claims 1 to 21 or a composition prepared according to Claim 22.

## Patentansprüche

## Patentansprüche für folgende Vertragsstaaten : GB, DE, FR, IT, CH, BE, NL, SE, LU, AT

**1.** Verbindungen der Formel (I)

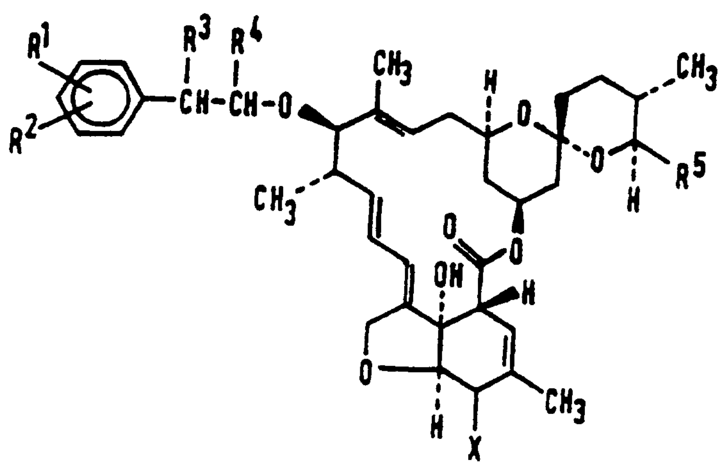

(I)

worin

$R^1$ und $R^2$ gleich oder verschieden sind und jeweils einen der folgenden Reste darstellen: ein Wasserstoffatom, ein Halogenatom, eine Cyangruppe, eine Nitrogruppe, eine $C_1$-$C_4$-Alkylgruppe, eine substituierte $C_1$-$C_4$-Alkylgruppe mit mindestens einem der nachstehend definierten Substituenten (a), eine $C_1$-$C_4$-Alkoxyruppe, eine $C_2$-$C_6$-Alkoxyalkoxygruppe, eine Gruppe der Formel $-(CH_2)_nNHR^9$,

worin n 0 oder die ganze Zahl 1 oder 2 darstellt und $R^9$ ein Wasserstoffatom oder eine $C_1$-$C_4$-Alkylgruppe darstellt,

eine Gruppe der Formel $-(CH_2)_nNR^9C(=O)R^6$, worin

n und $R^9$ wie vorstehend definiert sind, und $R^6$ einen der folgenden Reste darstellt: ein Wasserstoffatom, eine $C_1$-$C_4$-Alkylgruppe, eine substituierte $C_1$-$C_4$-Alkylgruppe mit mindestens einem der nachstehend definierten Substituenten (b), eine aliphatische $C_2$-$C_8$-Kohlenwasserstoffgruppe mit einer oder zwei ethylenisch ungesättigten Kohlenstoff-Kohlenstoff-Doppelbindung(en), wobei die Gruppe nicht substituiert ist oder mindestens einen der nachstehend definierten Substituenten (b) aufweist, eine $C_2$-$C_8$-Alkinylgruppe, eine substituierte $C_2$-$C_8$-Alkinylgruppe mit mindestens einem der nachstehend definierten Substituenten (b), eine $C_3$-$C_8$-Cycloalkylgruppe, eine substituierte $C_3$-$C_8$-Cycloalkylgruppe mit mindestens einem der nachstehend definierten Substituenten (c), eine carbocyclische Arylgruppe mit 6 bis 14 Ringkohlenstoffatomen, die nicht substituiert ist oder mindestens einen der nachstehend definierten Substituenten (c) aufweist, oder eine heterocyclische Gruppe mit 3 bis 6 Ringatomen, von denen mindestens eines ein Stickstoff- und/oder Sauerstoff- und/oder Schwefel-Heteroatom ist, wobei die heterocyclische Gruppe monocyclisch ist oder an einen oder zwei Benzolring(e) ankondensiert ist und nicht substituiert ist oder mindestens einen der nachstehend definierten Substituenten (c) aufweist,

eine Gruppe der Formel $-(CH_2)_nNR^9COCOR^6$,

worin n, $R^6$ und $R^9$ wie vorstehend definiert sind,

eine Gruppe der Formel $-(CH_2)_nR^9COCOOR^7$,
worin n und $R^9$ wie vorstehend definiert sind und $R^7$ eine $C_1$-$C_4$-Alkylgruppe, eine $C_3$-$C_8$-Cycloalkylgruppe oder eine Aralkylgruppe, wie sie nachstehend definiert ist, bedeutet,
eine Gruppe der Formel $-(CH_2)_nNR^9CHR^6NHCOR^6$
worin n, $R^6$ und $R^9$ wie vorstehend definiert sind,
eine Gruppe der Formel $-(CH_2)_nNR^9CHR^6NHCONHR^6$,
worin n, $R^6$ und $R^9$ wie vorstehend definiert sind,
eine Gruppe der Formel $-(CH_2)_nNR^9CHR^6NHCOOR^7$,
worin n, $R^6$, $R^7$ und $R^9$ wie vorstehend definiert sind,
eine Gruppe der Formel $-(CH_2)_nNR^9C(=Y)YR^6$,
worin n, $R^6$ und $R^9$ wie vorstehend definiert sind und die zwei Symbole Y gleich oder verschieden sind und jeweils ein Sauerstoffatom oder ein Schwefelatom darstellen,
Gruppen der Formel $-(CH_2)_nNR^9C(=Y)NR^{6'}R^{6'}$,
worin n, Y und $R^9$ wie vorstehend definiert sind und die zwei Symbole $R^{6'}$ gleich oder verschieden sind und jeweils eine der durch $R^6$ dargestellten Gruppen oder Atome darstellen, oder die zwei zusammen mit dem Stickstoffatom, an das sie gebunden sind, eine heterocyclische Gruppe mit 3 bis 7 Ringatomen bilden, von denen eines das Stickstoffatom ist und 0 oder 1 ein zusätzliches Stickstoff- und/oder Sauerstoff- und/oder Schwefel-Heteroatom ist,
eine Gruppe der Formel $-(CH_2)_nNR^9C(=Y)NR^{6''}NR^{6''}R^{6''}$,
worin n, Y und $R^9$ wie vorstehend definiert sind und die Symbole $R^{6''}$ jeweils gleich oder verschieden sind und jeweils eine der durch $R^6$ dargestellten Gruppen oder Atome darstellen, oder beliebige zwei der Symbole $R^{6''}$ zusammen mit dem Stickstoffatom, an das sie gebunden sind, eine heterocyclische Gruppe mit 3 bis 7 Ringatomen bilden, von denen eines oder zwei das Stickstoffatom ist oder die Stickstoffatome sind und 0 oder 1 ein zusätzliches Stickstoff- und/oder Sauerstoff-und/oder Schwefel-Heteroatom ist,
eine Gruppe der Formel $-(CH_2)_nNR^9C(=Y)NR^6NHZ$
worin n, Y, $R^6$ und $R^9$ wie vorstehend definiert sind und Z einen der folgenden Reste darstellt:
eine Gruppe der Formel $-COOR^7$, worin $R^7$ wie vorstehend definiert ist,
eine Gruppe der Formel $-COR^6$, worin $R^6$ wie vorstehend definiert ist, oder
eine Gruppe der Formel $-SO_2R^6$, worin $R^6$ wie vorstehend definiert ist,
eine Gruppe der Formel $-(CH_2)_nNR^9C(=NR^{10})NHR^{10}$
worin n und $R^9$ wie vorstehend definiert sind und die zwei Symbole $R^{10}$ gleich oder verschieden sind und jeweils eine der durch $R^6$ dargestellten Gruppen oder Atome oder eine Cyangruppe, eine Nitrogruppe, eine Gruppe der Formel $-COOR^7$, worin $R^7$ wie vorstehend definiert ist, oder eine Gruppe der Formel $-COR^6$, worin $R^6$ wie vorstehend definiert ist, darstellen,
eine Gruppe der Formel $-(CH_2)_nNR^9C(=NR^{10})R^6$,
worin n, $R^6$, $R^9$ und $R^{10}$ wie vorstehend definiert sind, eine Gruppe der Formel $-(CH_2)_nNR^9SO_mR^6$,
worin n, $R^6$ und $R^9$ wie vorstehend definiert sind und m 1 oder 2 ist,
eine Gruppe der Formel $-CONHR^6$,
worin $R^6$ wie vorstehend definiert ist, und
eine Gruppe der Formel $-COOR^7$,
worin $R^7$ wie vorstehend definiert ist,
$R^3$ und $R^4$ gleich oder verschieden sind und jeweils ein Wasserstoffatom, eine $C_1$-$C_4$-Alkylgruppe oder eine $C_1$-$C_4$-Alkoxygruppe darstellen,
$R^5$ eine Methylgruppe, eine Ethylgruppe, eine Isopropylgruppe oder eine sec.-Butylgruppe darstellt, und
X eine Hydroxygruppe, eine $C_1$-$C_5$-Alkanoyloxygruppe, eine substituierte $C_1$-$C_5$-Alkanoyloxygruppe mit mindestens einem der nachstehend definierten Substituenten (d) oder eine Hydroxyiminogruppe darstellt,
wobei die Aralkylgruppen in dem Alkylteil 1 bis 4 Kohlenstoffatome und in dem Arylteil, welcher eine carbocyclische Arylgruppe darstellt, die nicht substituiert ist, oder mindestens einen der nachstehend definierten Substituenten (c) aufweist, 6 bis 10 Ringatome aufweisen,
Substituenten (a):
Halogenatome, $C_1$-$C_4$-Alkoxygruppen, $C_1$-$C_4$-Alkylthiogruppen und $C_1$-$C_5$-Alkanoyloxygruppen,
Substituenten (b):
$C_3$-$C_8$-Cycloalkylgruppen, $C_1$-$C_4$-Alkoxygruppen, $C_1$-$C_4$-Alkylthiogruppen, $C_2$-$C_5$-Cyanalkylthiogruppen, $C_2$-$C_5$-Alkoxycarbonylgruppen, Halogenatome, Cyangruppen, Nitrogruppen, Aminogruppen, carbocyclische Arylgruppen mit 6 bis 10 Kohlenstoffatomen, die nicht substituiert sind oder mindestens einen der

nachstehend definierten Substituenten (c) aufweisen, aromatische heterocyclische Gruppen mit 5 bis 8 Ringatomen, von denen 1 bis 4 Stickstoff- und/oder Sauerstoff- und/oder Schwefel-Heteroatome sind, wobei die heterocyclische Gruppe monocyclisch ist oder entweder an einen Benzolring oder an eine heterocyclische Gruppe mit 5 bis 6 Ringatomen, von denen 1 bis 3 Stickstoffheteroatome sind, ankondensiert ist und nicht substituiert ist oder mindestens einen der nachstehend definierten Substituenten (c) aufweist, und Aryloxy- und Arylthiogruppen, worin der Arylteil 6 bis 10 Kohlenstoffatome aufweist und nicht substituiert ist oder mindestens einen der nachstehend definierten Substituenten (c) aufweist,

Substituenten (c) :

$C_1$-$C_4$-Alkylgruppen, $C_1$-$C_4$-Alkoxygruppen, $C_1$-$C_4$-Alkylthiogruppen, $C_1$-$C_5$-Alkanoyloxygruppen, $C_2$-$C_5$-Alkoxycarbonylgruppen, Halogenatome, Cyangruppen, Nitrogruppen, Aminogruppen, Mono- und Dialkylaminogruppen, worin der oder jeder Alkylteil $C_1$-$C_4$ ist, Carbamoylgruppen, Mono- und Dialkylcarbamoylgruppen, worin der oder jeder Alkylteil $C_1$-$C_4$ ist und $C_1$-$C_5$-Alkanoylaminogruppen,

Substituenten (d):

Halogenatome, $C_1$-$C_4$-Alkoxygruppen, $C_2$-$C_5$-Alkoxycarbonylgruppen und Carboxygruppen,
und Salze davon.

**2.** Verbindung nach Anspruch 1, worin:
$R^1$ ein Wasserstoffatom darstellt, und
$R^2$ ein Wasserstoffatom, eine $C_1$-$C_3$-Alkylgruppe, eine $C_1$-$C_3$-Alkoxygruppe, ein Fluor- oder ein Chloratom, eine Nitrogruppe oder eine Aminogruppe darstellt.

**3.** Verbindung nach Anspruch 1, worin:
$R^1$ ein Wasserstoffatom darstellt, und
$R^2$ eine Gruppe der Formel $-(CH_2)_nNR^{9a}COR^{6a}$ darstellt,
worin n 0 ist, $R^{9a}$ ein Wasserstoffatom oder eine Methylgruppe darstellt und $R^{6a}$ darstellt: eine $C_1$-$C_4$-Alkylgruppe, eine $C_3$-$C_5$-Cycloalkylgruppe, eine $C_1$-$C_3$-Alkylgruppe, die mit einem Halogen-, einem Cyan-, einem $C_1$-$C_3$-Alkoxy-, einem $C_1$-$C_3$-Alkylthio-, einem Cyanmethylthio- oder einem Phenoxy-Substituenten substituiert ist, eine Alkenylgruppe, eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, eine Pyridylgruppe, eine Pyrimidylgruppe, eine Pyrazylgruppe, eine Furylgruppe oder eine Thienylgruppe.

**4.** Verbindung nach Anspruch 1, worin:
$R^1$ ein Wasserstoffatom darstellt, und
$R^2$ eine Gruppe der Formel $-(CH_2)_nNR^{9a}COCOR^{6b}$ darstellt, worin n 0 ist, $R^{9a}$ ein Wasserstoffatom oder eine Methylgruppe darstellt und $R^{6b}$ ein Wasserstoffatom, eine $C_1$-$C_4$-Alkylgruppe, eine $C_3$-$C_5$-Cycloalkylgruppe, eine Alkenylgruppe, eine nicht substituierte Phenylgruppe oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**5.** Verbindung nach Anspruch 1, worin:
$R^1$ ein Wasserstoffatom darstellt, und
$R^2$ eine Gruppe der Formel $-(CH_2)_nNR^{9a}C(=Y)YR^{6c}$ darstellt,
worin n 0 ist, $R^{9a}$ ein Wasserstoffatom oder eine Methylgruppe darstellt, beide Y Sauerstoffatome sind und $R^{6c}$ eine $C_1$-$C_4$-Alkylgruppe, eine $C_1$-$C_4$-Alkylgruppe, die mit einem Halogen- oder einem $C_1$-$C_3$-Alkoxy-Substituenten substituiert ist, eine Alkenylgruppe, eine Benzylgruppe, eine Methoxybenzylgruppe, eine Nitrobenzylgruppe, eine Furfurylgruppe, eine Thienylgruppe oder eine Phenylgruppe darstellt.

**6.** Verbindung nach Anspruch 1, worin:
$R^1$ ein Wasserstoffatom darstellt, und
$R^2$ eine Gruppe der Formel $-(CH_2)_nNR^{9a}C(=Y)NR^{6d}R^{6e}$ darstellt,
worin n 0 ist, $R^{9a}$ ein Wasserstoffatom oder eine Methylgruppe darstellt, Y ein Sauerstoffatom oder ein Schwefelatom darstellt und $R^{6d}$ und $R^{6e}$ gleich oder verschieden sind und jeweils eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, oder $R^{6d}$ und $R^{6e}$ zusammen mit dem Stickstoffatom, an das sie gebunden sind,

eine Piperidino-, Piperazino-, Morpholino-, Pyrrolidino- oder Aziridinogruppe darstellen.

**7.** Verbindung nach Anspruch 1, worin:
$R^1$ ein Wasserstoffatom darstellt, und
$R^2$ eine Gruppe der Formel $-(CH_2)_nNR^{9a}C(=Y)NR^{6f}NR^{6g}R^{6h}$ darstellt,
worin n 0 ist, $R^{9a}$ ein Wasserstoffatom oder eine Methylgruppe darstellt, Y ein Sauerstoffatom oder ein Schwefelatom darstellt und $R^{6f}$, $R^{6g}$ und $R^{6h}$ gleich oder verschieden sind und jeweils einen der folgenden Reste darstellen: ein Wasserstoffatom, eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, oder $R^{6g}$ und $R^{6h}$ zusammen mit dem Stickstoffatom, an das sie gebunden sind, eine Piperidino-, Piperazino-, Morpholino-, Pyrrolidino- oder Aziridinogruppe darstellen, oder $R^{6f}$ und $R^{6g}$ zusammen mit den Stickstoffatomen, an die sie gebunden sind, eine Pyrazolidinyl- oder Tetrahydropyridazinylgruppe darstellen.

**8.** Verbindung nach Anspruch 1, worin:
$R^1$ ein Wasserstoffatom darstellt, und
$R^2$ eine Gruppe der Formel $-(CH_2)_nNR^{9a}C(=Y)NR^{6j}NHZ$ darstellt,
worin n 0 ist, $R^{9a}$ ein Wasserstoffatom oder eine Methylgruppe darstellt, Y ein Sauerstoffatom oder ein Schwefelatom darstellt, $R^{6j}$ ein Wasserstoffatom, eine $C_1$-$C_4$-Alkylgruppe oder eine $C_3$-$C_6$-Cycloalkylgruppe darstellt und Z eine Gruppe der Formel $-COOR^{7a}$ darstellt,
worin $R^{7a}$ eine $C_1$-$C_4$-Alkylgruppe, eine $C_3$-$C_6$-Cycloalkylgruppe oder eine Benzylgruppe darstellt,
eine Gruppe der Formel $-COR^{6k}$ darstellt,
worin $R^{6k}$ eine $C_1$-$C_4$-Alkylgruppe, eine $C_3$-$C_6$-Cycloalkylgruppe, eine nicht substituierte Phenylgruppe oder eine Phenylgruppe, die mit einem $C_1$-$C_3$-Alkyl-, einem $C_1$-$C_3$-Alkoxy-, einem Halogen, oder einem Nitro-Substituenten substituiert ist, oder
eine Gruppe der Formel $-SO_2R^{6m}$ darstellt,
worin $R^{6m}$ eine $C_1$-$C_4$-Alkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**9.** Verbindung nach Anspruch 1, worin:
$R^1$ ein Wasserstoffatom darstellt, und
$R^2$ eine Gruppe der Formel $-(CH_2)_nNR^{9a}C(=NR^{10a})NHR^{10b}$ darstellt,
worin n 0 ist, $R^{9a}$ ein Wasserstoffatom oder eine Methylgruppe darstellt, und $R^{10a}$ und $R^{10b}$ gleich oder verschieden sind und jeweils die folgenden Reste darstellen: ein Wasserstoffatom, eine $C_1$-$C_4$-Alkylgruppe, eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, eine Gruppe der Formel $-COOR^{7b}$, worin $R^{7b}$ eine $C_1$-$C_4$-Alkylgruppe, eine $C_3$-$C_6$-Cycloalkylgruppe oder eine Benzylgruppe darstellt, oder eine Gruppe der Formel $-COR^{6n}$, worin $R^{6n}$ eine $C_1$-$C_4$-Alkylgruppe, eine $C_3$-$C_6$-Cycloalkylgruppe, eine nicht substituierte Phenylgruppe oder eine Phenylgruppe, die mit einem $C_1$-$C_3$-Alkyl-, einem $C_1$-$C_3$-Alkoxy-, einem Halogen, oder einem Nitro-Substituenten substituiert ist, darstellt.

**10.** Verbindung nach Anspruch 1, worin:
$R^1$ ein Wasserstoffatom darstellt, und
$R^2$ eine Gruppe der Formel $-(CH_2)_nNR^{9a}C(=NR^{10c})R^{6p}$ darstellt,
worin n 0 ist, $R^{9a}$ ein Wasserstoffatom oder eine Methylgruppe darstellt, $R^{10c}$ einen der folgenden Reste darstellt: ein Wasserstoffatom, eine $C_1$-$C_4$-Alkylgruppe,eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, eine Gruppe der Formel $-COOR^{7c}$, worin $R^{7c}$ eine $C_1$-$C_4$-Alkylgruppe, eine $C_3$-$C_6$-Cycloalkylgruppe oder eine Benzylgruppe darstellt, oder eine Gruppe der Formel $-COR^{6q}$, worin $R^{6q}$ eine $C_1$-$C_4$-Alkylgruppe, eine $C_3$-$C_6$-Cycloalkylgruppe, eine nicht substituierte Phenylgruppe oder eine Phenylgruppe, die mit einem $C_1$-$C_3$-Alkyl-, einem $C_1$-$C_3$-Alkoxy-, einem Halogen-, oder einem Nitro-Substituenten substituiert ist, und $R^{6p}$ eine $C_1$-$C_4$-Alkylgruppe, eine $C_3$-$C_6$-Cyclo alkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**11.** Verbindung nach Anspruch 1, worin:
$R^1$ ein Wasserstoffatom darstellt, und
$R^2$ eine Gruppe der Formel $-(CH_2)_nNR^{9a}SO_mR^{6r}$ darstellt,
worin n 0 ist, $R^{9a}$ ein Wasserstoffatom oder eine Methylgruppe darstellt, m 1 oder 2 ist und $R^{6r}$ eine $C_1$-$C_4$-Alkylgruppe, eine $C_2$-$C_4$-Cyanalkylgruppe, eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**12.** Verbindung nach Anspruch 1, worin:
$R^1$ ein Wasserstoffatom darstellt, und
$R^2$ eine Gruppe der Formel $-CONHR^{6s}$ darstellt,
worin $R^{6s}$ eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**13.** Verbindung nach Anspruch 1, worin:
$R^1$ ein Wasserstoffatom darstellt, und
$R^2$ eine Gruppe der Formel $-COOR^{7d}$ darstellt,
worin $R^{7d}$ eine Methyl-, Ethyl-, oder Benzylgruppe darstellt.

**14.** Verbindung nach Anspruch 1, worin:
$R^1$ eine Methoxygruppe darstellt, und
$R^2$ eine $C_1$-$C_3$-Alkoxygruppe oder eine $C_2$-$C_4$-Alkoxyalkoxygruppe darstellt.

**15.** Verbindung nach einem der vorhergehenden Ansprüche, worin $R^2$ in der para-Position des Benzolrings gebunden ist.

**16.** Verbindung nach einem der vorhergehenden Ansprüche, worin $R^5$ eine Ethylgruppe darstellt.

**17.** Zusammensetzung, die ein Gemisch von Verbindungen, von denen jede wie in einem der Ansprüche 1 bis 15 definiert ist, umfaßt, worin in einer der Verbindungen $R^5$ eine Ethylgruppe darstellt und in der anderen der Verbindungen $R^5$ eine Methylgruppe darstellt.

**18.** Verbindung nach einem der vorhergehenden Ansprüche, worin $R^3$ und $R^4$ Wasserstoffatome sind.

**19.** Verbindung nach einem der vorhergehenden Ansprüche, worin X eine Hydroxygruppe darstellt.

**20.** 13-[2-(4-Acetamidophenyl)ethoxy]milbemycin $A_4$.

**21.** 13-[2-(4-Cyanacetamidophenyl)ethoxy]milbemycin $A_4$.

**22.** 13-{2-[4-(2-Cyanpropionamido)phenyl]ethoxy}milbemycin $A_4$.

**23.** 13-[2-(4-Methoxyacetamidophenyl)ethoxy]milbemycin $A_4$.

**24.** 13-{2-[4-(Cyclopropylcarbonylamino)phenyl]ethoxy}milbemycin $A_4$.

**25.** 13-{2-[4-(Cyclobutylcarbonylamino)phenyl]ethoxy}milbemycin $A_4$.

**26.** 13-{2-[4-(4-Cyanbenzamido)phenyl]ethoxy}milbemycin $A_4$.

**27.** 13-[2-(4-Methoxycarbonylaminophenyl)ethoxy]milbemycin $A_4$.

**28.** 13-[2-(4-Vinyloxycarbonylaminophenyl)ethoxy]milbemycin $A_4$.

**29.** 13-{2-[4-(3-Methylureido)phenyl]ethoxy}milbemycin $A_4$.

**30.** 13-{2-[4-(3-Phenylureido)phenyl]ethoxy}milbemycin $A_4$.

**31.** 13-{2-[4-(3-Cyclohexylureido)phenyl]ethoxy}milbemycin $A_4$.

**32.** 13-[2-(4-Methansulfonylaminophenyl)ethoxy]milbemycin $A_4$.

**33.** 13-[2-(4-Ethansulfonylaminophenyl)ethoxy]milbemycin $A_4$.

**34.** 13-{2-[4-(3,3-Dimethylcarbazoylamino)phenyl] ethoxy}milbemycin $A_4$ und Salze davon.

**35.** 13-{2-[4-(3-o-Fluorphenylureido)phenyl] ethoxy}milbemycin $A_4$.

**36.** 13-{2-[4-(3-p-Fluorphenylureido)phenyl]ethoxy}milbemycin $A_4$.

**37.** 13-{2-[4-(3-p-Methoxyphenylureido)phenyl] ethoxy}milbemycin $A_4$.

**38.** Anthelmintische, mitizide und insektizide Zusammensetzung, welche eine anthelmintische, mitizide und insektizide Verbindung im Gemisch mit einem pharmazeutisch, landwirtschaftlich, veterinärmedizinisch oder im Gartenbau geeignete Träger oder Verdünnungsmittel umfaßt, wobei die Verbindung mindestens eine der Verbindungen nach einem der vorhergehenden Ansprüche ist.

**39.** Verwendung mindestens einer Verbindung nach einem der Ansprüche 1 bis 37 oder einer Zusammensetzung nach Anspruch 38 zur Herstellung eines Arzneimittels zur Behandlung von Infektionen durch Würmer, Milben oder Insekten.

**40.** Verfahren zum Schutz von Pflanzen vor Schädigungen durch Milben, Würmer oder Insekten, bei dem: eine aktive Verbindung auf die Pflanzen oder die Sämlinge der Pflanzen oder auf einen Ort, an dem sich die Pflanzen oder die Sämlinge befinden ausgebracht wird, wobei die aktive Verbindung mindestens eine Verbindung nach einem der Ansprüche 1 bis 37 oder eine Zusammensetzung nach Anspruch 38 ist.

**41.** Verfahren zur Herstellung einer Verbindung nach einem der Ansprüche 1 bis 37, das die folgenden Schritte umfaßt:
Umsetzen einer Verbindung der Formel (IV)

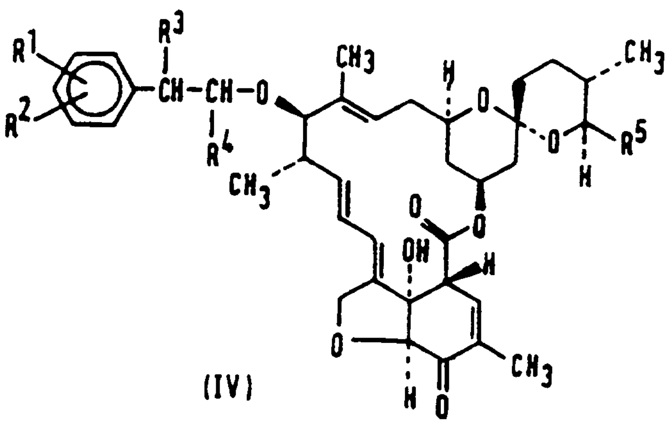

(IV)

(worin $R^1$, $R^2$, $R^3$, $R^4$ und $R^5$ wie in Anspruch 1 definiert sind) mit einem Reduktionsmittel, wobei eine Verbindung der Formel (I) hergestellt wird, in der X eine Hydroxylgruppe darstellt, und, falls erforderlich, Acylieren der Verbindung, in der X eine Hydroxygruppe darstellt, wobei eine Verbindung der Formel (I) hergestellt wird, in der X die Alkanoyloxygruppe darstellt,
oder Umsetzen der Verbindung der Formel (IV) mit Hydroxylamin oder mit einem Salz davon, wobei eine Verbindung der Formel (I) hergestellt wird, in der X eine Hydroxyiminogruppe darstellt.

**42.** Verfahren nach Anspruch 41, wobei die Verbindung der Formel (IV) durch Umsetzen einer Verbindung der Formel (III)

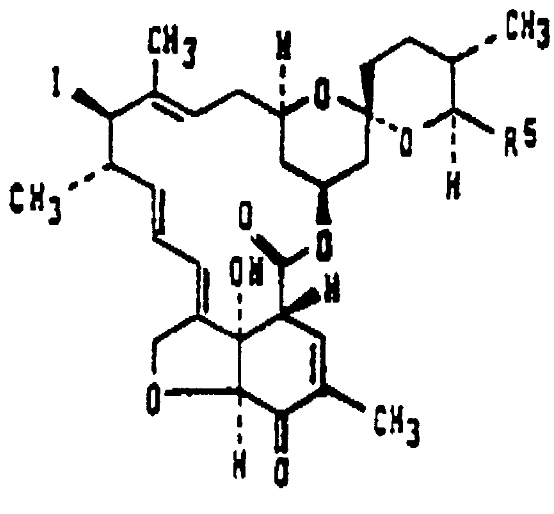

(III)

(in der $R^5$ wie in Anspruch 1 definiert ist) mit einer Verbindung der Formel (III')

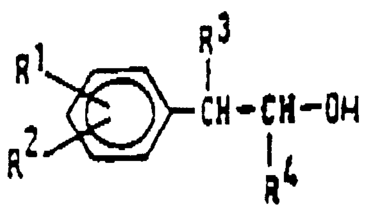

(III')

(in der $R^1$, $R^2$, $R^3$ und $R^4$ wie in Anspruch 1 definiert sind), hergestellt wird.

**Patentansprüche für folgende Vertragsstaaten : ES, GR**

**1.** Verfahren zur Herstellung einer Verbindung der Formel (I):

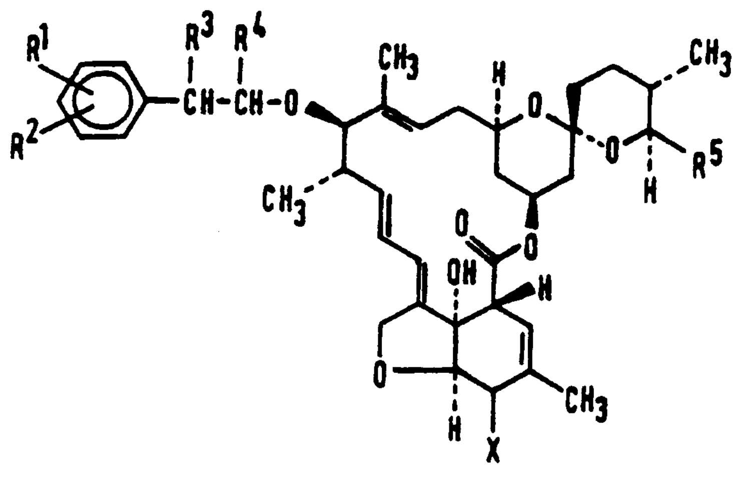

(I)

worin

$R^1$ und $R^2$ gleich oder verschieden sind und jeweils einen der folgenden Reste darstellen: ein Wasserstoffatom, ein Halogenatom, eine Cyangruppe, eine Nitrogruppe, eine $C_1$-$C_4$-Alkylgruppe, eine substituierte $C_1$-$C_4$-Alkylgruppe mit mindestens einem der nachstehend definierten Substituenten (a), eine $C_1$-$C_4$-Alkoxyruppe, eine $C_2$-$C_6$-Alkoxyalkoxygruppe, eine Gruppe der Formel $-(CH_2)_nNHR^9$,

worin n 0 oder die ganze Zahl 1 oder 2 darstellt und $R^9$ ein Wasserstoffatom oder eine $C_1$-$C_4$-Alkylgruppe darstellt,

eine Gruppe der Formel $-(CH_2)_nNR^9C(=O)R^6$, worin

n und $R^9$ wie vorstehend definiert sind, und $R^6$ einen der folgenden Reste darstellt: ein Wasserstoffatom, eine $C_1$-$C_4$-Alkylgruppe, eine substituierte $C_1$-$C_4$-Alkylgruppe mit mindestens einem der nachstehend definierten Substituenten (b), eine aliphatische $C_2$-$C_8$-Kohlenwasserstoffgruppe mit einer oder zwei ethylenisch ungesättigten Kohlenstoff-Kohlenstoff-Doppelbindung(en), wobei die Gruppe nicht substituiert ist oder mindestens einen der nachstehend definierten Substituenten (b) aufweist, eine $C_2$-$C_8$-Alkinylgruppe, eine substituierte $C_2$-$C_8$-Alkinylgruppe mit mindestens einem der nachstehend definierten Substituenten (b), eine $C_3$-$C_8$-Cycloalkylgruppe, eine substituierte $C_3$-$C_8$-Cycloalkylgruppe mit mindestens einem der nachstehend definierten Substituenten (c), eine carbocyclische Arylgruppe mit 6 bis 14 Ringkohlenstoffatomen, die nicht substituiert ist oder mindestens einen der nachstehend definierten Substituenten (c) aufweist, oder eine heterocyclische Gruppe mit 3 bis 6 Ringatomen, von denen mindestens eines ein Stickstoff- und/oder Sauerstoff- und/oder Schwefel-Heteroatom ist, wobei die heterocyclische Gruppe monocyclisch ist oder mit einem oder zwei Benzolring(en) kondensiert ist und nicht substituiert ist oder mindestens einen der nachstehend definierten Substituenten (c) aufweist,

eine Gruppe der Formel $-(CH_2)_nNR^9COCOR^6$,

worin n, $R^6$ und $R^9$ wie vorstehend definiert sind,

eine Gruppe der Formel $-(CH_2)_nR^9COCOOR^7$,

worin n und $R^9$ wie vorstehend definiert sind und $R^7$ eine $C_1$-$C_4$-Alkylgruppe, eine $C_3$-$C_8$-Cycloalkylgruppe oder eine Aralkylgruppe wie sie nachstehend definiert ist, bedeutet, eine Gruppe der Formel $-(CH_2)_nNR^9CHR^6NHCOR^6$

worin n, $R^6$ und $R^9$ wie vorstehend definiert sind,

eine Gruppe der Formel $-(CH_2)_nNR^9CHR^6NHCONHR^6$,

worin n, $R^6$ und $R^9$ wie vorstehend definiert sind, eine Gruppe der Formel $-(CH_2)_nNR^9CHR^6NHCOOR^7$,

worin n, $R^6$, $R^7$ und $R^9$ wie vorstehend definiert sind,

eine Gruppe der Formel $-(CH_2)_nNR^9C(=Y)YR^6$,

worin n, $R^6$ und $R^9$ wie vorstehend definiert sind und die zwei Symbole Y gleich oder verschieden sind und jeweils ein Sauerstoffatom oder ein Schwefelatom darstellen,

Gruppen der Formel $-(CH_2)_nNR^9C(=Y)NR^{6'}R^{6'}$,

worin n, Y und $R^9$ wie vorstehend definiert sind und die zwei Symbole $R^{6'}$ gleich oder verschieden sind und jeweils eine der durch $R^6$ dargestellten Gruppen oder Atome darstellen, oder die zwei zusammen mit dem Stickstoffatom, an das sie gebunden sind, eine heterocyclische Gruppe mit 3 bis 7 Ringatomen bilden, von denen eines das Stickstoffatom ist und 0 oder 1 ein zusätzliches Stickstoff- und/oder Sauerstoff- und/oder Schwefel-Heteroatom ist,

eine Gruppe der Formel $-(CH_2)_nNR^9C(=Y)NR^{6''}NR^{6''}R^{6''}$,

worin n, Y und $R^9$ wie vorstehend definiert sind und die Symbole $R^{6''}$ jeweils gleich oder verschieden sind und jeweils eine der durch $R^6$ dargestellten Gruppen oder Atome darstellen, oder beliebige zwei der Symbole $R^{6''}$, zusammen mit dem Stickstoffatom, an das sie gebunden sind, eine heterocyclische Gruppe mit 3 bis 7 Ringatomen bilden, von denen eines oder zwei das Stickstoffatom ist oder die Stickstoffatome sind und 0 oder 1 ein zusätzliches Stickstoff- und/oder Sauerstoff-und/oder Schwefel-Heteroatom ist,

eine Gruppe der Formel $-(CH_2)_nNR^9C(=Y)NR^6NHZ$

worin n, Y, $R^6$ und $R^9$ wie vorstehend definiert sind und Z einen der folgenden Reste darstellt:

eine Gruppe der Formel $-COOR^7$, worin $R^7$ wie vorstehend definiert ist,

eine Gruppe der Formel $-COR^6$, worin $R^6$ wie vorstehend definiert ist, oder

eine Gruppe der Formel $-SO_2R^6$, worin $R^6$ wie vorstehend definiert ist,

eine Gruppe der Formel $-(CH_2)_nNR^9C(=NR^{10})NHR^{10}$

worin n und $R^9$ wie vorstehend definiert sind und die zwei Symbole $R^{10}$ gleich oder verschieden sind und jeweils eine der durch $R^6$ dargestellten Gruppen oder Atome oder eine Cyangruppe, eine Nitrogruppe, eine Gruppe der Formel $-COOR^7$, worin $R^7$ wie vorstehend definiert ist, oder eine Gruppe der Formel $-COR^6$, worin $R^6$ wie vorstehend definiert ist, darstellen,

eine Gruppe der Formel $-(CH_2)_nNR^9C(=NR^{10})R^6$,

worin n, $R^6$, $R^9$ und $R^{10}$ wie vorstehend definiert sind,

eine Gruppe der Formel $-(CH_2)_nNR^9SO_mR^6$,

worin n, $R^6$ und $R^9$ wie vorstehend definiert sind und m 1 oder 2 ist,

eine Gruppe der Formel $-CONHR^6$,

worin $R^6$ wie vorstehend definiert ist, und

eine Gruppe der Formel $-COOR^7$,

worin $R^7$ wie vorstehend definiert ist,

$R^3$ und $R^4$ gleich oder verschieden sind und jeweils ein Wasserstoffatom, eine $C_1$-$C_4$-Alkylgruppe oder eine $C_1$-$C_4$-Alkoxygruppe darstellen,

$R^5$ eine Methylgruppe, eine Ethylgruppe, eine Isopropylgruppe oder eine sec.-Butylgruppe darstellt, und

X eine Hydroxygruppe, eine $C_1$-$C_5$-Alkanoyloxygruppe, eine substituierte $C_1$-$C_5$-Alkanoyloxygruppe mit mindestens einem der nachstehend definierten Substituenten (d) oder eine Hydroxyiminogruppe darstellt,

wobei die Aralkylgruppen in dem Alkylteil 1 bis 4 Kohlenstoffatome und in dem Arylteil, welcher eine carbocyclische Arylgruppe darstellt, die nicht substituiert ist, oder mindestens einen der nachstehend definierten Substituenten (c) aufweist, 6 bis 10 Ringatome aufweisen,

Substituenten (a):

Halogenatome, $C_1$-$C_4$-Alkoxygruppen, $C_1$-$C_4$-Alkylthiogruppen und $C_1$-$C_5$-Alkanoyloxygruppen,

Substituenten (b):

$C_3$-$C_8$-Cycloalkylgruppen, $C_1$-$C_4$-Alkoxygruppen, $C_1$-$C_4$-Alkylthiogruppen, $C_2$-$C_5$-Cyanalkylthiogruppen, $C_2$-$C_5$-Alkoxycarbonylgruppen, Halogenatome, Cyangruppen, Nitrogruppen, Aminogruppen, carbocyclische Arylgruppen mit 6 bis 10 Kohlenstoffatomen, die nicht substituiert sind oder mindestens einen der nachstehend definierten Substituenten (c) aufweisen, aromatische heterocyclische Gruppen mit 5 bis 8 Ringatomen, von denen 1 bis 4 Stickstoff- und/oder Sauerstoff- und/oder Schwefel-Heteroatome sind, wobei die heterocyclische Gruppe monocyclisch ist oder entweder an einen Benzolring oder an eine heterocyclische Gruppe mit 5 bis 6 Ringatomen, von denen 1 bis 3 Stickstoffheteroatome sind, kondensiert ist und nicht substituiert ist oder mindestens einen der nachstehend definierten Substituenten (c) aufweist, und Aryloxy- und Arylthiogruppen, worin der Arylteil 6 bis 10 Kohlenstoffatome aufweist und nicht substituiert ist oder mindestens einen der nachstehend definierten Substituenten (c) aufweist,

Substituenten (c) :

$C_1$-$C_4$-Alkylgruppen, $C_1$-$C_4$-Alkoxygruppen, $C_1$-$C_4$-Alkylthiogruppen, $C_1$-$C_5$-Alkanoyloxygruppen, $C_2$-$C_5$-Alkoxycarbonylgruppen, Halogenatome, Cyangruppen, Nitrogruppen, Aminogruppen, Mono- und Dialkylaminogruppen, worin der oder jeder Alkylteil $C_1$-$C_4$ ist, Carbamoylgruppen, Mono- und Dialkylcarbamoylgruppen, worin der oder jeder Alkylteil $C_1$-$C_4$ ist und $C_1$-$C_5$-Alkanoylaminogruppen,

Substituenten (d):

Halogenatome, $C_1$-$C_4$-Alkoxygruppen, $C_2$-$C_5$-Alkoxycarbonylgruppen und Carboxygruppen,

oder eines Salzes davon,

welches die folgenden Schritte umfaßt:

Umsetzen einer Verbindung der Formel (IV):

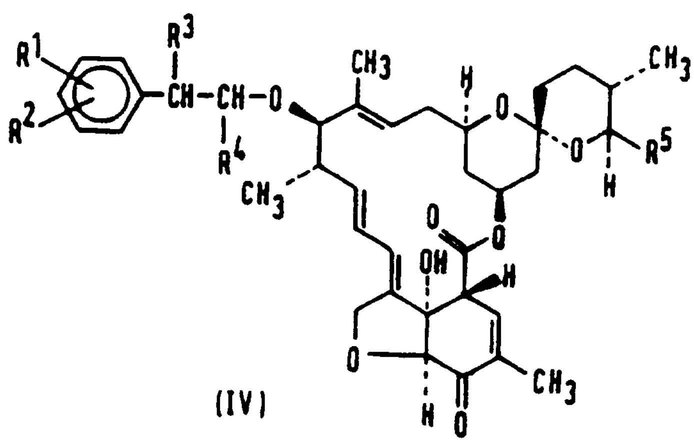

(IV)

(worin $R^1$, $R^2$, $R^3$, $R^4$ und $R^5$ wie vorstehend definiert sind) mit einem Reduktionsmittel, wobei eine Verbindung der Formel (I) hergestellt wird, in der X eine Hydroxygruppe darstellt, und, wenn erforderlich, Acylieren der Verbindung, in der X eine Hydroxygruppe darstellt, wobei eine Verbindung der Formel (I) hergestellt wird, in der X die Alkanoyloxygruppe darstellt, oder Umsetzen der Verbindung der Formel (IV) mit Hydroxylamin

oder mit einem Salz davon, wobei eine Verbindung der Formel (I) hergestellt wird, in der X eine

Hydroxyiminogruppe darstellt.

**2.** Verfahren nach Anspruch 1, wobei die Verbindung der Formel (IV) durch Umsetzen einer Verbindung der Formel (III)

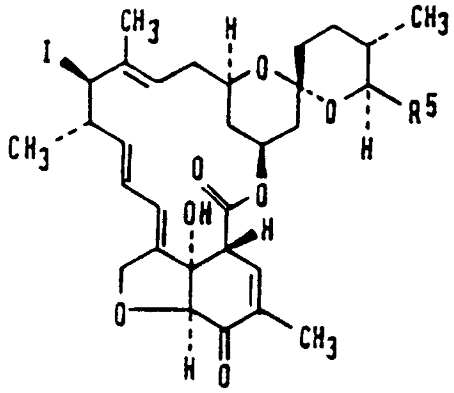

(III)

(worin $R^5$ wie in Anspruch 1 definiert ist) mit einer Verbindung der Formel (III'):

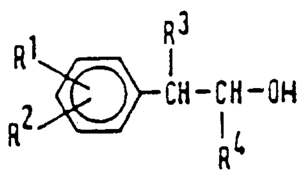

(III')

(worin $R^1$, $R^2$, $R^3$ und $R^4$ wie in Anspruch 1 definiert sind), hergestellt wird.

**3.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin :

$R^1$ ein Wasserstoffatom darstellt, und

$R^2$ ein Wasserstoffatom, eine $C_1$-$C_3$-Alkylgruppe, eine $C_1$-$C_3$-Alkoxygruppe, ein Fluor- oder ein Chloratom, eine Nitrogruppe oder eine Aminogruppe darstellt.

**4.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ ein Wasserstoffatom darstellt, und

$R^2$ eine Gruppe der Formel $-(CH_2)_nNR^{9a}COR^{6a}$ darstellt,

worin n 0 ist, $R^{9a}$ ein Wasserstoffatom oder eine Methylgruppe darstellt und $R^{6a}$ darstellt: eine $C_1$-$C_4$-Alkylgruppe, eine $C_3$-$C_5$-Cycloalkylgruppe, eine $C_1$-$C_3$-Alkylgruppe, die mit einem Halogen-, einem Cyan-, einem $C_1$-$C_3$-Alkoxy-, einem $C_1$-$C_3$-Alkylthio-, einem Cyanmethylthio- oder einem Phenoxy-Substituenten substituiert ist, eine Alkenylgruppe, eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, eine Pyridylgruppe, eine Pyrimidylgruppe, eine Pyrazylgruppe, eine Furylgruppe oder eine Thienylgruppe.

**5.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ ein Wasserstoffatom darstellt, und

$R^2$ eine Gruppe der Formel $-(CH_2)_nNR^{9a}COCOR^{6b}$ darstellt,

worin n 0 ist, $R^{9a}$ ein Wasserstoffatom oder eine Methylgruppe darstellt und $R^{6b}$ ein Wasserstoffatom, eine $C_1$-$C_4$-Alkylgruppe, eine $C_3$-$C_5$-Cycloalkylgruppe, eine Alkenylgruppe, eine nicht substituierte Phenylgruppe oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**6.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:
$R^1$ ein Wasserstoffatom darstellt, und
$R^2$ eine Gruppe der Formel $-(CH_2)_nNR^{9a}C(=Y)YR^{6c}$ darstellt,
worin n 0 ist, $R^{9a}$ ein Wasserstoffatom oder eine Methylgruppe darstellt, beide Y Sauerstoffatome sind und $R^{6c}$ eine $C_1$-$C_4$-Alkylgruppe, eine $C_1$-$C_4$-Alkylgruppe, die mit einem Halogen- oder einem $C_1$-$C_3$-Alkoxy-Substituenten substituiert ist, eine Alkenylgruppe, eine Benzylgruppe, eine Methoxybenzylgruppe, eine Nitrobenzylgruppe, eine Furfurylgruppe, eine Thienylgruppe oder eine Phenylgruppe darstellt.

**7.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:
$R^1$ ein Wasserstoffatom darstellt, und
$R^2$ eine Gruppe der Formel $-(CH_2)_nNR^{9a}C(=Y)NR^{6d}R^{6e}$ darstellt,
worin n 0 ist, $R^{9a}$ ein Wasserstoffatom oder eine Methylgruppe darstellt, Y ein Sauerstoffatom oder ein Schwefelatom darstellt und $R^{6d}$ und $R^{6e}$ gleich oder verschieden sind und jeweils eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, oder $R^{6d}$ und $R^{6e}$ zusammen mit dem Stickstoffatom, an das sie gebunden sind, eine Piperidino-, Piperazino-, Morpholino-, Pyrrolidino- oder Aziridinogruppe darstellen.

**8.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:
$R^1$ ein Wasserstoffatom darstellt, und
$R^2$ eine Gruppe der Formel $-(CH_2)_nNR^{9a}C(=Y)NR^{6f}NR^{6g}R^{6h}$ darstellt,
worin n 0 ist, $R^{9a}$ ein Wasserstoffatom oder eine Methylgruppe darstellt, Y ein Sauerstoffatom oder ein Schwefelatom darstellt und $R^{6f}$, $R^{6g}$ und $R^{6h}$ gleich oder verschieden sind und jeweils einen der folgenden Reste darstellen: ein Wasserstoffatom, eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, oder $R^{6g}$ und $R^{6h}$ zusammen mit dem Stickstoffatom, an das sie gebunden sind, eine Piperidino-, Piperazino-, Morpholino-, Pyrrolidino- oder Aziridinogruppe darstellen, oder $R^{6f}$ und $R^{6g}$ zusammen mit den Stickstoffatomen, an die sie gebunden sind, eine Pyrazolidinyl- oder Tetrahydropyridazinylgruppe darstellen.

**9.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:
$R^1$ ein Wasserstoffatom darstellt, und
$R^2$ eine Gruppe der Formel $-(CH_2)_nNR^{9a}C(=Y)NR^{6j}NHZ$ darstellt,
worin n 0 ist, $R^{9a}$ ein Wasserstoffatom oder eine Methylgruppe darstellt, Y ein Sauerstoffatom oder ein Schwefelatom darstellt, $R^{6j}$ ein Wasserstoffatom, eine $C_1$-$C_4$-Alkylgruppe oder eine $C_3$-$C_6$-Cycloalkylgruppe darstellt und Z
eine Gruppe der Formel $-COOR^{7a}$ darstellt,
worin $R^{7a}$ eine $C_1$-$C_4$-Alkylgruppe, eine $C_3$-$C_6$-Cycloalkylgruppe oder eine Benzylgruppe darstellt,
eine Gruppe der Formel $-COR^{6k}$ darstellt,
worin $R^{6k}$ eine $C_1$-$C_4$-Alkylgruppe, eine $C_3$-$C_6$-Cycloalkylgruppe, eine nicht substituierte Phenylgruppe oder eine Phenylgruppe, die mit einem $C_1$-$C_3$-Alkyl-, einem $C_1$-$C_3$-Alkoxy-, einem Halogen, oder einem Nitro-Substituenten substituiert ist, oder
eine Gruppe der Formel $-SO_2R^{6m}$ darstellt,
worin $R^{6m}$ eine $C_1$-$C_4$-Alkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**10.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:
$R^1$ ein Wasserstoffatom darstellt, und
$R^2$ eine Gruppe der Formel $-(CH_2)_nNR^{9a}C(=NR^{10a})NHR^{10b}$ darstellt,
worin n 0 ist, $R^{9a}$ ein Wasserstoffatom oder eine Methylgruppe darstellt, und $R^{10a}$ und $R^{10b}$ gleich oder verschieden sind und jeweils die folgenden Reste darstellen: ein Wasserstoffatom, eine $C_1$-$C_4$-

Alkylgruppe, eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, eine Gruppe der Formel -$COOR^{7b}$, worin $R^{7b}$ eine $C_1$-$C_4$-Alkylgruppe, eine $C_3$-$C_6$-Cycloalkylgruppe oder eine Benzylgruppe darstellt, oder eine Gruppe der Formel -$COR^{6n}$, worin $R^{6n}$ eine $C_1$-$C_4$-Alkylgruppe, eine $C_3$-$C_6$-Cycloalkylgruppe, eine nicht substituierte Phenylgruppe oder eine Phenylgruppe, die mit einem $C_1$-$C_3$-Alkyl-, einem $C_1$-$C_3$-Alkoxy-, einem Halogen, oder einem Nitro-Substi tuenten substituiert ist, darstellt.

**11.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ ein Wasserstoffatom darstellt, und

$R^2$ eine Gruppe der Formel -$(CH_2)_nNR^{9a}C(=NR^{10c})R^{6p}$ darstellt,

worin n 0 ist, $R^{9a}$ ein Wasserstoffatom oder eine Methylgruppe darstellt, $R^{10c}$ einen der folgenden Reste darstellt: ein Wasserstoffatom, eine $C_1$-$C_4$-Alkylgruppe,eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, eine Gruppe der Formel -$COOR^{7c}$, worin $R^{7c}$ eine $C_1$-$C_4$-Alkylgruppe, eine $C_3$-$C_6$-Cycloalkylgruppe oder eine Benzylgruppe darstellt, oder eine Gruppe der Formel -$COR^{6q}$, worin $R^{6q}$ eine $C_1$-$C_4$-Alkylgruppe, eine $C_3$-$C_6$-Cycloalkylgruppe, eine nicht substituierte Phenylgruppe oder eine Phenylgruppe, die mit einem $C_1$-$C_3$-Alkyl-, einem $C_1$-$C_3$-Alkoxy-, einem Halogen-, oder einem Nitro-Substituenten substituiert ist, und $R^{6p}$ eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**12.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ ein Wasserstoffatom darstellt, und

$R^2$ eine Gruppe der Formel -$(CH_2)_nNR^{9a}SO_mR^{6r}$ darstellt,

worin n 0 ist, $R^{9a}$ ein Wasserstoffatom oder eine Methylgruppe darstellt, m 1 oder 2 ist und $R^{6r}$ eine $C_1$-$C_4$-Alkylgruppe, eine $C_2$-$C_4$-Cyanalkylgruppe, eine nicht substituierte Phenylgruppe, eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**13.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ ein Wasserstoffatom darstellt, und

$R^2$ eine Gruppe der Formel -$CONHR^{6s}$ darstellt,

worin $R^{6s}$ eine $C_1$-$C_4$-Alkylgruppe, eine $C_3$-$C_6$-Cycloalkylgruppe, eine nicht substituierte Phenylgruppe, oder eine Phenylgruppe, die mit einem $C_1$-$C_3$-Alkyl-, einem $C_1$-$C_3$-Alkoxy-, einem Halogen- oder einem Nitro-Substituenten substituiert ist, darstellt.

**14.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ ein Wasserstoffatom darstellt, und

$R^2$ eine Gruppe der Formel -$COOR^{7d}$ darstellt,

worin $R^{7d}$ eine Methyl-, Ethyl-, oder Benzylgruppe darstellt.

**15.** Verfahren nach Anspruch 1 oder Anspruch 2, wobei die Reagenzien und Reaktionsbedingungen so gewählt werden, daß eine Verbindung der Formel (I) oder ein Salz davon hergestellt wird, worin:

$R^1$ eine Methoxygruppe darstellt, und

$R^2$ eine $C_1$-$C_3$-Alkoxygruppe oder eine $C_2$-$C_4$-Alkoxyalkoxygruppe darstellt.

**16.** Verfahren nach einem der vorhergehenden Ansprüche, wobei die Reagenzien und Reaktionsbedingungen so gewählt werden, daß eine Verbindung der Formel (I) oder ein Salz davon hergestellt wird, worin $R^2$ in der para-Position des Benzolrings gebunden ist.

**17.** Verfahren nach einem der vorhergehenden Ansprüche, wobei die Reagenzien und Reaktionsbedingungen so gewählt werden, daß eine Verbindung der Formel (I) oder ein Salz davon hergestellt wird, worin $R^5$ eine Ethylgruppe darstellt.

**18.** Verfahren nach einem der Ansprüch 1 bis 15, wobei die Reagenzien und Reaktionsbedingungen so gewählt werden, daß eine Zusammensetzung hergestellt wird, die ein Gemisch von Verbindungen der Formel (I) oder Salze davon umfaßt, worin in einer der Verbindungen $R^5$ eine Ethylgruppe darstellt und in der anderen der Verbindungen $R^5$ eine Methylgruppe darstellt.

**19.** Verfahren nach einem der vorhergehenden Ansprüche, wobei die Reagenzien und Reaktionsbedingungen so gewählt werden, daß eine Verbindung der Formel (I) oder ein Salz davon hergestellt wird, in der $R^3$ und $R^4$ Wasserstoffatome sind.

**20.** Verfahren nach einem der vorhergehenden Ansprüche, wobei die Reagenzien und Reaktionsbedingungen so gewählt werden, daß eine Verbindung der Formel (I) oder ein Salz davon hergestellt wird, in der X eine Hydroxygruppe darstellt.

**21.** Verfahren nach einem der vorhergehenden Ansprüche, wobei die Reagenzien und Reaktionsbedingungen so gewählt werden, daß die folgenden Verbindungen oder ein Salz davon hergestellt werden:
13-[2-(4-Acetamidophenyl)ethoxy]milbemycin $A_4$,
13-[2-(4-Cyanacetamidophenyl)ethoxy]milbemycin $A_4$,
13-{2-[4-(2-Cyanpropionamido)phenyl]ethoxy}milbemycin $A_4$,
13-[2-(4-Methoxyacetamidophenyl)ethoxy]milbemycin $A_4$,
13-{2-[4-(Cyclopropylcarbonylamino)phenyl]ethoxy}milbemycin $A_4$,
13-{2-[4-(Cyclobutylcarbonylamino)phenyl]ethoxy}milbemycin $A_4$,
13-{2-[4-(4-Cyanbenzamido)phenyl]ethoxy}milbemycin $A_4$,
13-[2-(4-Methoxycarbonylaminophenyl)ethoxy]milbemycin $A_4$,
13-[2-(4-Vinyloxycarbonylaminophenyl)ethoxy]milbemycin $A_4$,
13-{2-[4-(3-Methylureido)phenyl]ethoxy}milbemycin $A_4$,
13-{2-[4-(3-Phenylureido)phenyl]ethoxy}milbemycin $A_4$,
13-{2-[4-(3-Cyclohexylureido)phenyl]ethoxy}milbemycin $A_4$,
13-[2-(4-Methansulphonylaminophenyl)ethoxy]milbemycin $A_4$,
13-[2-(4-Ethansulphonylaminophenyl)ethoxy]milbemycin $A_4$,
13-{2-[4-(3,3-Dimethylcarbazoylamino)phenyl]ethoxy}milbemycin $A_4$,
13-{2-[4-(3-o-Fluorphenylureido)phenyl]ethoxy}milbemycin $A_4$,
13-{2-[4-(3-p-Fluorphenylureido)phenyl]ethoxy}milbemycin $A_4$,
13-{2-[4-(3-p-Methoxyphenylureido)phenyl]ethoxy}milbemycin $A_4$,

**22.** Verfahren zur Herstellung einer anthelmintischen, mitiziden und insektiziden Zusammensetzung, bei dem eine anthelmintische, mitizide und insektizide Verbindung mit einem pharmazeutisch, landwirtschaftlich, veterinärmedizinisch oder im Gartenbau geeigneten Träger oder Verdünnungsmittel vermischt wird, wobei die Verbindung mindestens eine der nach einem der vorhergehenden Ansprüche hergestellten Verbindungen ist.

**23.** Verwendung von mindestens einer Verbindung der Formel (I) nach einem der Ansprüche 1 bis 21 oder einer Zusammensetzung nach Anspruch 22 zur Herstellung eines Arzneimittels zur Behandlung von Infektionen durch Würmer, Milben oder Insekten.

**Revendications**

**Revendications pour les Etats contractants suivants : GB, DE, FR, IT, CH, BE, NL, SE, LU, AT**

**1.** Composés de formule (I) :

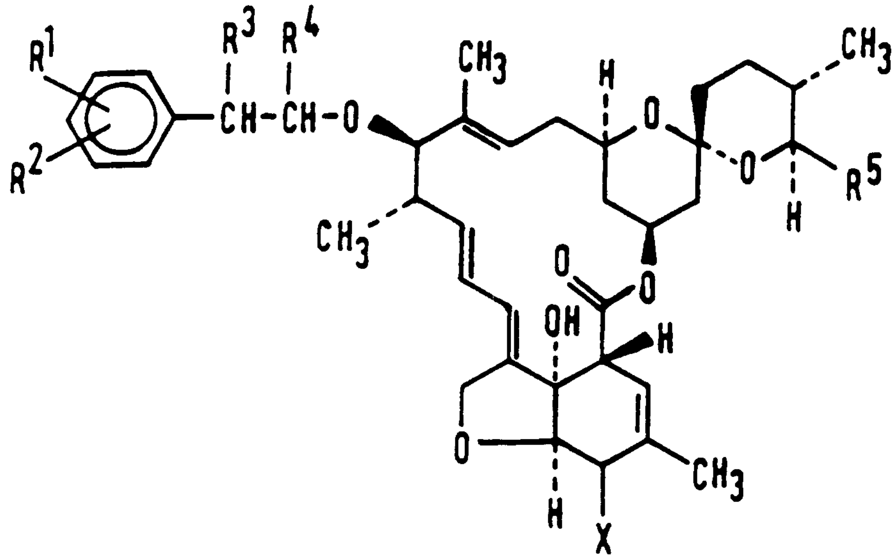

(I)

dans laquelle :

$R^1$ et $R^2$ sont identiques ou différents et représentent chacun : un atome d'hydrogène ; un atome d'halogène ; un groupe cyano ; un groupe nitro ; un groupe alkyle en $C_1$-$C_4$ ; un groupe alkyle en $C_1$-$C_4$ substitué ayant au moins un des substituants (a) définis ci-après ;

un groupe alcoxy en $C_1$-$C_4$ ; un groupe alcoxyalcoxy en $C_2$-$C_6$ ; un groupe de formule $-(CH_2)_nNHR^9$,

dans laquelle : n représente 0 ou l'entier 1 ou 2, et $R^9$ représente un atome d'hydrogène ou un groupe alkyle en $C_1$-$C_4$ ;

un groupe de formule $-(CH_2)_nNR^9C(=O)R^6$,

dans laquelle :

n et $R^9$ sont tels que définis ci-dessus, et $R^6$ représente : un atome d'hydrogène ; un groupe alkyle en $C_1$-$C_4$ ; un groupe alkyle en $C_1$-$C_4$ substitué ayant au moins un des substituants (b) définis ci-après ; un groupe hydrocarboné aliphatique en $C_2$-$C_8$ ayant une ou deux doubles liaisons carbone-carbone à insaturation éthylénique, ledit groupe étant non substitué ou ayant au moins un des substituants (b) définis ci-après ; un groupe alcynyle en $C_2$-$C_8$ ; un groupe alcynyle en $C_2$-$C_8$ substitué ayant au moins un des substituants (b) définis ci-après ; un groupe cycloalkyle en $C_3$-$C_8$ ; un groupe cycloalkyle en $C_3$-$C_8$ substitué ayant au moins un des substituants (c) définis ci-après ; un groupe aryle carbocyclique ayant de 6 à 14 atomes de carbone formant le cycle et étant non substitué ou ayant au moins un des substituants (c) définis ci-après ; ou un groupe hétérocyclique ayant de 3 à 6 atomes formant le cycle dont au moins un est un hétéroatome d'azote et/ou d'oxygène et/ou de soufre, ledit groupe hétérocyclique étant monocyclique ou étant condensé à un ou à deux cycles benzène et étant non substitué ou ayant au moins un des substituants (c) définis ci-après ;

un groupe de formule $-(CH_2)_nNR^9COCOR^6$

dans laquelle n, $R^6$ et $R^9$ sont tels que définis ci-dessus ;

un groupe de formule $-(CH_2)_nR^9COCOOR^7$

dans laquelle n et $R^9$ sont tels que définis ci-dessus et $R^7$ représente un groupe alkyle en $C_1$-$C_4$, un groupe cycloalkyle en $C_3$-$C_8$ ou un groupe aralkyle tel que défini ci-après ;

un groupe de formule $-(CH_2)_nNR^9CHR^6NHCOR^6$

dans laquelle n, $R^6$ et $R^9$ sont tels que définis ci-dessus ;

un groupe de formule $-(CH_2)_nNR^9CHR^6NHCONHR^6$

dans laquelle n, $R^6$ et $R^9$ sont tels que définis ci-dessus ;

un groupe de formule $-(CH_2)_nNR^9CHR^6NHCOOR^7$

dans laquelle n, $R^6$, $R^7$ et $R^9$ sont tels que définis ci-dessus ;

un groupe de formule $-(CH_2)_nNR^9C(=Y)YR^6$

dans laquelle n, $R^6$ et $R^9$ sont tels que définis ci-dessus et les deux symboles Y sont identiques ou différents et représentent chacun un atome d'oxygène ou un atome de soufre ;

un groupe de formule $-(CH_2)_nNR^9C(=Y)NR^{6'}R^{6'}$

dans laquelle n, Y et $R^9$ sont tels que définis ci-dessus et les deux symboles $R^{6'}$ sont identiques ou différents et représentent chacun un des groupes ou atomes représentés par $R^6$ ou, tous deux, avec l'atome d'azote auquel ils sont fixés, forment un groupe hétérocyclique ayant de 3 à 7 atomes formant le cycle dont un est ledit atome d'azote et 0 ou 1 est un hétéroatome additionnel d'azote et/ou d'oxygène et/ou de soufre ;

un groupe de formule $-(CH_2)_nNR^9C(=Y)NR^{6''}NR^{6''}R^{6''}$

dans laquelle n, Y et $R^9$ sont tels que définis ci-dessus et les symboles $R^{6''}$ sont identiques ou différents et représentent chacun un des groupes ou atomes représentés par $R^6$, ou deux quelconques des symboles $R^{6''}$, avec l'atome d'azote auquel ils sont chacun fixés, forment un groupe hétérocyclique ayant de 3 à 7 atomes formant le cycle dont un ou les deux est le ou sont lesdits atomes d'azote et 0 ou 1 est un hétéroatome additionnel d'azote et/ou d'oxygène et/ou de soufre ;

un groupe de formule $-(CH_2)_nNR^9C(=Y)NR^6NHZ$

dans laquelle n, Y, $R^6$ et $R^9$ sont tels que définis ci-dessus et Z représente

un groupe de formule $-COOR^7$, dans laquelle $R^7$ est tel que défini ci-dessus,

un groupe de formule $-COR^6$, dans laquelle $R^6$ est tel que défini ci-dessus, ou

un groupe de formule $-SO_2R^6$, dans laquelle $R^6$ est tel que défini ci-dessus ;

un groupe de formule $-(CH_2)_nNR^9C(=NR^{10})NHR^{10}$

dans laquelle n et $R^9$ sont tels que définis ci-dessus et les deux symboles $R^{10}$ sont identiques ou différents et représentent chacun un des groupes ou atomes représentés par $R^6$ ou un groupe cyano, un groupe nitro, un groupe de formule $-COOR^7$, dans laquelle $R^7$ est tel que défini ci-dessus, ou un groupe de formule $-COR^6$, dans laquelle $R^6$ est tel que défini ci-dessus ;

un groupe de formule $-(CH_2)_nNR^9C(=NR^{10})R^6$

dans laquelle n, $R^6$, $R^9$ et $R^{10}$ sont tels que définis ci-dessus ; un groupe de formule $-(CH_2)_nNR^9SO_mR^6$

dans laquelle n, $R^6$ et $R^9$ sont tels que définis ci-dessus et m est 1 ou 2 ;

un groupe de formule $-CONHR^6$

dans laquelle $R^6$ est tel que défini ci-dessus ; et

un groupe de formule $-COOR^7$

dans laquelle $R^7$ est tel que défini ci-dessus ;

$R^3$ et $R^4$ sont identiques ou différents et représentent chacun un atome d'hydrogène, un groupe alkyle en $C_1$-$C_4$ ou un groupe alcoxy en $C_1$-$C_4$ ; $R^5$ représente un groupe méthyle, un groupe éthyle, un groupe

isopropyle ou un groupe sec-butyle ; et

X représente un groupe hydroxy, un groupe alcanoyloxy en $C_1$-$C_5$, un groupe alcanoyloxy en $C_1$-$C_5$ substitué ayant au moins un des substituants (d) définis ci-après, ou un groupe hydroxyimino ; lesdits groupes aralkyle ont de 1 à 4 atomes de carbone dans la partie alkyle et de 6 à 10 atomes formant le cycle dans la partie aryle, qui est un groupe aryle carbocyclique qui est non substitué ou qui a au moins un des substituants (c) définis ci-après ;

<u>substituants (a)</u> :

atomes d'halogène, groupes alcoxy en $C_1$-$C_4$, groupes alkylthio en $C_1$-$C_4$ et groupes alcanoyloxy en $C_1$-$C_5$ ;

<u>substituants (b)</u> :

groupes cycloalkyle en $C_3$-$C_8$ ; groupes alcoxy en $C_1$-$C_4$ ; groupes alkylthio en $C_1$-$C_4$ ; groupes cyanoalkylthio en $C_2$-$C_5$ ; groupes alcoxycarbonyle en $C_2$-$C_5$ ; atomes d'halogène ; groupes cyano ; groupes nitro ; groupes amino ; groupes aryle carbocycliques ayant de 6 à 10 atomes de carbone et étant non substitués ou ayant au moins un des substituants (c) définis ci-après ; groupes hétérocycliques aromatiques ayant de 5 à 8 atomes formant le cycle dont 1 à 4 sont des hétéroatomes d'azote et/ou d'oxygène et/ou de soufre, ledit groupe hétérocyclique étant monocyclique ou étant condensé soit à un cycle benzène, soit à un groupe hétérocyclique qui a 5 ou 6 atomes formant le cycle dont 1 à 3 sont des hétéroatomes d'azote, et étant non substitué ou ayant au moins un des substituants (c) définis ci-après ; et groupes aryloxy et arylthio dont la partie aryle a de 6 à 10 atomes de carbone et est non substituée ou a au moins un des substituants (c) définis ci-après ;

<u>substituants (c)</u> :

groupes alkyle en $C_1$-$C_4$, groupes alcoxy en $C_1$-$C_4$, groupes alkylthio en $C_1$-$C_4$, groupes alcanoyloxy en $C_1$-$C_5$, groupes alcoxycarbonyle en $C_2$-$C_5$, atomes d'halogène, groupes cyano, groupes nitro,

groupes amino, groupes mono- et dialkylamino dont la ou chaque partie alkyle est en $C_1$-$C_4$, groupes carbamoyle, groupes mono- et dialkylcarbamoyle dont la ou chaque partie alkyle est en $C_1$-$C_4$, et groupes alcanoylamino en $C_1$-$C_5$ ;
substituants (d) :
atomes d'halogène, groupes alcoxy en $C_1$-$C_4$, groupes alcoxycarbonyle en $C_2$-$C_5$ et groupes carboxy ;
et leurs sels.

**2.** Composé selon la revendication 1, où :
$R^1$ représente un atome d'hydrogène ; et
$R^2$ représente un atome d'hydrogène, un groupe alkyle en $C_1$-$C_3$, un groupe alcoxy en $C_1$-$C_3$, un atome de fluor ou de chlore, un groupe nitro ou un groupe amino.

**3.** Composé selon la revendication 1, où :
$R^1$ représente un atome d'hydrogène ; et
$R^2$ représente un groupe de formule $-(CH_2)_nNR^{9a}COR^{6a}$
dans laquelle n est 0, $R^{9a}$ représente un atome d'hydrogène ou un groupe méthyle et $R^{6a}$ représente : un groupe alkyle en $C_1$-$C_4$ ; un groupe cycloalkyle en $C_3$-$C_5$ ; un groupe alkyle en $C_1$-$C_3$ substitué avec un substituant halogène, cyano, alcoxy en $C_1$-$C_3$, alkylthio en $C_1$-$C_3$, cyanométhylthio ou phénoxy ; un groupe alcényle ; un groupe phényle non substitué ; un groupe phényle substitué avec un substituant alkyle en $C_1$-$C_3$, alcoxy en $C_1$-$C_3$, halogène ou nitro ; un groupe pyridyle ; un groupe pyrimidyle ; un groupe pyrazyle ; un groupe furyle ; ou un groupe thiényle.

**4.** Composé selon la revendication 1, où :
$R^1$ représente un atome d'hydrogène ; et
$R^2$ représente un groupe de formule $-(CH_2)_nNR^{9a}COCOR^{6b}$
dans laquelle n est 0, $R^{9a}$ représente un atome d'hydrogène ou un groupe méthyle et $R^{6b}$ représente un atome d'hydrogène, un groupe alkyle en $C_1$-$C_4$, un groupe cycloalkyle en $C_3$-$C_5$, un groupe alcényle, un groupe phényle non substitué ou un groupe phényle substitué avec un substituant alkyle en $C_1$-$C_3$, alcoxy en $C_1$-$C_3$, halogène ou nitro.

**5.** Composé selon la revendication 1, où :
$R^1$ représente un atome d'hydrogène ; et
$R^2$ représente un groupe de formule $-(CH_2)_nNR^{9a}C(=Y)YR^{6c}$
dans laquelle n est 0, $R^{9a}$ représente un atome d'hydrogène ou un groupe méthyle, les deux symboles Y sont des atomes d'oxygène, et $R^{6c}$ représente un groupe alkyle en $C_1$-$C_4$, un groupe alkyle en $C_1$-$C_4$ substitué avec un substituant halogène ou alcoxy en $C_1$-$C_3$, un groupe alcényle, un groupe benzyle, un groupe méthoxybenzyle, un groupe nitrobenzyle, un groupe furfuryle, un groupe thiényle ou un groupe phényle.

**6.** Composé selon la revendication 1, où :
$R^1$ représente un atome d'hydrogène ; et
$R^2$ représente un groupe de formule $-(CH_2)_nNR^{9a}C(=Y)NR^{6d}R^{6e}$
dans laquelle n est 0, $R^{9a}$ représente un atome d'hydrogène ou un groupe méthyle, Y représente un atome d'oxygène ou un atome de soufre et $R^{6d}$ et $R^{6e}$ sont identiques ou différents et représentent chacun un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$,alcoxy en $C_1$-$C_3$, halogène ou nitro, ou $R^{6d}$ et $R^{6e}$, avec l'atome d'azote auquel ils sont fixés, représentent un groupe pipéridino, pipérazino, morpholino, pyrrolidino ou aziridino.

**7.** Composé selon la revendication 1, où :
$R^1$ représente un atome d'hydrogène ; et
$R^2$ représente un groupe de formule $-(CH_2)_nNR^{9a}C(=Y)NR^{6f}NR^{6g}R^{6h}$
dans laquelle n est 0, $R^{9a}$ représente un atome d'hydrogène ou un groupe méthyle, Y représente un atome d'oxygène ou un atome de soufre et $R^{6f}$, $R^{6g}$ et $R^{6h}$ sont identiques ou différents et représentent chacun : un atome d'hydrogène ; un groupe alkyle en $C_1$-$C_4$ ; un groupe cycloalkyle en $C_3$-$C_6$ ; un groupe phényle non substitué ; ou un groupe phényle substitué avec un substituant alkyle en $C_1$-$C_3$, alcoxy en $C_1$-$C_3$, halogène ou nitro ; ou $R^{6g}$ et $R^{6h}$, avec l'atome d'azote auquel ils sont fixés, représentent un groupe pipéridino, pipérazino, morpholino, pyrrolidino ou aziridino ; ou $R^{6f}$ et $R^{6g}$, avec

les atomes d'azote auxquels ils sont fixés, représentent un groupe pyrazolidinyle ou tétrahydropyridazinyle.

**8.** Composé selon la revendication 1, où :
$R^1$ représente un atome d'hydrogène ; et
$R^2$ représente un groupe de formule $-(CH_2)_nNR^{9a}C(=Y)NR^{6j}NHZ$
dans laquelle n est 0, $R^{9a}$ représente un atome d'hydrogène ou un groupe méthyle, Y représente un atome d'oxygène ou un atome de soufre, $R^{6j}$ représente un atome d'hydrogène, un groupe alkyle en $C_1$-$C_4$ ou un groupe cycloalkyle en $C_3$-$C_6$ ; et Z représente un groupe de formule $-COOR^{7a}$
dans laquelle $R^{7a}$ représente un groupe alkyle en $C_1$-$C_4$, un groupe cycloalkyle en $C_3$-$C_6$ ou un groupe benzyle,
un groupe de formule $-COR^{6k}$
dans laquelle $R^{6k}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro, ou
un groupe de formule $-SO_2R^{6m}$
dans laquelle $R^{6m}$ représente un groupe alkyle en $C_1$-$C_4$, un groupe phényle non substitué ou un groupe phényle substitué avec un substituant alkyle en $C_1$-$C_3$, alcoxy en $C_1$-$C_3$, halogène ou nitro.

**9.** Composé selon la revendication 1, où :
$R^1$ représente un atome d'hydrogène ; et
$R^2$ représente un groupe de formule $-(CH_2)_nNR^{9a}C(=NR^{10a})NHR^{10b}$
dans laquelle n est 0, $R^{9a}$ représente un atome d'hydrogène ou un groupe méthyle et $R^{10a}$ et $R^{10b}$ sont identiques ou différents et représentent chacun : un atome d'hydrogène ; un groupe alkyle en $C_1$-$C_4$ ; un groupe phényle non substitué ; un groupe phényle substitué avec un substituant alkyle en $C_1$-$C_3$, alcoxy en $C_1$-$C_3$, halogène ou nitro ; un groupe de formule $-COOR^{7b}$
dans laquelle $R^{7b}$ représente un groupe alkyle en $C_1$-$C_4$, un groupe cycloalkyle en $C_3$-$C_6$ ou un groupe benzyle ; ou
un groupe de formule $-COR^{6n}$
dans laquelle $R^{6n}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro.

**10.** Composé selon la revendication 1, où :
$R^1$ représente un atome d'hydrogène ; et
$R^2$ représente un groupe de formule $-(CH_2)_nNR^{9a}C(=NR^{10c})R^{6p}$
dans laquelle n est 0 ; $R^{9a}$ représente un atome d'hydrogène ou un groupe méthyle ; $R^{10c}$ représente un atome d'hydrogène, un groupe alkyle en $C_1$-$C_4$, un groupe phényle non substitué, un groupe phényle substitué avec un substituant alkyle en $C_1$-$C_3$, alcoxy en $C_1$-$C_3$, halogène ou nitro, un groupe de formule $-COOR^{7c}$
dans laquelle $R^{7c}$ représente un groupe alkyle en $C_1$-$C_4$, un groupe cycloalkyle en $C_3$-$C_6$ ou un groupe benzyle ;
ou un groupe de formule $-COR^{6q}$
dans laquelle $R^{6q}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro et
$R^{6p}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro.

**11.** Composé selon la revendication 1, où :
$R^1$ représente un atome d'hydrogène ; et
$R^2$ représente un groupe de formule $-(CH_2)_nNR^{9a}SO_mR^{6r}$
dans laquelle n est 0, $R^{9a}$ représente un atome d'hydrogène ou un groupe méthyle, m est 1 ou 2, et $R^{6r}$ représente un groupe alkyle en $C_1$-$C_4$, un groupe cyanoalkyle en $C_2$-$C_4$, un groupe phényle non substitué ou un groupe phényle substitué avec un substituant alkyle en $C_1$-$C_3$, alcoxy en $C_1$-$C_3$, halogène ou nitro.

12. Composé selon la revendication 1, où :
$R^1$ représente un atome d'hydrogène et
$R^2$ représente un groupe de formule $-CONHR^{6s}$
dans laquelle $R^{6s}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$,alcoxy en $C_1$-$C_3$, halogène ou nitro.

13. Composé selon la revendication 1, où :
$R^1$ représente un atome d'hydrogène et
$R^2$ représente un groupe de formule $-COOR^{7d}$
dans laquelle $R^{7d}$ représente un groupe méthyle, éthyle ou benzyle.

14. Composé selon la revendication 1, où :
$R^1$ représente un groupe méthoxy et
$R^2$ représente un groupe alcoxy en $C_1$-$C_3$ ou un groupe alcoxyalcoxy en $C_2$-$C_4$.

15. Composé selon l'une quelconque des revendications précédentes, où $R^2$ est fixé à la position p du cycle benzène.

16. Composé selon l'une quelconque des revendications précédentes, où $R^5$ représente un groupe éthyle.

17. Composition comprenant un mélange de composés dont chacun est tel que défini dans l'une quelconque des revendications 1 à 15, où, dans un desdits composés, $R^5$ représente un groupe éthyle et, dans l'autre desdits composés, $R^5$ représente un groupe méthyle.

18. Composé selon l'une quelconque des revendications précédentes, où $R^3$ et $R^4$ sont des atomes d'hydrogène.

19. Composé selon l'une quelconque des revendications précédentes, où X représente un groupe hydroxy.

20. 13-[2-(4-acétamidophényl)éthoxy]milbémycine $A_4$.

21. 13-[2-(4-cyanoacétamidophényl)éthoxy]milbémycine $A_4$.

22. 13-{2-[4-(2-cyanopropionamido)phényl]éthoxy}milbémycine $A_4$.

23. 13-[2-(4-méthoxyacétamidophényl)éthoxy]milbémycine $A_4$.

24. 13-{2-[4-(cyclopropylcarbonylamino)phényl]éthoxy} milbémycine $A_4$.

25. 13-{2-[4-(cyclobutylcarbonylamino)phényl]éthoxy} milbémycine $A_4$.

26. 13-{2-[4-(4-cyanobenzamido)phényl]éthoxy}milbémycine $A_4$.

27. 13-[2-(4-méthoxycarbonylaminophényl)éthoxy]milbémycine $A_4$.

28. 13-[2-(4-vinyloxycarbonylaminophényl)éthoxy]milbémycine $A_4$.

29. 13-{2-[4-(3-méthyluréido)phényl]éthoxy}milbémycine $A_4$.

30. 13-{2-[4-(3-phényluréido)phényl]éthoxy}milbémycine $A_4$.

31. 13-{2-[4-(3-cyclohexyluréido)phényl]éthoxy)milbémycine $A_4$.

32. 13-[2-(4-méthanesulfonylaminophényl)éthoxy]milbémycine $A_4$.

33. 13-[2-(4-éthanesulfonylaminophényl)éthoxy]milbémycine $A_4$.

**34.** 13-{2-[4-(3,3-diméthylcarbazoylamino)phényl]éthoxy} milbémycine $A_4$ et ses sels.

**35.** 13-{2-[4-(3-o-fluorophényluréido)phényl]éthoxy}milbémycine $A_4$.

**36.** 13-{2-[4-(3-p-fluorophényluréido)phényl]éthoxy}milbémycine $A_4$.

**37.** 13-{2-[4-(3-p-méthoxyphényluréido)phényl]éthoxy} milbémycine $A_4$.

**38.** Composition anthelminthique, acaricide et insecticide comprenant un composé anthelminthique, acaricide et insecticide en mélange avec un support ou diluant convenant dans le domaine de la pharmacie, de l'agriculture, de la médecine vétérinaire ou de l'horticulture, dans laquelle ledit composé est au moins un composé selon l'une quelconque des revendications précédentes.

**39.** Utilisation, pour la fabrication d'un médicament pour le traitement des infections provoquées par les helminthes, les acariens ou les insectes, d'au moins un composé selon l'une quelconque des revendications 1 à 37 ou d'une composition selon la revendication 38.

**40.** Procédé de protection des plantes contre les dommages provoqués par les acariens, les helminthes et les insectes, qui comprend l'application d'un composé actif auxdites plantes ou à des semences desdites plantes ou à un site comprenant lesdites plantes ou semences, dans lequel le composé actif est au moins un composé selon l'une quelconque des revendications 1 à 37 ou une composition selon la revendication 38.

**41.** Procédé pour préparer un composé selon l'une quelconque des revendications 1 à 37, qui comprend les étapes de : réaction d'un composé de formule (IV) :

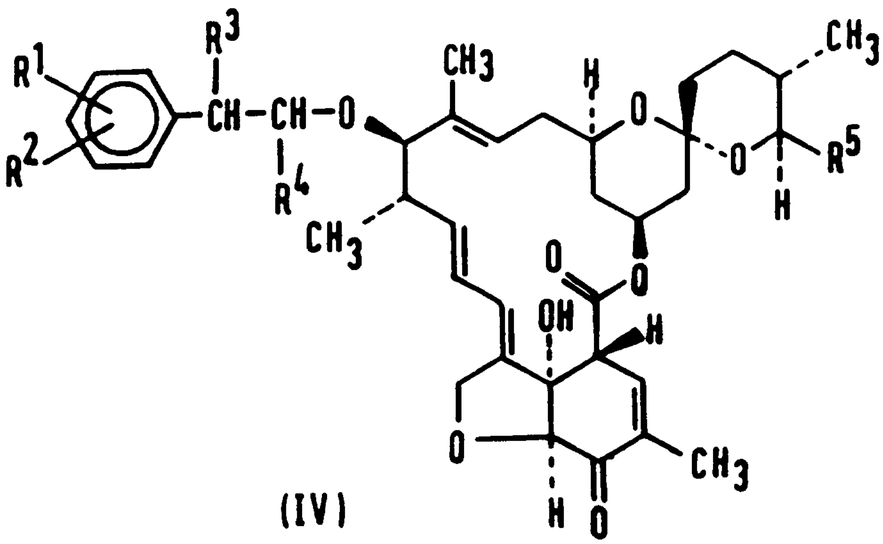

(IV)

(dans laquelle $R^1$, $R^2$, $R^3$, $R^4$ et $R^5$ sont tels que définis dans la revendication 1) avec un agent réducteur, pour préparer un composé de formule (I) dans laquelle X représente un groupe hydroxy ;
et, le cas échéant, acylation dudit composé dans lequel X représente un groupe hydroxy, pour préparer un composé de formule (I) dans laquelle X représente ledit groupe alcanoyloxy ;
ou réaction dudit composé de formule (IV) avec l'hydroxylamine ou avec un sel de celle-ci, pour préparer un composé de formule (I) dans laquelle X représente un groupe hydroxyimino.

**42.** Procédé selon la revendication 41, où ledit composé de formule (IV) est préparé par réaction d'un composé de formule (III) :

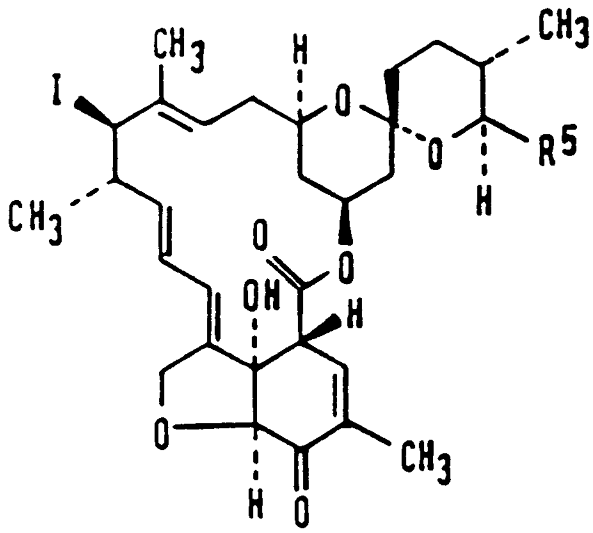

(III)

(dans laquelle $R^5$ est tel que défini dans la revendication 1) avec un composé de formule (III') :

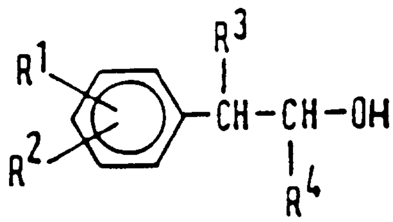

(III')

(dans laquelle $R^1$, $R^2$, $R^3$ et $R^4$ sont tels que définis dans la revendication 1).

**Revendications pour les Etats contractants suivants : ES, GR**

**1.** Procédé pour préparer un composé de formule (I) :

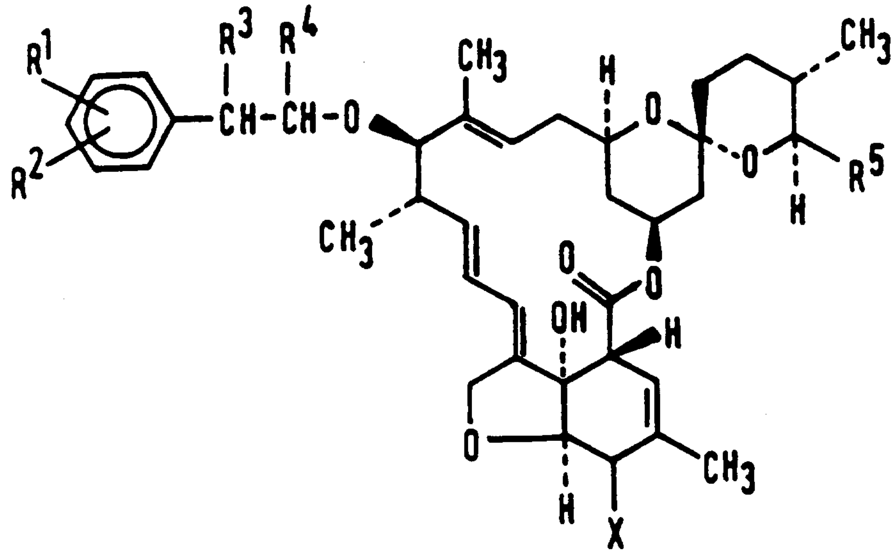

(I)

{dans laquelle :
$R^1$ et $R^2$ sont identiques ou différents et représentent chacun : un atome d'hydrogène ; un atome

d'halogène ; un groupe cyano ; un groupe nitro ; un groupe alkyle en $C_1$-$C_4$ ; un groupe alkyle en $C_1$-$C_4$ substitué ayant au moins un des substituants (a) définis ci-après ; un groupe alcoxy en $C_1$-$C_4$ ; un groupe alcoxyalcoxy en $C_2$-$C_6$ ; un groupe de formule $-(CH_2)_nNHR^9$,

dans laquelle : n représente 0 ou l'entier 1 ou 2, et $R^9$ représente un atome d'hydrogène ou un groupe alkyle en $C_1$-$C_4$ ;

un groupe de formule $-(CH_2)_nNR^9C(=O)R^6$,

dans laquelle :

n et $R^9$ sont tels que définis ci-dessus, et $R^6$ représente : un atome d'hydrogène ; un groupe alkyle en $C_1$-$C_4$ ; un groupe alkyle en $C_1$-$C_4$ substitué ayant au moins un des substituants (b) définis ci-après ; un groupe hydrocarboné aliphatique en $C_2$-$C_8$ ayant une ou deux doubles liaisons carbone-carbone à insaturation éthylénique, ledit groupe étant non substitué ou ayant au moins un des substituants (b) définis ci-après ; un groupe alcynyle en $C_2$-$C_8$ ; un groupe alcynyle en $C_2$-$C_8$ substitué ayant au moins un des substituants (b) définis ci-après ; un groupe cycloalkyle en $C_3$-$C_8$ ; un groupe cycloalkyle en $C_3$-$C_8$ substitué ayant au moins un des substituants (c) définis ci-après ; un groupe aryle carbocyclique ayant de 6 à 14 atomes de carbone formant le cycle et étant non substitué ou ayant au moins un des substituants (c) définis ci-après ; ou un groupe hétérocyclique ayant de 3 à 6 atomes formant le cycle dont au moins un est un hétéroatome d'azote et/ou d'oxygène et/ou de soufre, ledit groupe hétérocyclique étant monocyclique ou étant condensé à un ou à deux cycles benzène et étant non substitué ou ayant au moins un des substituants (c) définis ci-après ;

un groupe de formule $-(CH_2)_nNR^9COCOR^6$

dans laquelle n, $R^6$ et $R^9$ sont tels que définis ci-dessus ;

un groupe de formule $-(CH_2)_nR^9COCOOR^7$

dans laquelle n et $R^9$ sont tels que définis ci-dessus et $R^7$ représente un groupe alkyle en $C_1$-$C_4$, un groupe cycloalkyle en $C_3$-$C_8$ ou un groupe aralkyle tel que défini ci-après ;

un groupe de formule $-(CH_2)_nNR^9CHR^6NHCOR^6$

dans laquelle n, $R^6$ et $R^9$ sont tels que définis ci-dessus ;

un groupe de formule $-(CH_2)_nNR^9CHR^6NHCONHR^6$

dans laquelle n, $R^6$ et $R^9$ sont tels que définis ci-dessus ;

un groupe de formule $-(CH_2)_nNR^9CHR^6NHCOOR^7$

dans laquelle n, $R^6$, $R^7$ et $R^9$ sont tels que définis ci-dessus ;

un groupe de formule $-(CH_2)_nNR^9C(=Y)YR^6$

dans laquelle n, $R^6$ et $R^9$ sont tels que définis ci-dessus et les deux symboles Y sont identiques ou différents et représentent chacun un atome d'oxygène ou un atome de soufre ;

un groupe de formule $-(CH_2)_nNR^9C(=Y)NR^{6'}R^{6'}$

dans laquelle n, Y et $R^9$ sont tels que définis ci-dessus et les deux symboles $R^{6'}$ sont identiques ou différents et représentent chacun un des groupes ou atomes représentés par $R^6$ ou, tous deux, avec l'atome d'azote auquel ils sont fixés, forment un groupe hétérocyclique ayant de 3 à 7 atomes formant le cycle dont un est ledit atome d'azote et 0 ou 1 est un hétéroatome additionnel d'azote et/ou d'oxygène et/ou de soufre ;

un groupe de formule $-(CH_2)_nNR^9C(=Y)NR^{6''}NR^{6''}R^{6''}$

dans laquelle n, Y et $R^9$ sont tels que définis ci-dessus et les symboles $R^{6''}$ sont identiques ou différents et représentent chacun un des groupes ou atomes représentés par $R^6$, ou deux quelconques des symboles $R^{6''}$, avec l'atome d'azote auquel ils sont chacun fixés, forment un groupe hétérocyclique ayant de 3 à 7 atomes formant le cycle dont un ou les deux est le ou sont lesdits atomes d'azote et 0 ou 1 est un hétéroatome additionnel d'azote et/ou d'oxygène et/ou de soufre ;

un groupe de formule $-(CH_2)_nNR^9C(=Y)NR^6NHZ$

dans laquelle n, Y, $R^6$ et $R^9$ sont tels que définis ci-dessus et Z représente

un groupe de formule $-COOR^7$, dans laquelle $R^7$ est tel que défini ci-dessus,

un groupe de formule $-COR^6$, dans laquelle $R^6$ est tel que défini ci-dessus, ou

un groupe de formule $-SO_2R^6$, dans laquelle $R^6$ est tel que défini ci-dessus ;

un groupe de formule $-(CH_2)_nNR^9C(=NR^{10})NHR^{10}$

dans laquelle n et $R^9$ sont tels que définis ci-dessus et les deux symboles $R^{10}$ sont identiques ou différents et représentent chacun un des groupes ou atomes représentés par $R^6$ ou un groupe cyano, un groupe nitro, un groupe de formule $-COOR^7$, dans laquelle $R^7$ est tel que défini ci-dessus, ou un groupe de formule $-COR^6$, dans laquelle $R^6$ est tel que défini ci-dessus ;

un groupe de formule $-(CH_2)_nNR^9C(=NR^{10})R^6$

dans laquelle n, $R^6$, $R^9$ et $R^{10}$ sont tels que définis ci-dessus ;

un groupe de formule $-(CH_2)_nNR^9SO_mR^6$

dans laquelle n, $R^6$ et $R^9$ sont tels que définis ci-dessus et m est 1 ou 2 ;
un groupe de formule $-CONHR^6$
dans laquelle $R^6$ est tel que défini ci-dessus ; et
un groupe de formule $-COOR^7$
dans laquelle $R^7$ est tel que défini ci-dessus ;
$R^3$ et $R^4$ sont identiques ou différents et représentent chacun un atome d'hydrogène, un groupe alkyle en $C_1$-$C_4$ ou un groupe alcoxy en $C_1$-$C_4$ ;
$R^5$ représente un groupe méthyle, un groupe éthyle, un groupe isopropyle ou un groupe sec-butyle ; et
X représente un groupe hydroxy, un groupe alcanoyloxy en $C_1$-$C_5$, un groupe alcanoyloxy en $C_1$-$C_5$ substitué ayant au moins un des substituants (d) définis ci-après, ou un groupe hydroxyimino ; lesdits groupes aralkyle ont de 1 à 4 atomes de carbone dans la partie alkyle et de 6 à 10 atomes formant le cycle dans la partie aryle, qui est un groupe aryle carbocyclique qui est non substitué ou qui a au moins un des substituants (c) définis ci-après ;

substituants (a) :
atomes d'halogène, groupes alcoxy en $C_1$-$C_4$, groupes alkylthio en $C_1$-$C_4$ et groupes alcanoyloxy en $C_1$-$C_5$ ;

substituants (b) :
groupes cycloalkyle en $C_3$-$C_8$ ; groupes alcoxy en $C_1$-$C_4$ ; groupes alkylthio en $C_1$-$C_4$ ; groupes cyanoalkylthio en $C_2$-$C_5$ ; groupes alcoxycarbonyle en $C_2$-$C_5$ ; atomes d'halogène ; groupes cyano ; groupes nitro ; groupes amino ; groupes aryle carbocycliques ayant de 6 à 10 atomes de carbone et étant non substitués ou ayant au moins un des substituants (c) définis ci-après ; groupes hétérocycliques aromatiques ayant de 5 à 8 atomes formant le cycle dont 1 à 4 sont des hétéroatomes d'azote et/ou d'oxygène et/ou de soufre, ledit groupe hétérocyclique étant monocyclique ou étant condensé soit à un cycle benzène, soit à un groupe hétérocyclique qui a 5 ou 6 atomes formant le cycle dont 1 à 3 sont des hétéroatomes d'azote, et étant non substitué ou ayant au moins un des substituants (c) définis ci-après ; et groupes aryloxy et arylthio dont la partie aryle a de 6 à 10 atomes de carbone et est non substituée ou a au moins un des substituants (c) définis ci-après ;

substituants (c) :
groupes alkyle en $C_1$-$C_4$, groupes alcoxy en $C_1$-$C_4$, groupes alkylthio en $C_1$-$C_4$, groupes alcanoyloxy en $C_1$-$C_5$, groupes alcoxycarbonyle en $C_2$-$C_5$, atomes d'halogène, groupes cyano, groupes nitro, groupes amino, groupes mono- et dialkylamino dont la ou chaque partie alkyle est en $C_1$-$C_4$, groupes carbamoyle, groupes mono- et dialkylcarbamoyle dont la ou chaque partie alkyle est en $C_1$-$C_4$, et groupes alcanoylamino en $C_1$-$C_5$ ;

substituants (d) :
atomes d'halogène, groupes alcoxy en $C_1$-$C_4$, groupes alcoxycarbonyle en $C_2$-$C_5$ et groupes carboxy} ;
ou un de ses sels,
lequel procédé comprend les étapes de :
réaction d'un composé de formule (IV) :

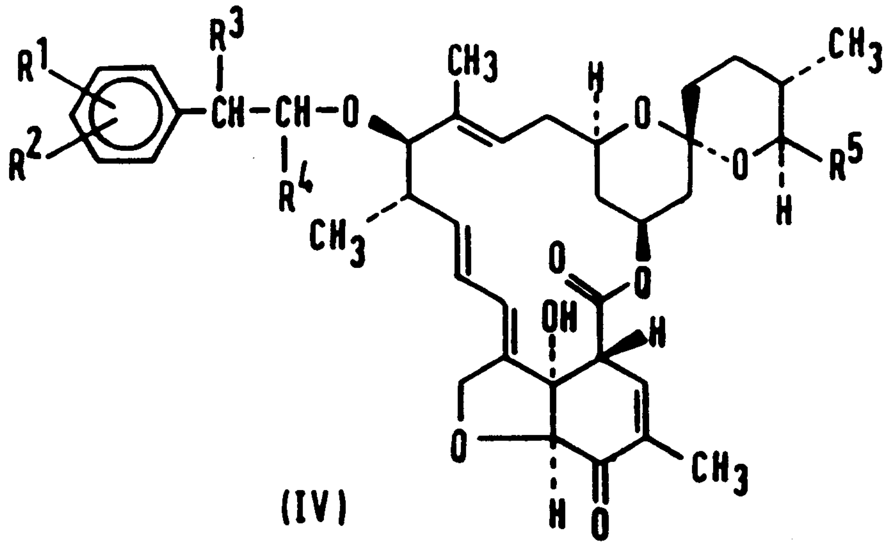

(IV)

(dans laquelle $R^1$, $R^2$, $R^3$, $R^4$ et $R^5$ sont tels que définis ci-dessus) avec un agent réducteur, pour préparer un composé de formule (I) dans laquelle X représente un groupe hydroxy ;
et, le cas échéant, acylation dudit composé dans lequel X représente un groupe hydroxy, pour préparer un composé de formule (I) dans laquelle X représente ledit groupe alcanoyloxy ;
ou réaction dudit composé de formule (IV) avec l'hydroxylamine ou avec un sel de celle-ci, pour préparer un composé de formule (I) dans laquelle X représente un groupe hydroxyimino.

**2.** Procédé selon la revendication 1, où ledit composé de formule (IV) est préparé par réaction d'un composé de formule (III) :

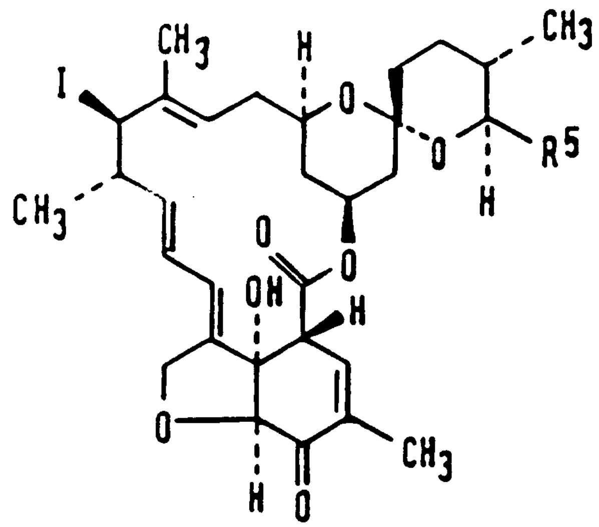

(III)

(dans laquelle $R^5$ est tel que défini dans la revendication 1) avec un composé de formule (III') :

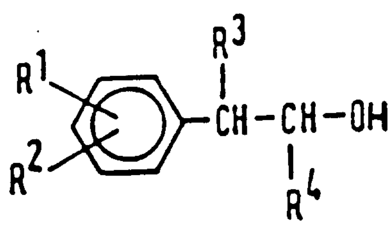

(III')

(dans laquelle $R^1$, $R^2$, $R^3$ et $R^4$ sont tels que définis dans la revendication 1).

**3.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un atome d'hydrogène, un groupe alkyle en $C_1$-$C_3$, un groupe alcoxy en $C_1$-$C_3$, un atome de fluor ou de chlore, un groupe nitro ou un groupe amino.

**4.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}COR^{6a}$
dans laquelle n est 0, $R^{9a}$ représente un atome d'hydrogène ou un groupe méthyle et $R^{6a}$ représente : un groupe alkyle en $C_1$-$C_4$ ; un groupe cycloalkyle en $C_3$-$C_5$ ; un groupe alkyle en $C_1$-$C_3$ substitué avec un substituant halogène, cyano, alcoxy en $C_1$-$C_3$, alkylthio en $C_1$-$C_3$, cyanométhylthio où phénoxy ; un groupe alcényle ; un groupe phényle non substitué ; un groupe phényle substitué avec un substituant alkyle en $C_1$-$C_3$, alcoxy en $C_1$-$C_3$, halogène ou nitro ; un groupe pyridyle ; un groupe pyrimidyle ; un groupe pyrazyle ; un groupe furyle ; ou un groupe thiényle.

**5.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}COCOR^{6b}$
dans laquelle n est 0, $R^{9a}$ représente un atome d'hydrogène ou un groupe méthyle et $R^{6b}$ représente un atome d'hydrogène, un groupe alkyle en $C_1$-$C_4$, un groupe cycloalkyle en $C_3$-$C_5$, un groupe alcényle, un groupe phényle non substitué ou un groupe phényle substitué avec un substituant alkyle en $C_1$-$C_3$, alcoxy en $C_1$-$C_3$, halogène ou nitro.

**6.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}C(=Y)YR^{6c}$
dans laquelle n est 0, $R^{9a}$ représente un atome d'hydrogène ou un groupe méthyle, les deux symboles Y sont des atomes d'oxygène, et $R^{6c}$ représente un groupe alkyle en $C_1$-$C_4$, un groupe alkyle en $C_1$-$C_4$ substitué avec un substituant halogène ou alcoxy en $C_1$-$C_3$, un groupe alcényle, un groupe benzyle, un groupe méthoxybenzyle, un groupe nitrobenzyle, un groupe furfuryle, un groupe thiényle ou un groupe phényle.

**7.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}C(=Y)NR^{6d}R^{6e}$
dans laquelle n est 0, $R^{9a}$ représente un atome d'hydrogène ou un groupe méthyle, Y représente un atome d'oxygène ou un atome de soufre et $R^{6d}$ et $R^{6e}$ sont identiques ou différents et représentent chacun un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$,alcoxy en $C_1$-$C_3$, halogène ou nitro, ou $R^{6d}$ et $R^{6e}$, avec l'atome d'azote auquel ils sont fixés, représentent un groupe pipéridino, pipérazino, morpholino, pyrrolidino ou aziridino.

**8.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}C(=Y)NR^{6f}NR^{6g}R^{6h}$
dans laquelle n est 0, $R^{9a}$ représente un atome d'hydrogène ou un groupe méthyle, Y représente un atome d'oxygène ou un atome de soufre et $R^{6f}$, $R^{6g}$ et $R^{6h}$ sont identiques ou différents et représentent chacun : un atome d'hydrogène ; un groupe alkyle en $C_1$-$C_4$ ; un groupe cycloalkyle en $C_3$-$C_6$ ; un groupe phényle non substitué ; ou un groupe phényle substitué avec un substituant alkyle en $C_1$-$C_3$, alcoxy en $C_1$-$C_3$, halogène ou nitro ; ou $R^{6g}$ et $R^{6h}$, avec l'atome d'azote auquel ils sont fixés, représentent un groupe pipéridino, pipérazino, morpholino, pyrrolidino ou aziridino ; ou $R^{6f}$ et $R^{6g}$, avec les atomes d'azote auxquels ils sont fixés, représentent un groupe pyrazolidinyle ou tétrahydropyridazinyle.

**9.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}C(=Y)NR^{6j}NHZ$
dans laquelle n est 0, $R^{9a}$ représente un atome d'hydrogène ou un groupe méthyle, Y représente un atome d'oxygène ou un atome de soufre, $R^{6j}$ représente un atome d'hydrogène, un groupe alkyle en $C_1$-$C_4$ ou un groupe cycloalkyle en $C_3$-$C_6$ ; et Z représente un groupe de formule $-COOR^{7a}$
dans laquelle $R^{7a}$ représente un groupe alkyle en $C_1$-$C_4$, un groupe cycloalkyle en $C_3$-$C_6$ ou un groupe benzyle,
un groupe de formule $-COR^{6k}$
dans laquelle $R^{6k}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro ou
un groupe de formule $-SO_2R^{6m}$
dans laquelle $R^{6m}$ représente un groupe alkyle en $C_1$-$C_4$, un groupe phényle non substitué ou un

groupe phényle substitué avec un substituant alkyle en $C_1$-$C_3$, alcoxy en $C_1$-$C_3$, halogène ou nitro.

**10.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}C(=NR^{10a})NHR^{10b}$
dans laquelle n est 0, $R^{9a}$ représente un atome d'hydrogène ou un groupe méthyle et $R^{10a}$ et $R^{10b}$ sont identiques ou différents et représentent chacun : un atome d'hydrogène ; un groupe alkyle en $C_1$-$C_4$ ; un groupe phényle non substitué ; un groupe phényle substitué avec un substituant alkyle en $C_1$-$C_3$, alcoxy en $C_1$-$C_3$, halogène ou nitro ; un groupe de formule $-COOR^{7b}$
dans laquelle $R^{7b}$ représente un groupe alkyle en $C_1$-$C_4$, un groupe cycloalkyle en $C_3$-$C_6$ ou un groupe benzyle ; ou
un groupe de formule $-COR^{6n}$
dans laquelle $R^{6n}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro.

**11.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}C(=NR^{10c})R^{6p}$
dans laquelle n est 0 ; $R^{9a}$ représente un atome d'hydrogène ou un groupe méthyle ; $R^{10c}$ représente un atome d'hydrogène, un groupe alkyle en $C_1$-$C_4$, un groupe phényle non substitué, un groupe phényle substitué avec un substituant alkyle en $C_1$-$C_3$, alcoxy en $C_1$-$C_3$, halogène ou nitro, un groupe de formule $-COOR^{7c}$
dans laquelle $R^{7c}$ représente un groupe alkyle en $C_1$-$C_4$, un groupe cycloalkyle en $C_3$-$C_6$ ou un groupe benzyle ;
ou un groupe de formule $-COR^{6q}$
dans laquelle $R^{6q}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro et
$R^{6p}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$, alcoxy en $C_1$-$C_3$, halogène ou nitro.

**12.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène ; et
$R^2$ représente un groupe de formule $-(CH_2)_nNR^{9a}SO_mR^{6r}$
dans laquelle n est 0, $R^{9a}$ représente un atome d'hydrogène ou un groupe méthyle, m est 1 ou 2, et $R^{6r}$ représente un groupe alkyle en $C_1$-$C_4$, un groupe cyanoalkyle en $C_2$-$C_4$, un groupe phényle non substitué ou un groupe phényle substitué avec un substituant alkyle en $C_1$-$C_3$, alcoxy en $C_1$-$C_3$, halogène ou nitro.

**13.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène et
$R^2$ représente un groupe de formule $-CONHR^{6s}$
dans laquelle $R^{6s}$ représente un groupe alkyle en $C_1$-$C_4$, un groupe cycloalkyle en $C_3$-$C_6$, un groupe phényle non substitué ou un groupe phényle substitué avec un substituant alkyle en $C_1$-$C_3$,alcoxy en $C_1$-$C_3$, halogène ou nitro.

**14.** Procédé selon la revendication 1 ou la revendication 2, dans lequel les composés réagissants et les conditions de réaction sont choisis pour préparer un composé de formule (I) ou un de ses sels, où :
$R^1$ représente un atome d'hydrogène et
$R^2$ représente un groupe de formule $-COOR^{7d}$
dans laquelle $R^{7d}$ représente un groupe méthyle, éthyle ou benzyle.

**15.** Procédé selon la revendication 1 ou la revendication 2, dans lequel les composés réagissants et les conditions de réaction sont choisis pour préparer un composé de formule (I) ou un de ses sels, où :
$R^1$ représente un groupe méthoxy et
$R^2$ représente un groupe alcoxy en $C_1$-$C_3$ ou un groupe alcoxyalcoxy en $C_2$-$C_4$.

**16.** Procédé selon l'une quelconque des revendications précédentes, dans lequel les composés réagissants et les conditions de réaction sont choisis pour préparer un composé de formule (I) ou un de ses sels, où $R^2$ est fixé à la position p du cycle benzène.

**17.** Procédé selon l'une quelconque des revendications précédentes, dans lequel les composés réagissants et les conditions de réaction sont choisis pour préparer un composé de formule (I) ou un de ses sels, où $R^5$ représente un groupe éthyle.

**18.** Procédé selon l'une quelconque des revendications 1 à 15, dans lequel les composés réagissants et les conditions de réaction sont choisis pour préparer une composition comprenant un mélange de composés de formule (I) ou de leurs sels, où, dans un desdits composés, $R^5$ représente un groupe éthyle et, dans l'autre desdits composés, $R^5$ représente un groupe méthyle.

**19.** Procédé selon l'une quelconque des revendications précédentes, dans lequel les composés réagissants et les conditions de réaction sont choisis pour préparer un composé de formule (I) ou un de ses sels, où $R^3$ et $R^4$ sont des atomes d'hydrogène.

**20.** Procédé selon l'une quelconque des revendications précédentes, dans lequel les composés réagissants et les conditions de réaction sont choisis pour préparer un composé de formule (I) ou un de ses sels, où X représente un groupe hydroxy.

**21.** Procédé selon l'une quelconque des revendications précédentes, dans lequel les composés réagissants et les conditions de réaction sont choisis pour préparer les :
13-[2-(4-acétamidophényl)éthoxy]milbémycine $A_4$ ;
13-[2-(4-cyanoacétamidophényl)éthoxy]milbémycine $A_4$ ;
13-{2-[4-(2-cyanopropionamido)phényl]éthoxy}milbémycine $A_4$ ;
13-[2-(4-méthoxyacétamidophényl)éthoxy]milbémycine $A_4$ ;
13-{2-[4-(cyclopropylcarbonylamino)phényl]éthoxy}milbémycine $A_4$ ;
13-{2-[4-(cyclobutylcarbonylamino)phényl]éthoxy}milbémycine $A_4$ ;
13-{2-[4-(4-cyanobenzamido)phényl]éthoxy}milbémycine $A_4$ ;
13-[2-(4-méthoxycarbonylaminophényl)éthoxy]milbémycine $A_4$ ;
13-[2-(4-vinyloxycarbonylaminophényl)éthoxy]milbémycine $A_4$ ;
13-{2-[4-(3-méthyluréido)phényl]éthoxy}milbémycine $A_4$ ;
13-{2-[4-(3-phényluréido)phényl]éthoxy}milbémycine $A_4$ ;
13-{2-[4-(3-cyclohexyluréido)phényl)éthoxy}milbémycine $A_4$ ;
13-[2-(4-méthanesulfonylaminophényl)éthoxy]milbémycine $A_4$ ;
13-[2-(4-éthanesulfonylaminophényl)éthoxy]milbémycine $A_4$ ;
13-{2-[4-(3,3-diméthylcarbazoylamino)phényl]éthoxy}milbémycine $A_4$ ;
13-{2-[4-(3-o-fluorophényluréido)phényl]éthoxy}milbémycine $A_4$ ;
13-{2-[4-(3-p-fluorophényluréido)phényl]éthoxy}milbémycine $A_4$
13-{2-[4-(3-p-méthoxyphényluréido)phényl]éthoxy}milbémycine $A_4$ ;
ou un de leurs sels.

**22.** Procédé pour préparer une composition anthelminthique, acaricide et insecticide comprenant le mélange d'un composé anthelminthique, acaricide et insecticide avec un support ou diluant convenant dans le domaine de la pharmacie, de l'agriculture, de la médecine vétérinaire ou de l'horticulture, dans lequel ledit composé est au moins un composé préparé selon l'une quelconque des revendications précédentes.

**23.** Utilisation, pour la fabrication d'un médicament pour le traitement des infections provoquées par les helminthes, les acariens ou les insectes, d'au moins un composé de formule (I) défini dans l'une quelconque des revendications 1 à 21 ou d'une composition préparée selon la revendication 22.